(19) Europäisches Patentamt
European Patent Office
Office européen des brevets

(11) **EP 2 605 816 B1**

(12) **EUROPEAN PATENT SPECIFICATION**

(45) Date of publication and mention of the grant of the patent:
**23.01.2019 Bulletin 2019/04**

(51) Int Cl.:
*A61M 15/08* *(2006.01)*   *A61M 11/02* *(2006.01)*
*A61M 19/00* *(2006.01)*   *A61K 9/127* *(2006.01)*

(21) Application number: **11818832.5**

(22) Date of filing: **19.08.2011**

(86) International application number:
**PCT/US2011/048435**

(87) International publication number:
**WO 2012/024595 (23.02.2012 Gazette 2012/08)**

(54) **CIRCUMFERENTIAL AEROSOL DEVICE FOR DELIVERING DRUGS TO OLFACTORY EPITHELIUM AND BRAIN**

UMLAUFENDE AEROSOLVORRICHTUNG ZUR ABGABE VON ARZNEIMITTELN AN DIE RIECHSCHLEIMHAUT UND DAS GEHIRN

PULVÉRISATEUR CIRCONFÉRENTIEL POUR ADMINISTRATION DE MÉDICAMENTS À L'ÉPITHÉLIUM OLFACTIF ET AU CERVEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2010 US 375682 P**
**05.10.2010 US 389920 P**

(43) Date of publication of application:
**26.06.2013 Bulletin 2013/26**

(73) Proprietor: **University of Washington**
**Seattle, WA 98105-4608 (US)**

(72) Inventors:
• **HOEKMAN, John D.**
**Seattle**
**WA 98102 (US)**

• **HO, Rodney, J.y.**
**Mercer Island**
**WA 98040 (US)**

(74) Representative: **Abel & Imray**
**Westpoint Building**
**James Street West**
**Bath BA1 2DA (GB)**

(56) References cited:
WO-A1-02/09707   WO-A2-2009/100383
US-A- 5 910 301   US-A1- 2003 017 119
US-B1- 6 294 153

**Description**

This invention was made with U.S. Government support under AI052663 awarded by National Institutes of Health (NIH). The U.S. Government has certain rights.

BACKGROUND OF THE INVENTION

Many drugs cannot reach the brain in significant concentrations. Deposition of therapeutic drugs on the olfactory epithelium has been shown to lead to rapid and direct uptake into the brain. Direct nose-to-brain drug delivery bypasses the blood-brain barrier.

WO 2002/09707 discloses a fentanyl composition for transmucosal nasal delivery by squirt or spray.

SUMMARY OF THE INVENTION

The present invention provides a pharmaceutical aerosol suspension for use in a method of delivering a therapeutic compound to an olfactory epithelium in a nasal cavity of a mammal, the method comprising:

providing a pharmaceutical aerosol suspension comprising an aerosol and the therapeutic compound, wherein the therapeutic compound is an opioid analgesic, mannitol, TS-002, IGF-1, orexin-A, interferon-$\beta$, MSH or an RGD peptide;

aerosolizing the suspension to generate a stream of droplets; and,

administering the stream of droplets having a rotational component having a circumferential velocity after entering the nasal cavity such that at least 15% of the stream of droplets are delivered directly to the olfactory epithelium.

The present application describes a pressurized olfactory delivery (POD) device generates an aerosol nasal spray having a narrow spray plume with circumferential velocity. The device displaces the residual olfactory air volume to deliver therapeutic compounds, that is one or more active pharmaceutical ingredients (APIs) and/or inactive pharmaceutical ingredients) to the olfactory region of the nasal cavity. The aerosol composition containing the API, aerosol and inactive pharmaceutical ingredients may be referred to herein as an aerosol dosage form.

For example, the POD device may include a container containing a mixture of a pressurized fluid (e.g., an aerosol) and an API. The POD device includes a plurality of longitudinal helical channels. Each helical channel includes an inlet and an outlet disposed at the nasal proximal-most end of the device. A metering device selectively discharges the aerosol composition through the helical channels. The outlets are configured to discharge a plurality of aerosol spray jets that converge into a single spray plume having a circumferential helical velocity.

The present invention finds utility in a method which includes depositing (i.e., delivering) the API to the olfactory epithelium in the nasal cavity of a human or animal subject (e.g., a patient in need of being treated with the API). The method includes administering the aerosol composition using the POD device into the nasal cavity. The device discharges the aerosol composition in the form of a spray having a circumferential velocity upon exiting the nozzle.

The present application discloses a pressurized olfactory delivery (POD) device that produces an aerosol nasal spray having a narrow spray plume with circumferential velocity. The device disclosed herein is designed to displace the residual olfactory air volume under low pressure to increase the efficiency and consistency with which pharmaceutical compounds are delivered to olfactory epithelium, and further to enhance patient tolerability.

DESCRIPTION OF DRAWINGS OF EXEMPLARY FEATURES

FIG. 1 is a schematic illustration of one example of a pressurized olfactory delivery (POD) device.
FIG. 2A is a partial cross sectional view of the nasal delivery device of FIG. 1.
FIG. 2B is a partial cross sectional view of the nasal delivery device of FIG. 1 in operation.
FIG. 3 is a partial cross sectional view of the pressurized olfactory delivery device of FIG. 1.
FIG. 4 is a cross sectional view of the pressurized olfactory delivery device of FIG. 1.
FIG. 5 is a partial cross sectional view of the pressurized olfactory delivery device of FIG.1.
FIG. 6A is an axial view of the nozzle shown in FIG. 5.
FIG. 6B is an isometric view of the upper portion of the nozzle shown in FIG. 5.
FIG. 6C is an isometric view of the lower portion of the nozzle shown in FIG. 5.

FIG. 7 is a partial cross sectional view of the pressurized olfactory delivery device of FIG. 1.

FIG. 8A is a cut-away view of the pressurized olfactory delivery device of FIG. 1.

FIG. 8B is a top perspective view of the device shown in FIG. 8A.

FIG. 8C is an exploded view of the device shown in FIG. 8B.

FIG. 8D is a bottom perspective view of the device shown in FIG. 8A.

creates circumferential velocity, whereby the nozzles had the same outlet surface area and initial aerosol release parameters and all were simulated after a 0.1 second duration of aerosol spray, whereby the velocity shows a cross section of the cone and nozzle and displays the air velocity while deposition shows the surface of the cone and the mass flux of liquid aerosol particles impacting the cone, and whereby it can be seen that the nozzle with

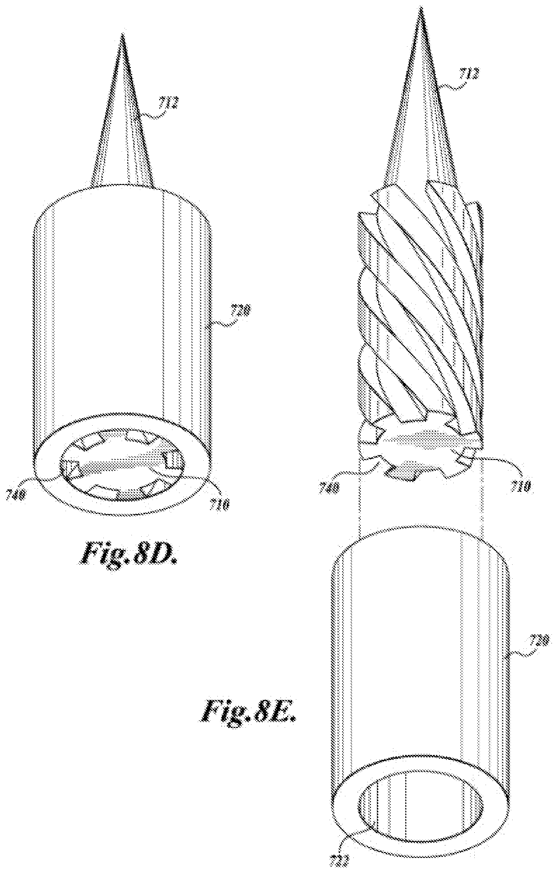

FIG. 8E is an exploded view of the device shown in FIG. 8D.

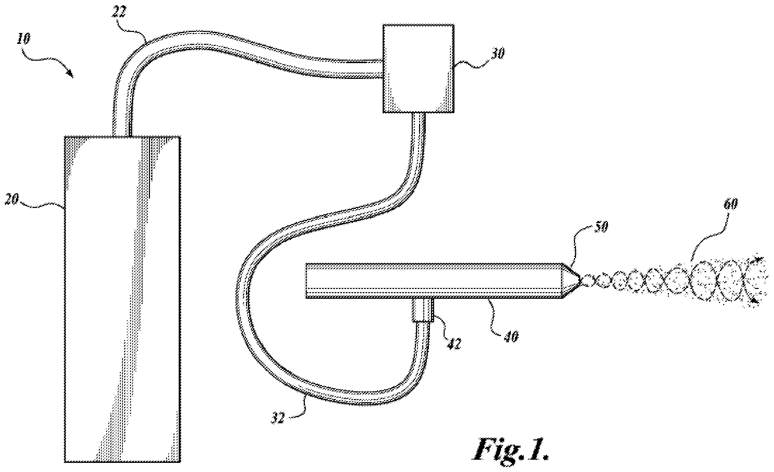

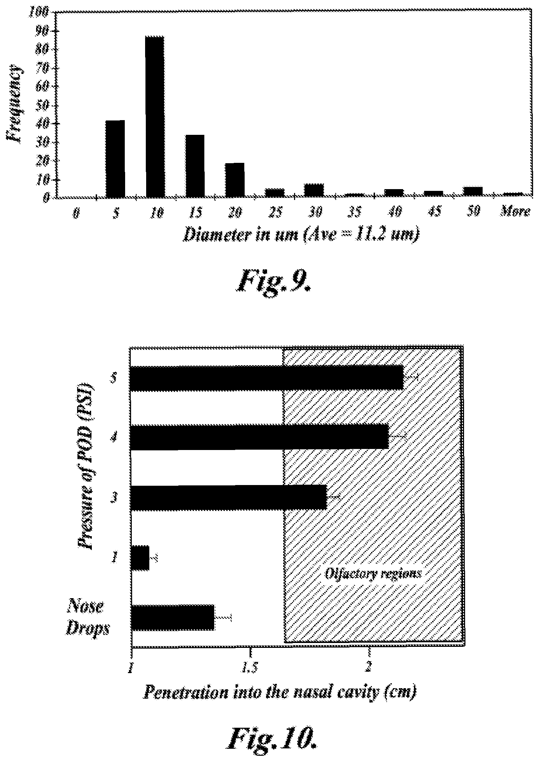

FIG. 9 is a graph illustrating the particle size generated in Example 1. The POD device used is shown in FIG. 1.

FIG. 10 is a graph illustrating the penetration of blue dye into the nasal cavity of rats administered in Example 1, wherein the dye was administered from the device at different air pressures, and each horizontal bar represents the mean value for 4-6 rats. The error bars represent standard deviation. The POD device used is shown in FIG. 1

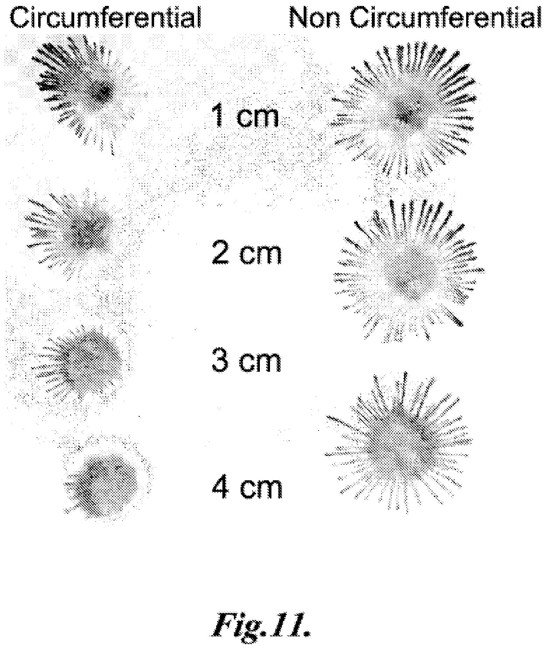

FIG. 11 is a series of photographs on the left illustrating the spray pattern produced by the device compared to spray patterns of a prior art device on the right, whereby the side-by-side comparison illustrates circumferential velocity achieved by the device but not by the prior art device, see Example 1. The POD device used is shown in FIG. 1

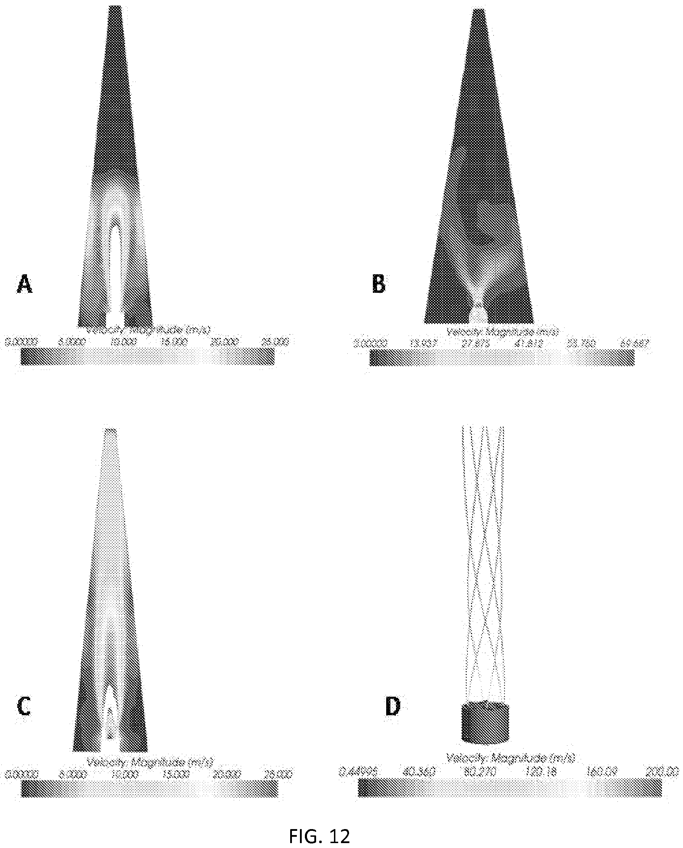

FIG. 12A shows the simplex air flow pattern and velocity of the spray from a flow simulation using an outlet absent circumferential velocity in a flow simulation, whereby poor penetration of the cone, as described in Example 3, is demonstrated. The POD device used is shown in FIG. 1

FIG. 12B shows the circumferential flow pattern and velocity of the spray from a flow simulation using a nozzle having a single outlet generating circumferential movement, as described in Example 3, also demonstrates poor penetration of the cone. The POD device used is shown in FIG. 1

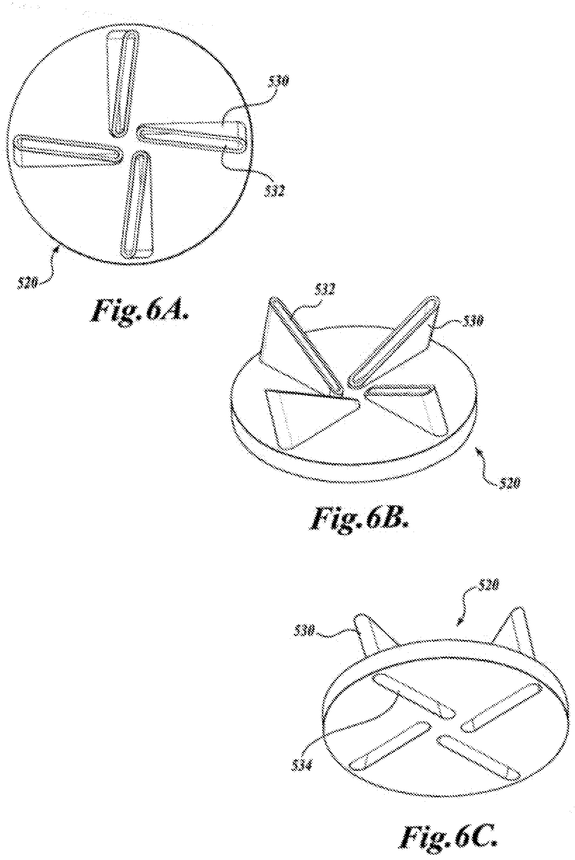

FIG. 12C shows the rotational flow pattern and velocity of the spray from a flow simulation using the device shown in FIG. 6, whereby improved penetration of the cone attributable to a narrow spray having circumferential and axial velocity, as described in Example 3, is demonstrated. The POD device used is shown in FIG. 1

FIG. 12D illustrates the flow-streams from the spray pattern shown in FIG. 12C. The POD device used is shown in FIG. 1

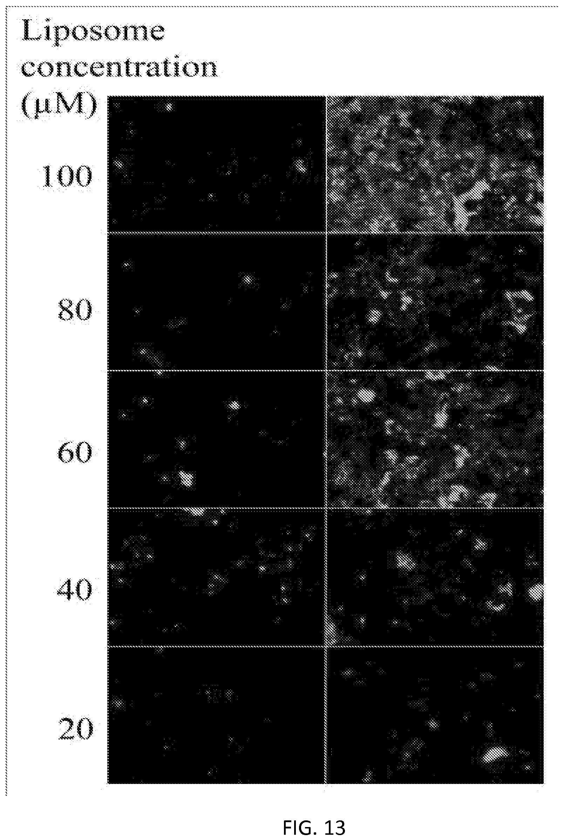

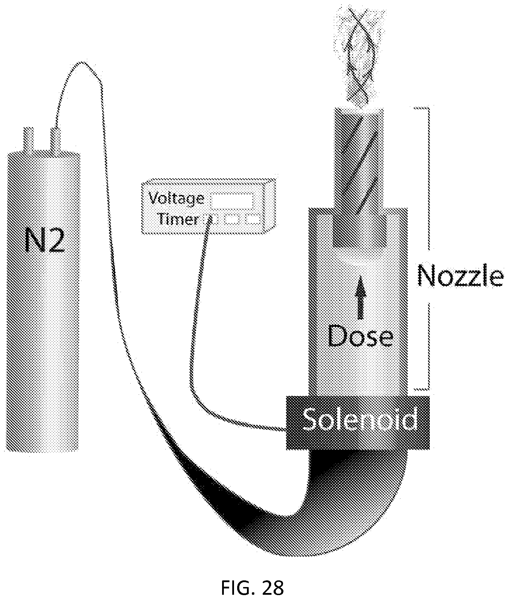

FIG. 13 illustrates in vitro binding of integrin-targeted vs. non-targeted liposome nanoparticles to ILC-PKI epithelial cells being a cell model for olfactory epithelium, whereby the liposome nanoparticles were labeled with NBD-lipid that provide green fluorescence on fluoromicrograph, and whereby the integrin-targeted RGD liposomes exhibit a significant degree of liposome nanoparticle binding (appearing as green fluorescence) but the non-targeted liposomes failed to exhibit significant binding. It also shows concentration-dependent binding of 1% integrin targeted and non-targeted DMPC:DMPG liposomes to LLC-PK1 epithelial cells after a 30 minute incubation. The POD device used is shown in FIG. 28.

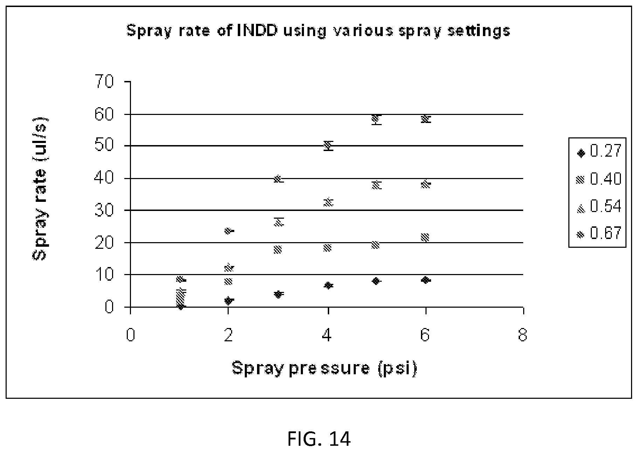

FIG. 14 is a graph demonstrating the effects of the pressure and the diameter of the nozzle opening on the rate of aerosol dispensed (measured as the spray rate), whereby the legend numbers represent the distance, in mm, the pin was pulled away from the spray nozzle. The POD device used is shown in FIG. 28.

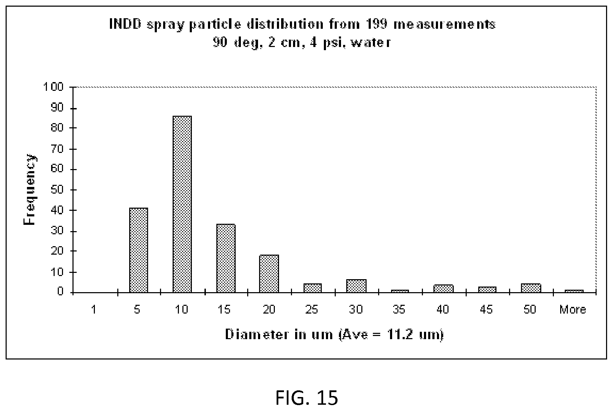

FIG. 15 is bar graph demonstrating the particle size distribution or aerosolized water discharged from the POD device shown in FIG. 28.

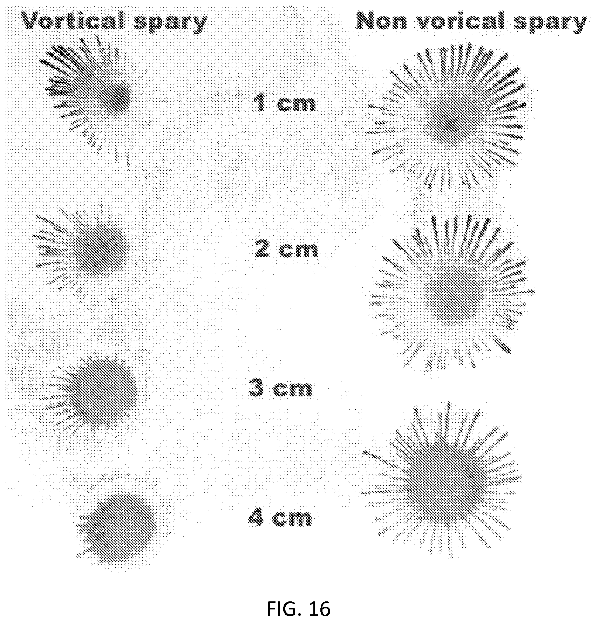

FIG. 16 demonstrates the vortical or centrifugal patterns dispensed from the POD (left) device shown in FIG. 28 as compared to control nozzle (right) without vortical spray attachment. Note that the asymmetrical pattern of the dye rotates as the distance increase from the POD spray nozzle.

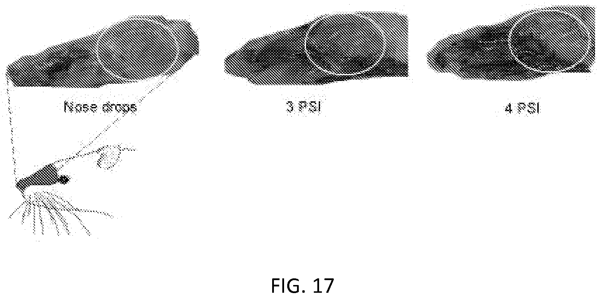

FIG. 17 is an illustration demonstrating the experimental distribution to olfactory epithelium with bench-top prototype POD device shown in FIG. 28 or nasal drops, whereby the degree of penetration into the nasal cavity for POD is evaluated with two different pressure settings (3 and 4 psi), whereby the target olfactory region is shown within the yellow circle, and whereby the inset drawing shows the anatomical location of the dissected areas.

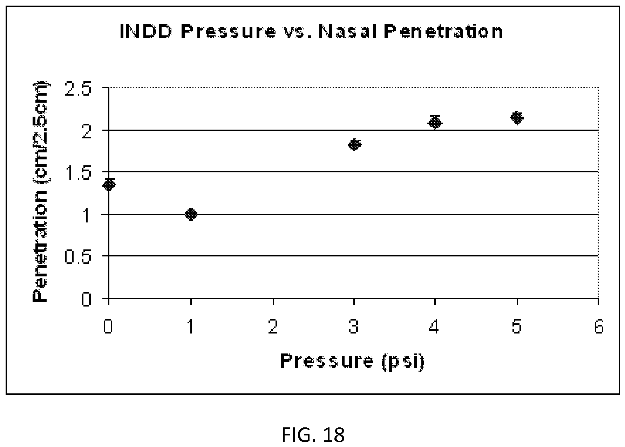

FIG. 18 is a graph demonstrating the effects of varying pressure on the extent of aerosolized dye penetration in the nasal cavity with a maximum distance of 2.5 cm for rats, whereby the distance was measured from the naris to the furthest edge of dye penetration as shown in FIG. 17. The POD device used is shown in FIG. 28.

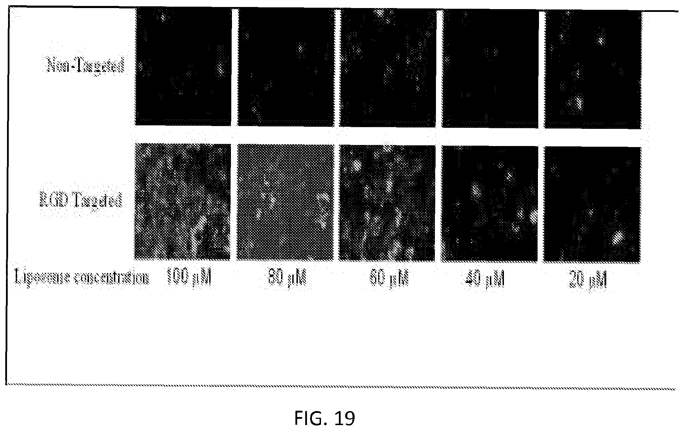

FIG. 19 demonstrates the concentration-dependent binding of targeted and non-targeted DMPC:DMPG liposomes to LLC-PK1 epithelial cells. The POD device used is shown in FIG. 28.

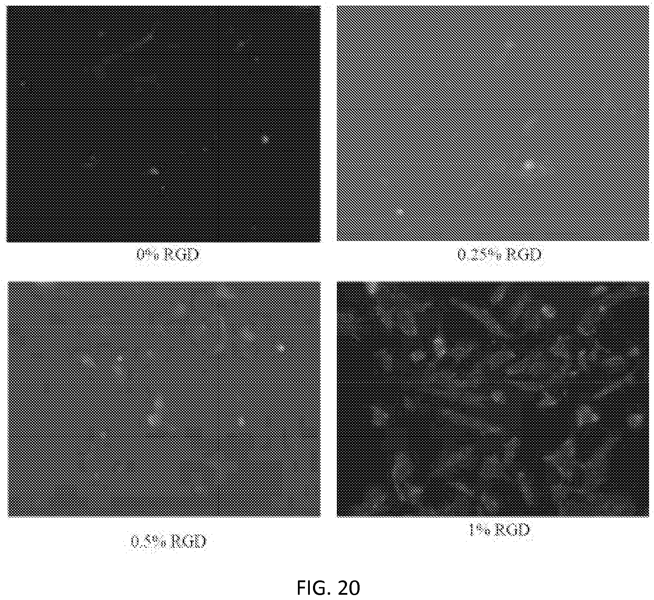

FIG. 20 demonstrates the effects of GRGDS density expression on liposomes in binding to HUVEC epithelial cells, whereby the binding of 0.4 mM DMPC:DMPG liposomes labeled with 1% NBD-PE fluorescence was evaluated for liposomes expressing 0%, 0.25%, 0.5%, or 1.0% PA-GRGDS. The POD device used is shown in FIG. 28.

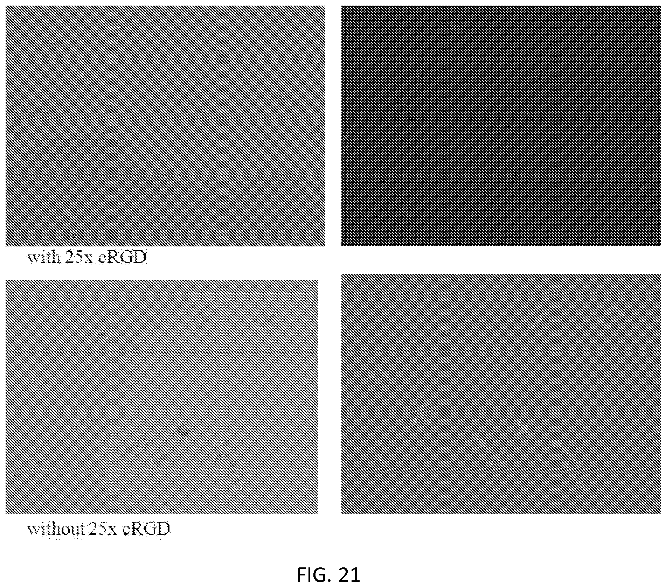

FIG. 21 demonstrates the binding of DMPC:DMPG liposomes with 1% NBD-PE and 1% PA-GRGDS is inhibited when incubated with free cRGD (25 mole excess) (top two panels), whereby the bottom two panels were done without free cRGD blocking. The POD device used is shown in FIG. 28.

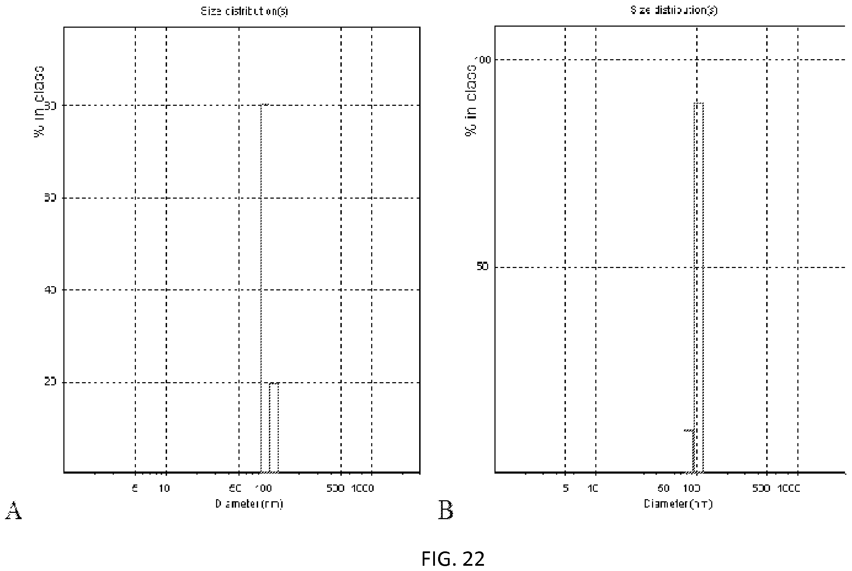

FIG. 22 are bar graphs demonstrating the targeted liposome size distribution does not significantly change before, A, (mean of 96.5 ± 6.1 nm) and after, B, (mean of 104.1 ± 4.9) being aerosolized. (P>0.05). The POD device used is shown in FIG. 28.

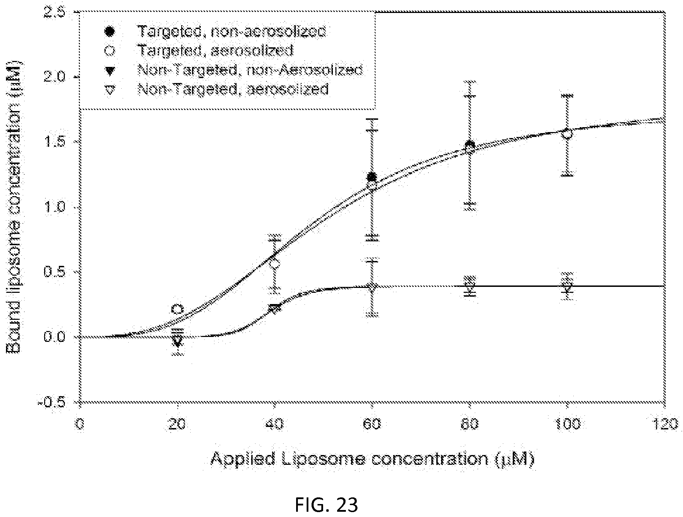

FIG. 23 is a graph demonstrating the RGD-expressed liposomes and the non-targeted liposomes binding affinity to integrin expressing LLC-PK1 epithelial cells does not significantly change before and after being aerosolized. The POD device used is shown in FIG. 28.

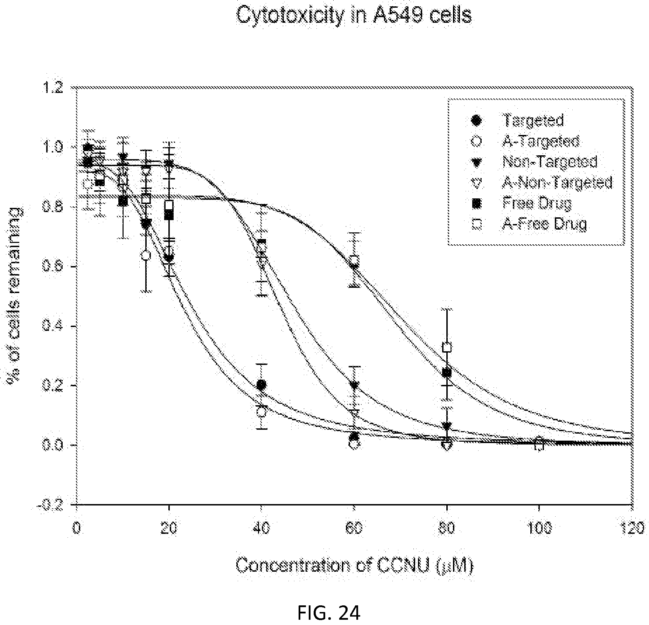

Fig. 24 is a graph demonstrating the cytotoxic effects of CCNU on A549 lung cancer cells when encapsulated in non-targeted DMPC:DMPG liposomes, RGD-expressed liposomes, or as free drug. The POD device used is shown in FIG. 28.

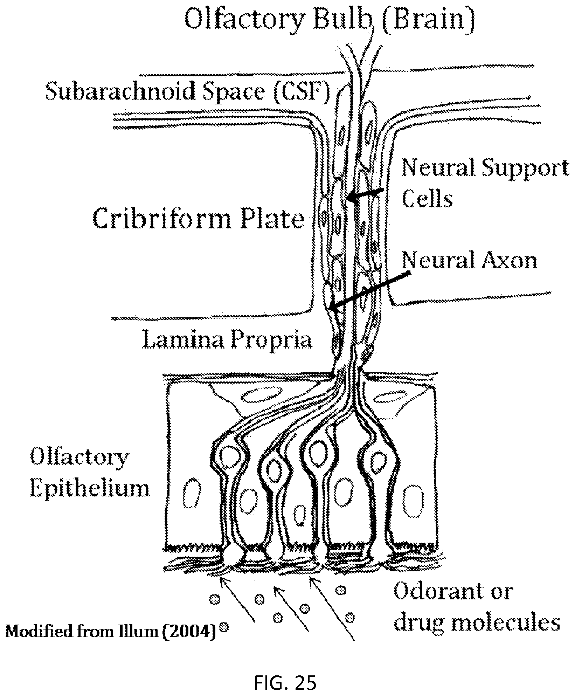

FIG. 25 is an illustration of the olfactory epithelium has direct connections to the CSF containing subarachnoid space and the brain. The POD device used is shown in FIG. 28.

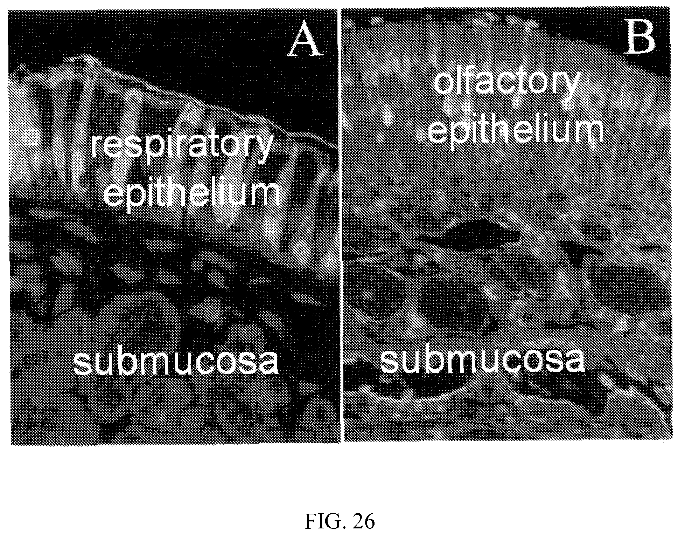

FIG. 26 demonstrates paracellular diffusion across the olfactory epithelium. The respiratory epithelium (A) is bound by tight junctions and limits passive diffusion to small lipophilic molecules, whereby a fluorescently labeled 5 kDa dextran molecule is unable to diffuse into the respiratory epithelium submucosa, whereby the olfactory epithelium (B) is more porous due to olfactory neurons penetrating the epithelium, and whereby such allows the fluorescent dextran to readily diffuse along paracellular pathways to the lamina propria of the olfactory epithelium. The POD device used is shown in FIG. 28.

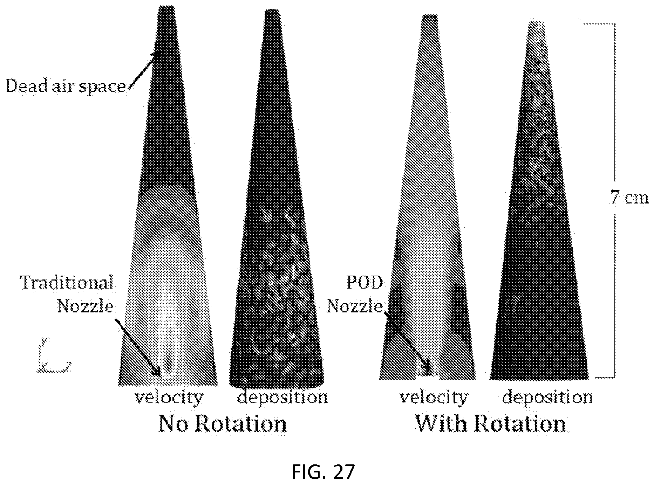

FIG. 27 illustrates a comparison of the CFD simulation of a simplex traditional style nozzle and a POD nozzle (shown in FIG. 28) that circumferential velocity penetrates further into the cone while the simplex aerosol is not able to penetrate the unmoving air in the top of the cone. Any apparent size differences may be due to picture.

FIG. 28 is a schematic illustration of another example of a pressurized olfactory delivery (POD) device, whereby the pressurized nitrogen is controlled by a pneumatic solenoid that releases the gas in increments of 0.1 seconds, and whereby the gas enters the nozzle outlet and mixes with the liquid dose that flows through the curved outlets producing a narrow flow with rotational velocity.

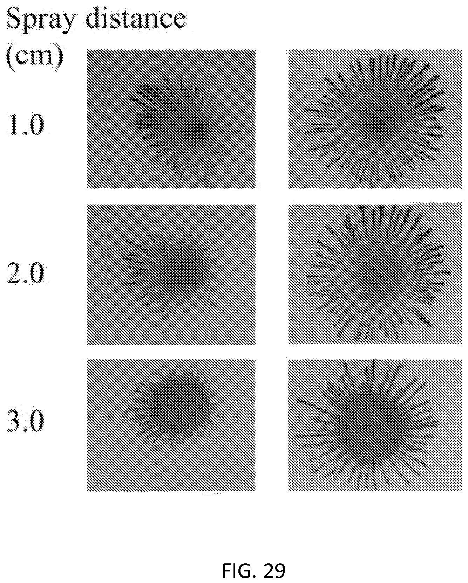

FIG. 29 demonstrates the vortical flow pattern from POD aerosol spray, whereby the vortical or centrifugal patterns dispensed from the POD (left) are compared to control nozzle (right) without vortical spray attachment, whereby the asymmetrical pattern of the dye rotates as the distance increases from the POD spray nozzle, and whereby the aerosol plume rotates at approximately 22.57cm. The POD device used is shown in FIG. 28.

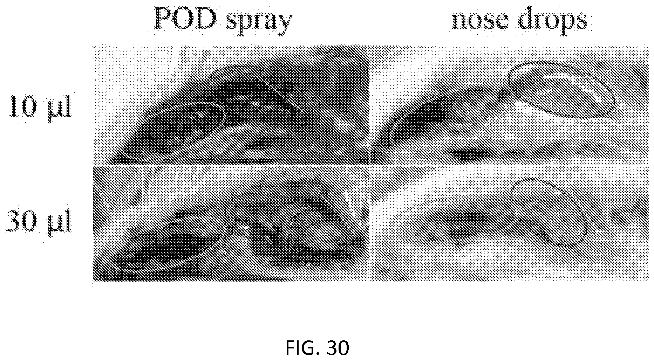

FIG. 30 shows dye deposition of POD or nose drops within rat nasal cavity, whereby deposition of dye in the rat nasal cavity using the POD spray device or nose drops is shown, whereby 10 $\mu$l (upper panels) or 30 $\mu$l (lower panels) of blue dye was administered to the rat nasal cavity using a single spray from the POD device at 20psi pressure (left panels) or nose drops administered in 5 ul drops every minute (right panels). The POD device used is shown in FIG. 28.

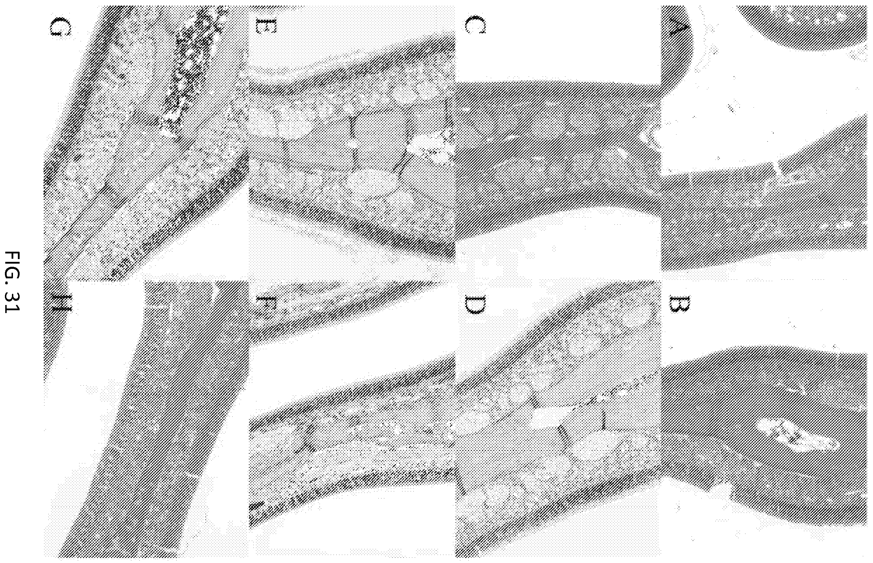

FIG. 31 show histopathology images after POD spray, whereby all images are from septum of nasal cavity in the olfactory region of rat nasal cavity, whereby A,B show control animals which received no POD spray, whereby C,D received POD spray with a driving pressure of 10 psi, E,F with a driving pressure of 20 psi, and G,H with a driving pressure of 30 psi. No histological damage was observed from the POD spray. The POD device used is shown in FIG. 28.

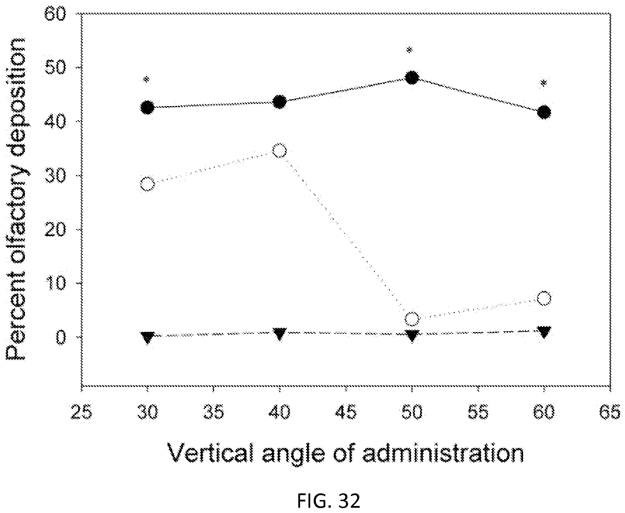

FIG. 32 is a graph illustrating olfactory deposition in a human nasal cavity model, whereby the various POD devices included rotation inducing nozzle (filled circles), a non-rotation inducing nozzle (empty circles), and a standard nasal pump (filled triangles) and were dispensed into a human nasal cavity model at several vertical angles, whereby the 50 $\mu$l doses were actuated with 45 psi of driving pressure for the POD device configurations and hand actuation for the nasal pump, whereby the POD device with a rotational aerosol output resulted in significantly higher deposition in the olfactory region at 30, 50, and 60° vertical angles compared to the POD device with a straight aerosol plume and resulted in a higher percentage of olfactory deposition at all angles tested compared to the nasal pump (* = P < 0.05 compared to POD with no rotational component in the aerosol). The POD device used is shown in FIG. 28.

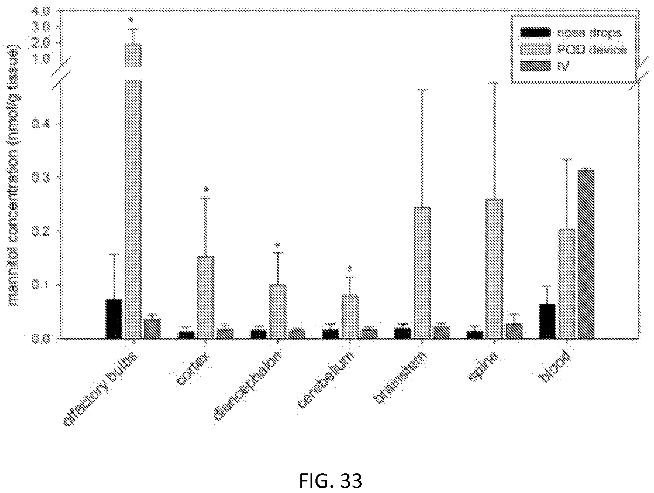

Fig. 33 is a bar graph illustrating brain concentrations of mannitol 30 minutes after a 0.2 mg dose, whereby the brain concentrations in the olfactory bulbs, cortex, diencephalon, and cerebellum were significantly greater when delivered near the cribriform plate with the POD spray than when delivered to the respiratory region with nose drops or systemically via IV. (* = p < 0.05). The POD device used is shown in FIG. 28.

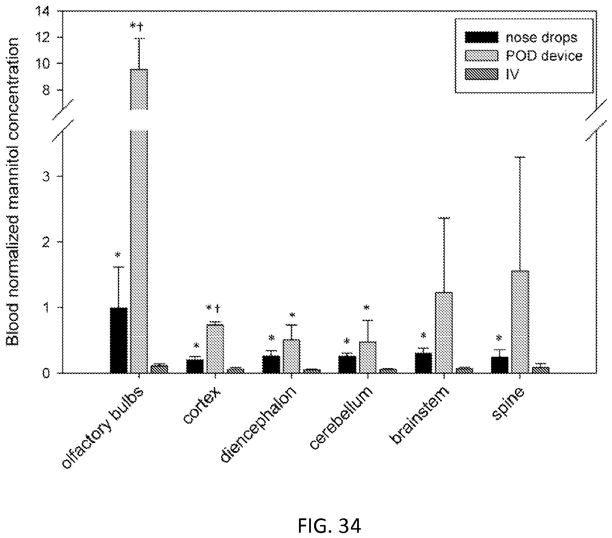

FIG. 34 is a bar graph demonstrating blood normalized brain concentrations of mannitol 30 minutes after a 0.2 mg dose, whereby the blood normalized brain concentrations after nose drop were significantly higher than after IV administration in all brain regions, whereby the blood normalized olfactory bulb and cortex mannitol concentrations after POD administration were significantly higher than after either nose drop or IV delivery while the diencephalon and cerebellum blood normalized concentrations were significantly greater than after IV delivery. (* = p < 0.05 compared with IV administration; † = p < 0.05 compared with nose drop administration). The POD device used is shown in FIG. 28.

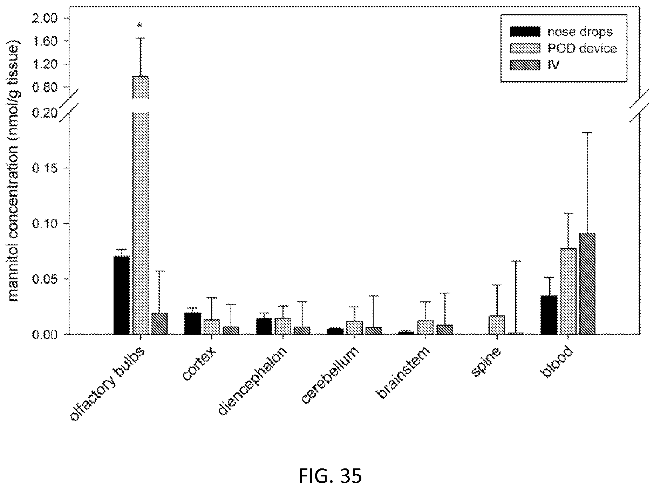

FIG. 35 is a bar graph illustrating brain concentrations of mannitol 150 minutes after a 0.2 mg dose, whereby the brain concentrations in the olfactory bulbs were significantly higher when delivered with POD than IV or nose drops (*) while the cerebellum and brainstem concentrations after nose drops were significantly lower than concentrations after POD spray or IV (+). (p < 0.05). The POD device used is shown in FIG. 28.

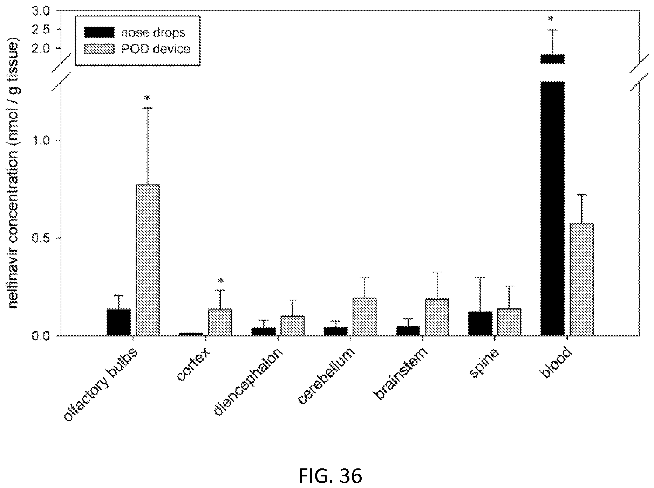

FIG. 36 is a bar graph illustrating brain concentrations of nelfinavir. The brain concentrations 30 minutes after a 0.14 mg dose of nelfinavir were significantly greater (* = p < 0.05) when delivered near the cribriform plate than when delivered to the respiratory region with nose drops. The POD device used is shown in FIG. 28.

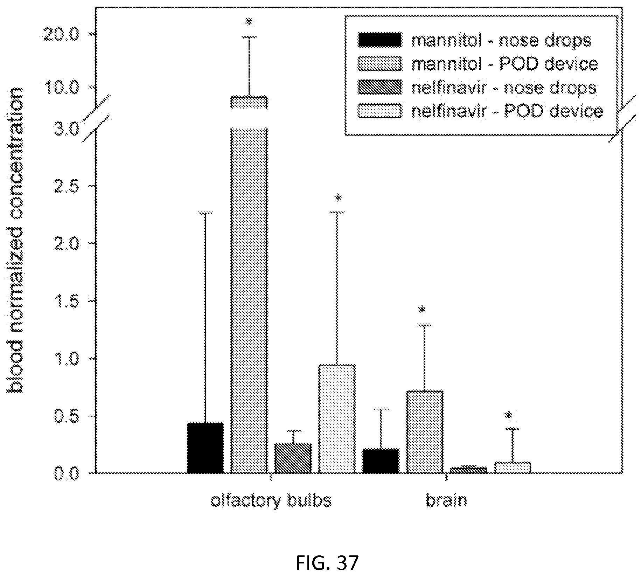

FIG. 37 is a bar graph illustrating blood normalized drug concentrations 30 minutes after delivery, whereby the blood normalized olfactory bulb and brain concentrations of nelfinavir and mannitol were significantly higher after delivery to the olfactory region with the POD spray compared to the nose drop treatments. (* = p < 0.05). The POD device used is shown in FIG. 28.

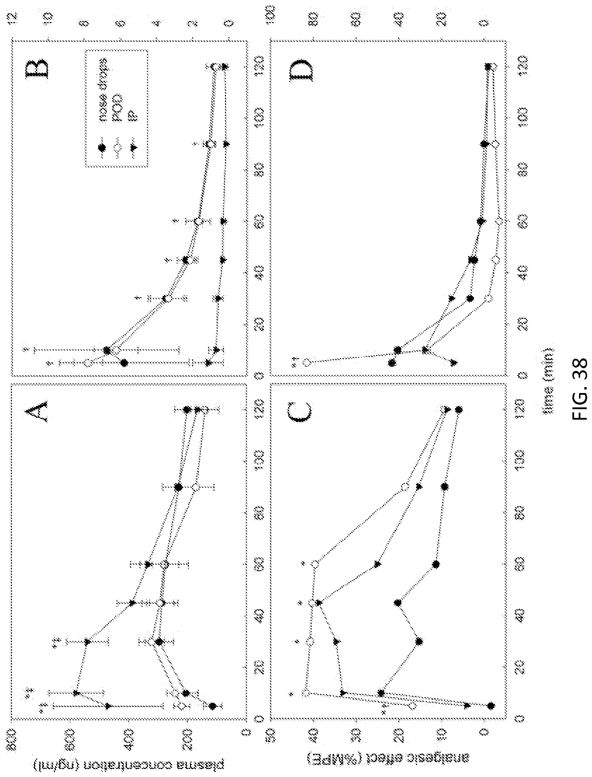

FIG. 38 includes graphs demonstrating the effects of POD administered 5.0 mg/kg morphine (A, C) and 15 μg/kg fentanyl (B, D) on plasma and analgesic time course, whereby IP morphine (A) had a significantly higher plasma concentration at time points up to 30 minutes while no difference was seen between plasma levels of POD and nose drop administered morphine, whereby, in contrast, the POD administered morphine to the cribriform plate region induced greater analgesic effect (C) than either nose drops or IP at 5 minutes and greater than nose drops at all time points up to 60 minutes, whereby IP administered 15 μg/kg fentanyl produced similar plasma levels (B) after either POD or nose drop administration while IP administration lead to significantly lower plasma concentrations, and whereby the analgesic effect of POD administered fentanyl (D), which produced a very strong analgesic effect, was significantly higher at the earliest time point (5 min). (* = P < 0.05 compared to nose drops; † = P < 0.05 compared to IP; ‡ = P < 0.05 compared to POD). The POD device used is shown in FIG. 28.

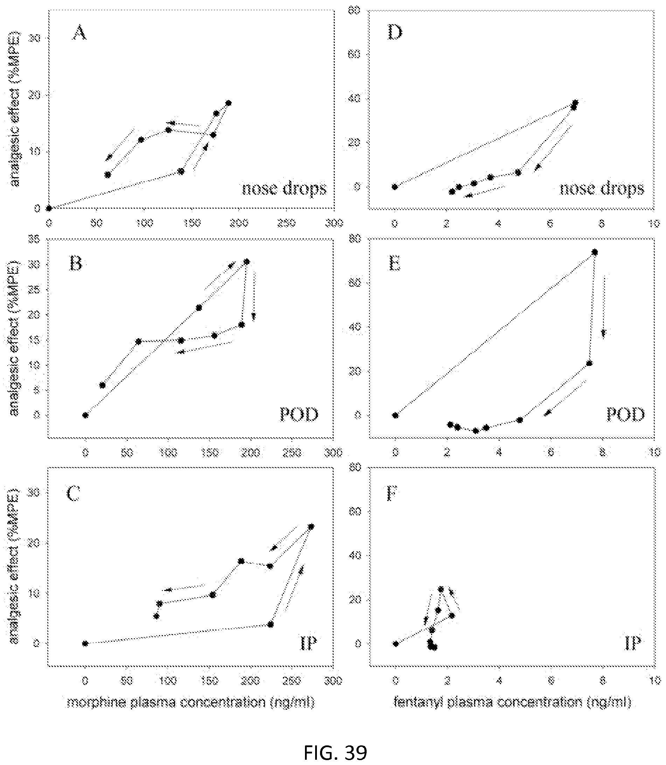

FIG. 39 includes graphs demonstrating plasma concentration vs analgesic effect plots after 2.5 mg/kg morphine and 15 μg/kg fentanyl, whereby the morphine panels (A-C) show a clear distinction between routes of delivery, whereby after nose drop and IP administration there is indication of counterclockwise hysteresis while after POD administration to the olfactory region (B) there is indication of a clockwise hysteresis within the first 60 minutes, whereby the fentanyl panels (D-F) also show a distinction between delivery methods while nose drops and IP (D,F) show no clear hysteresis, and whereby after POD administration (E) there is indication of a clockwise hysteresis at the first time point. The POD device used is shown in FIG. 28.

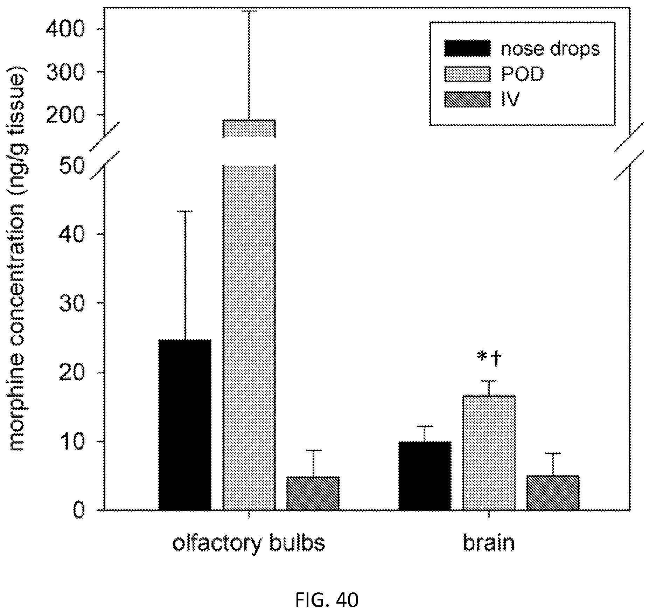

FIG. 40 is a bar graph demonstrating brain concentrations of morphine 5 minutes after a 2.5 mg/kg dose, whereby total brain concentrations of morphine were higher after delivery to the olfactory region with POD than either nose drop delivery to the respiratory region via nose drops or systemic administration via IP injection. (* = P < 0.05 compared to nose drops; † = P < 0.05 compared to IP). The POD device used is shown in FIG. 28.

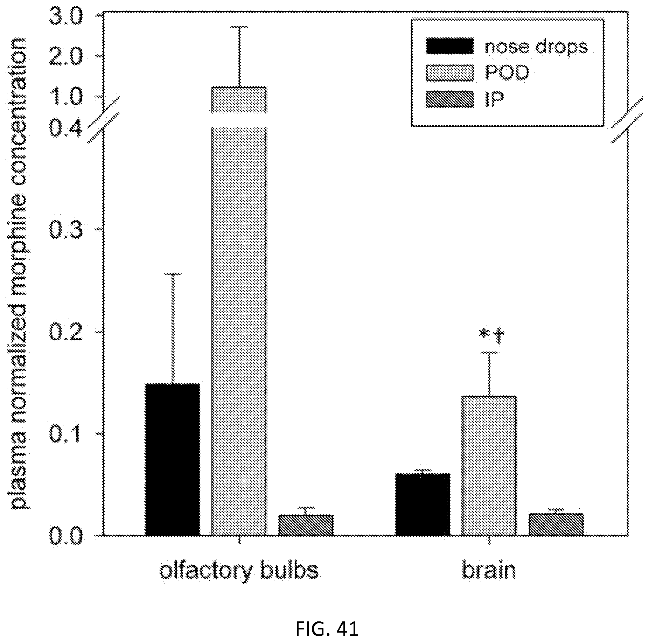

FIG. 41 is a bar graph demonstrating plasma normalized morphine brain concentrations 5 minutes after delivery, whereby the plasma normalized concentrations of morphine in the brain were significantly higher when delivered to the cribriform plate region of the nasal cavity via POD spray than when delivered to the respiratory region via nose drops or systemically via IP. (* = P < 0.05 compared to nose drops; † = P < 0.05 compared to IP). The POD device used is shown in FIG. 28.

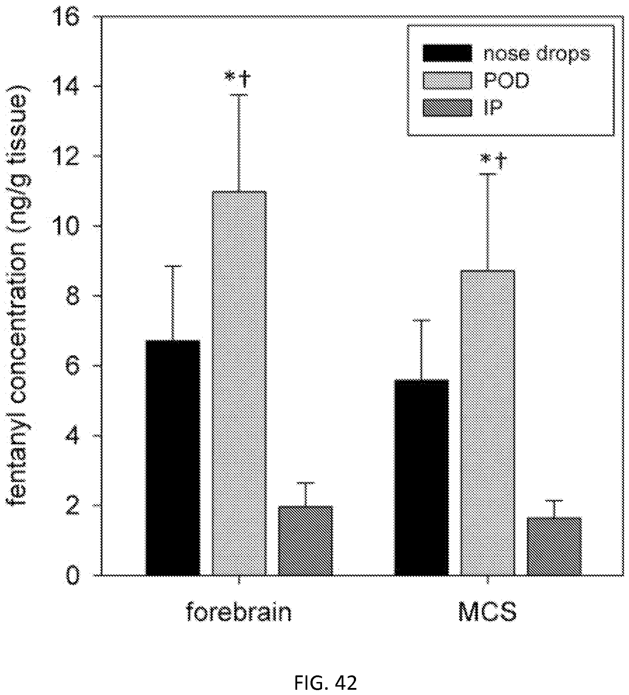

FIG. 42 is a bar graph demonstrating that POD administration leads to increased brain concentrations 5 minutes after administration, whereby fentanyl concentrations in the forebrain and the midbrain, cerebellum and upper cervical spinal cord (MCS) were significantly higher when delivered to the cribriform plate region of the nasal cavity via POD spray than when delivered to the respiratory region via nose drops or systemically via POD spray. (* = P < 0.05 compared to nose drops; † = P < 0.05 compared to IP). The POD device used is shown in FIG. 28.

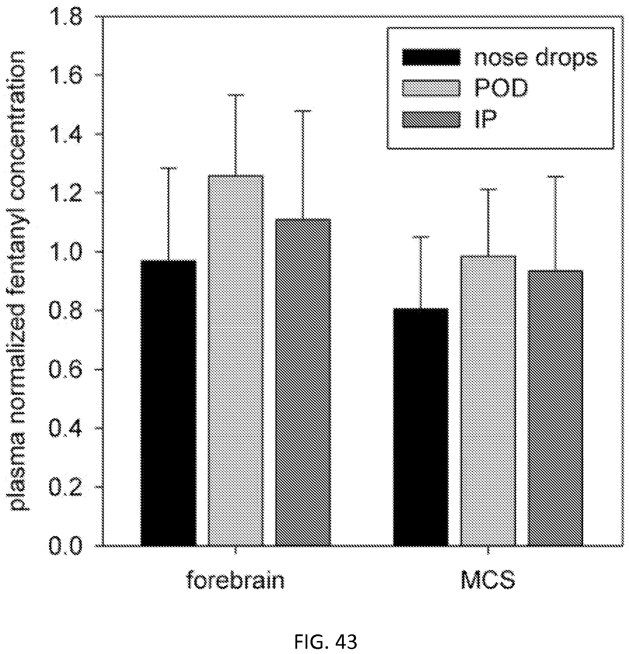

FIG. 43 is a bar graph demonstrating plasma normalized fentanyl brain concentrations 5 minutes after delivery, whereby the blood normalized concentrations of fentanyl in the brain are not significantly different in any of the three delivery methods. The POD device used is shown in FIG. 28.

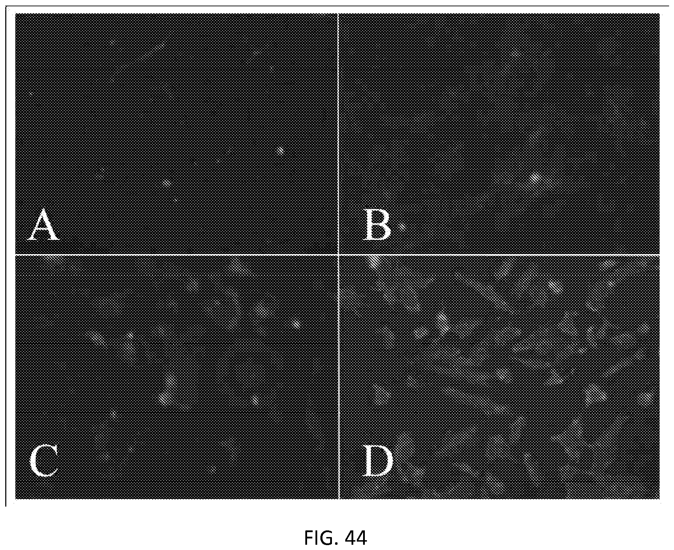

FIG. 44 shows liposome cell binging with varying density of RGD, whereby the RGD peptide density in the liposome membrane correlates with increased targeting, whereby binding of 0.4 mM DMPC:DMPG liposomes expressing 0%, 0.25%, 0.5%, or 1.0% PA-GRGDS, and whereby the liposomes were incubated for 30 minutes with αVβ3 integrin expressing HUVEC cells. The POD device used is shown in FIG. 28.

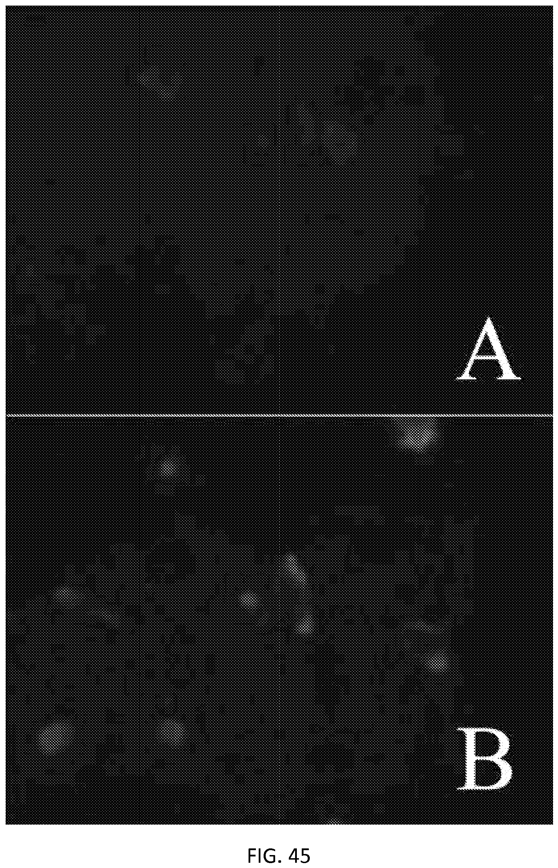

FIG. 45 shows RGD Liposome binding in vitro, whereby the binding of targeted DMPC:DMPG liposomes with 1% NBD-PE and 1% PA-GRGDS is inhibited when incubated with free cRGD in 25 mole excess (A) compared to those incubated with no cRGD (B). The POD device used is shown in FIG. 28.

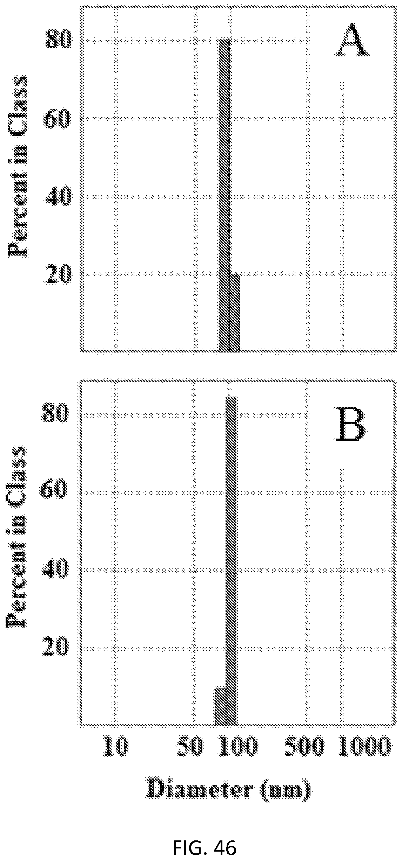

FIG. 46 are bar graphs demonstrating liposome sizing before and after aerosolization, whereby the RGD-liposome size distribution did not significantly change before, A, (mean of 96.5 ± 6.1 nm) and after, B, (mean of 104.1 ± 4.9) being aerosolized at 5 psi. The POD device used is shown in FIG. 28.

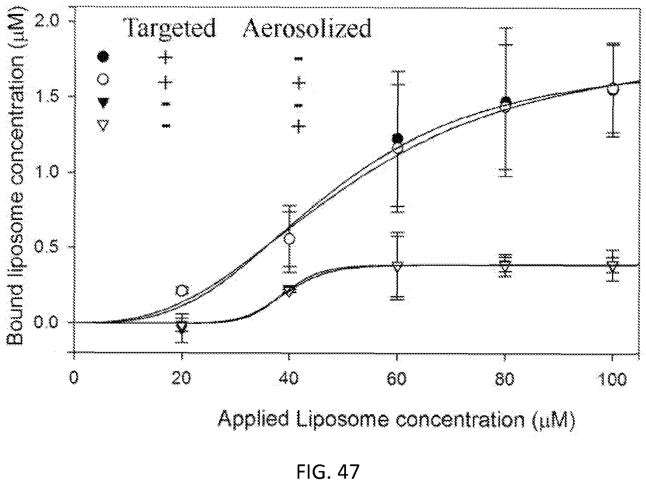

FIG. 47 is a graph demonstrating RGD-liposome quantitative cell binding in vitro, whereby after a 30 minute incubation the binding affinity to integrin expressing LLC-PK1 epithelial cells was greater with RGD-liposomes (= P<0.05) compared to non-targeted liposomes, and whereby the cell binding did not significantly change before and after being aerosolized for either the RGD-liposomes and the non-targeted liposomes (P>0.05). The POD device used is shown in FIG. 28.

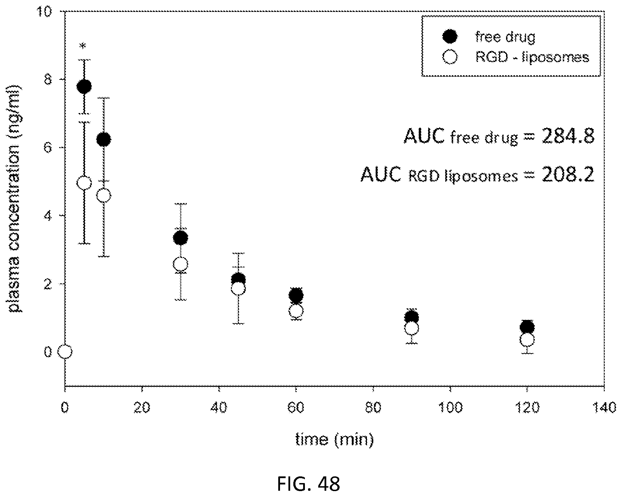

FIG. 48 is a graph demonstrating fentanyl plasma concentration over time after delivery of free fentanyl or fentanyl incorporated in RGD-liposome, whereby free fentanyl (closed circles) and fentanyl incorporated in RGD liposomes were nasally administered and plasma samples were drawn over a 120 minute period, and whereby incorporation of fentanyl into the RGD liposomes did not change the overall plasma curve shape but did lead to a non-significantly lower AUC and a significantly lower plasma concentration at 5 minutes after dose. (* = $p < 0.05$). The POD device used is shown in FIG. 28.

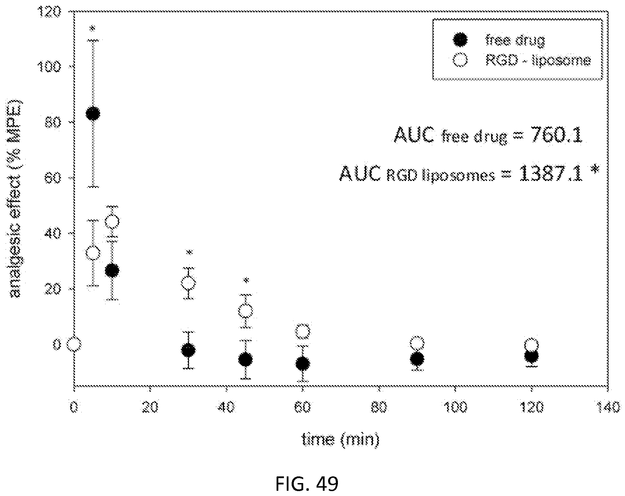

FIG. 49 is a graph demonstrating analgesic effect after nasal delivery of either free fentanyl or RGD-liposome incorporated fentanyl, whereby free fentanyl (closed circles) and fentanyl incorporated in RGD liposomes were nasally administered and the tail flick test was performed over a 120 minute period, and whereby incorporation of fentanyl into the RGD liposomes resulted in a lower analgesic effect at 5 minutes and significantly higher analgesic levels at 30 and 45 minutes as well as a significantly higher $AUC_{effect}$. (* = $p < 0.05$). The POD device used is shown in FIG. 28.

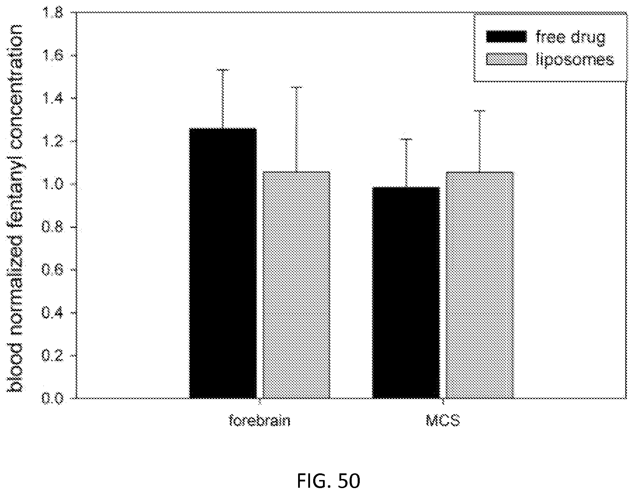

FIG. 50 is a bar graph demonstrating blood normalized fentanyl brain concentrations 5 minutes after administration, whereby the fentanyl brain concentrations when normalized by blood were not significantly different between the free drug fentanyl and the fentanyl incorporated into the RGD-liposomes, whereby it is implied that the RGD-liposomes did not result in fentanyl distributing directly from the nasal cavity to the brain, and that, in both cases, fentanyl was primarily, if not completely, transported from the nasal cavity to the blood stream to the brain. The POD device used is shown in FIG. 28.

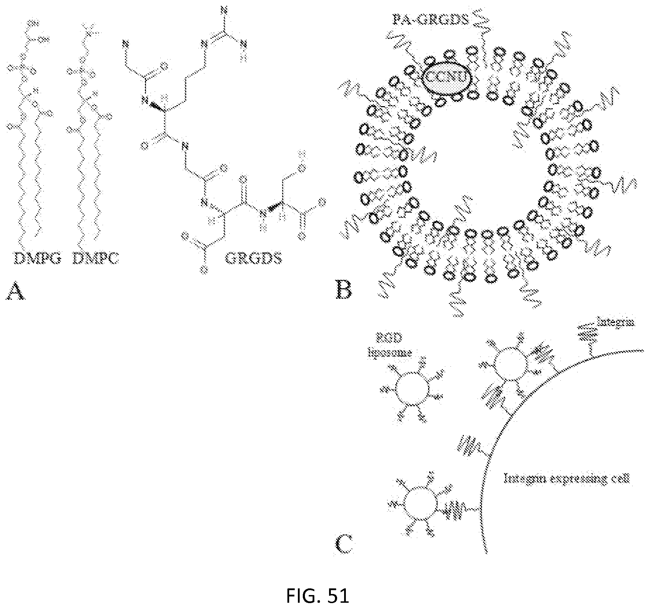

FIG. 51 illustrates an overview of the RGD-liposome and interaction with the cell, whereby (A) the liposome were made with a 1:1 mixture of DMPC:DMPG phospholipids and with palmitic acid (PA) linked GRGDS used as the targeting peptide, whereby (B) the liposomes formed an enclosed bilayer with PA embedded into the bilayer exposing the GRGDS peptide to the outside environment, whereby lipophilic drugs such as CCNU can be embedded into the lipid bilayer for drug delivery, whereby (C) the RGD-liposomes demonstrated an increased binding to integrin expressing cells, and whereby the RGD liposomes are demonstrated to be suitable for aerosol delivery as aerosolization was shown to have little impact to no material impact on the structural integrity of the liposome or targeting ability of the RGD-liposomes.

## DETAILED DESCRIPTION OF EXEMPLARY EMBODIMENTS

Intra-nasal administration of pharmacologics and compounds is one route for direct access to the brain and the central nervous system (CNS). At least two obstacles exist that limit the use of the nasal route for CNS drug delivery in humans. First, no existing nasal delivery device deposits a majority fraction of drug in the portion of the olfactory region where the drug is directly absorbed into the CNS. Second, clearance mechanisms within the nasal cavity limit CNS uptake of drug deposited at this region. By increasing olfactory region deposition and residence time, the fraction of nasally administered drug delivered directly to the CNS is substantially improved leading to a faster time of therapeutic onset and a reduction in systemic toxicities.

## PATENT APPLICATION DEFINITIONS

As used herein, the term "administering" refers to any mode of transferring, delivering, introducing or transporting drug or other agent to a subject.

As used herein, the term "%" when used without qualification (as with w/v, v/v, or w/w) means % weight-in-volume for solutions of solids in liquids (w/v), % weight-in-volume for solutions of gases in liquids (w/v), % volume-in-volume for solutions of liquids in liquids (v/v) and weight-in-weight for mixtures of solids and semisolids (w/w) (Remington's Pharmaceutical Sciences (2005); 21.sup.st Edition, Troy, David B. Ed. Lippincott, Williams and Wilkins).

As used herein, the term "effective amount" refers to that amount which, when administered to a patient (e.g., a mammal) for a period of time is sufficient to cause an intended effect or physiological outcome.

The term "treatment of a disease" as used herein refers to the care of a patient (biological matter) having developed the disease, condition or disorder. The purpose of treatment is to diminish the negative effects of the disease, condition or disorder. Treatment includes the administration of the effective compounds to eliminate or control the disease, condition or disorder as well as to modify or reduce the symptoms associated with the disease, condition or disorder.

Certain methods in which the present invention finds utility include pre-treating a biological material, e.g., a patient, prior to a disease insult. The POD device may also deliver a drug for treating a seizure.

In various other examples, the present invention finds utility in the treatment of neurodegenerative diseases and hyperproliferative disorders, and in the treatment of immune disorders. In various other examples, the biological condition is any one or combination of the following: neurological disease or disorders (epilepsy and seizure disorders), cardiovascular disease, metabolic disease, infectious disease, lung disease, genetic disease, autoimmune disease, and immune-related disease.

The term "biological matter" refers to any living biological material, including cells, tissues, organs, and/or organisms, and any combination thereof. It is contemplated that the present invention will find utility in methods practiced on a part of an organism (such as in cells, in tissue, and/or in one or more organs), whether that part remains within the organism or is removed from the organism, or on the whole organism. Moreover, it is contemplated in the context of cells and tissues that homogenous and heterogeneous cell populations may be used. The term "in vivo biological matter" refers to biological matter that is in vivo, i.e., still within or attached to an organism. Moreover, the term "biological matter" will be understood as synonymous with the term "biological material." One or more cells, tissues, or organs may be separate from an organism. The term "isolated" can be used to describe such biological matter. It is contemplated that the methods in which the present invention finds utility may be practiced on in vivo and/or isolated biological matter.

A cell treated according to a method in which the present invention finds utility may be eukaryotic or prokaryotic. For example, the cell may be eukaryotic. More particularly, , the cell may be a mammalian cell. Mammalian cells include, but are not limited to those from a human, monkey, mouse, rat, rabbit, hamster, goat, pig, dog, cat, ferret, cow, sheep, or horse. Moreover, cells may be diploid, but, in some cases, the cells are haploid (sex cells). Additionally, cells may be polyploid, aneuploid, or anucleate. The cell can be from a particular tissue or organ, such as heart, lung, kidney, liver, bone marrow, pancreas, skin, bone, vein, artery, cornea, blood, small intestine, large intestine, brain, spinal cord, smooth muscle, skeletal muscle, ovary, testis, uterus, and umbilical cord. The cell can for example be characterized as one of the following cell types: platelet, myelocyte, erythrocyte, lymphocyte, adipocyte, fibroblast, epithelial cell, endothelial cell, smooth muscle cell, skeletal muscle cell, endocrine cell, glial cell, neuron, secretory cell, barrier function cell, contractile cell, absorptive cell, mucosal cell, limbus cell (from cornea), stem cell (totipotent, pluripotent or multipotent), unfertilized or fertilized oocyte, or sperm.

The terms "tissue" and "organ" are used according to their ordinary and plain meanings. Though tissue is composed of cells, it will be understood that the term "tissue" refers to an aggregate of similar cells forming a definite kind of structural material. Moreover, an organ is a particular type of tissue. The tissue or organ may be "isolated," meaning that it is not located within an organism.

Biological material may be exposed to the pharmaceutical compositions of the current invention for about, at least, at least about, or at most about 30 seconds, 1, 2, 3, 4, 5, 10, 15, 20, 25, 30, 35, 40, 45, 50, 55 minutes, 1, 2, 3, 4, 5, 6, 7, 8, 9, 10, 11, 12, 13, 14, 15, 16, 17, 18, 19, 20, 21 , 22, 23, 24 hours, 1, 2, 3, 4, 5, 6, 7 days or more, and any range or combination therein.

An amount of time may be about, at least about, or at most about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 minutes, 1, 2, 3, 4, 5, 6, 7, 8, 9, 10, 11, 12, 13, 14, 15, 16, 17, 18, 19, 20, 21, 22, 23, 24 hours, 1, 2, 3, 4, 5, 6, 7 days, 1, 2, 3, 4, 5 weeks, and/or 1, 2, 3, 4, 5, 6, 7, 8, 9, 10, 11, 12 months, or any range derivable therein.

Volumes of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 mls or liters, or any range therein, may be administered overall or in a single session.

A distribution route exists between the nasal cavity and the brain that is both a rapid and non-specific extracellular pathway that relies on the fluid filled channels surrounding the olfactory neurons to deliver substances to the CNS. The space between the olfactory neurons and the supporting olfactory ensheathing cells is filled with fluid. These fluid filled channels create a direct connection between the olfactory epithelium of the nasal cavity and the subarachnoid space surrounding the olfactory bulb.

In addition, paracellular pathways in the olfactory epithelium develop when the olfactory nerve cells die out and are replaced by olfactory ensheathing cells that differentiate into mature olfactory neurons. The present application describes a pressurized olfactory delivery (POD) device specifically that targets the olfactory epithelium within the nasal cavity. An advantage of the POD device is that its use produces no detectable epithelial damage or discomfort. The POD device also includes scaled up versions for non-human primate and human use, achieving $57.0 \pm 7.2$ % aerosol deposition on the olfactory region within the nose. In one experiment, administration of a water soluble marker, mannitol (log P = -3.1) in a rodent model with the POD device provided a 3.4 fold increase in brain-to-blood ratio, compared to identical dose given with a typical nose drop approach. The nose drops primarily deposits drug on the respiratory, not olfactory epithelium.

Evaluation with a hydrophobic drug, nelfinavir (log P = 4.1) provided a 2.1 fold advantage in brain-to-blood ratio with POD administration, compared to nose drops. Two opioid analgesic drugs were tested and evaluated, morphine and fentanyl, with the POD device and compared with nose drops. Compared to nose drops, POD administration of morphine resulted in significantly higher overall therapeutic effects ($AUC_{effect}$) without a significant increase in plasma drug exposure ($AUC_{plasma}$). POD delivery of fentanyl on the other hand, lead to a faster (5 min vs 10 min) and more intense analgesic compared to nose drops but did not increase $AUC_{effect}$.

The POD device is advantageously useful for delivering integrin-targeted liposome formulation with fentanyl which significantly increased the $AUC_{effect}$ of fentanyl analgesia. The POD device and liposome-formulated drug combination are useful for advantageously improving delivery of CNS drugs. This combination provides effective and safe delivery of existing CNS targeted drugs and compounds under development.

A pressurized olfactory drug delivery (POD) device 10 is shown in FIG. 1. The device 10 comprises a pressurized tank 20 suitable for storing a pressurized fluid, such as a compressed gas or propellant. The compressed gas may be compressed air, nitrogen, or any other suitable pharmaceutical gas. The propellant may be a pressurized fluid such as chlorofluorocarbon (CFC) or hydrofluoroalkane (HFA). The pressurized tank 20 is in fluid connection/communication by tubing 22 to a pneumatic solenoid 30. The pneumatic solenoid 30 is in fluid connection/communication by tubing 32 to an air chamber 42. The air chamber 42 is connected via an internal compartment to to a nasal delivery device 40 having an applicator 50 with an orifice suitable for discharging an aerosol spray into the nasal cavity of an animal subject.

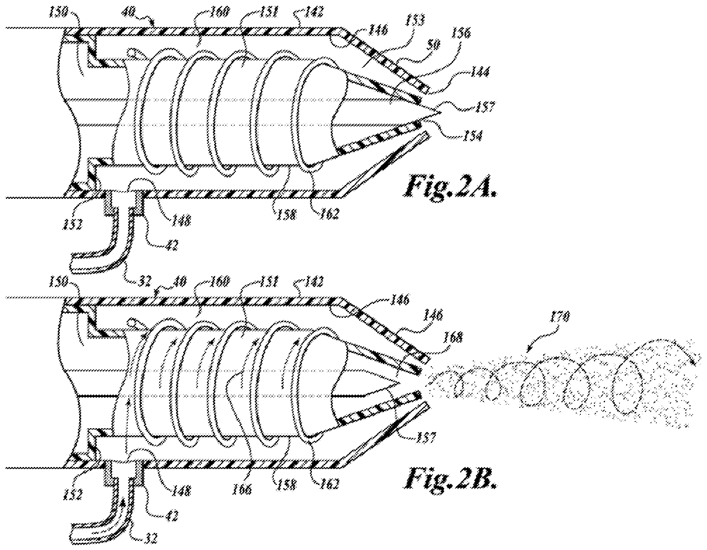

Referring to FIG. 2A, the nasal delivery device 40, the nasal delivery device 40 includes a generally elongate tubular-shaped housing 142 having an exterior and interior and an opening or orifice (i.e., aperture) 144 at the nasal proximal end that is radially-aligned about the longitudinal axis of the housing. The housing 142 is closed at the nasal distal end. The housing 142 may be cylindrical, tubular or any other suitable shape. The housing 142 also includes a conically-shaped applicator 50 disposed at the proximal end adjacent to (and surrounding) the orifice 144. The housing 142 surrounds a generally tubular, cylindrically-shaped fluid reservoir 150 that extends along a portion of the longitudinal axis of the housing.

The fluid reservoir 150 has a proximal orifice 154 disposed near the orifice 144 of the housing 142, the proximal orifice 154 having a diameter smaller than the orifice 144 and being generally radially-aligned about the longitudinal axis of the housing 142. The proximal end is conically-shaped adjacent to and surrounding the orifice 154. The proximal portion 151 of the fluid reservoir 150 has a diameter narrower than the diameter of the housing 142, thereby forming a channel 153 extending from the distal portion of the reservoir 152 to the orifice 154. The distal portion 152 of the fluid reservoir 150 has a wider diameter such that the exterior surface 158 of the fluid reservoir contacts the interior surface 146 of the housing 142 creating a seal that prevents flow of pressurized gas in a distal direction.

The fluid reservoir 150 also includes an elongate needle 156 having a long axis that runs along the longitudinal axis of the housing 142. The elongate needle 156 moveably disposed within the interior proximal portion of the fluid reservoir 150. The proximal end (or tip) 157 of the needle 156 is configured to seal the orifice 154 of the fluid reservoir 150. The fluid reservoir 150 including a vent (not shown) to prevent a vacuum that would increase the pressure required to remove fluid from the orifice 154.

The housing 142 also including a spin chamber 160 defined by the space between the interior surface 146 of the housing 142 and the exterior surface 158 of the fluid reservoir 150. The housing 142 also includes a compressed gas inlet 148 in communication with the spin chamber 160 and in fluid connection to the pneumatic solenoid 30. The spin chamber 160 further including a coiled wire 162 wrapped around the exterior 158 of the fluid chamber. The coiled wire 162 being helical (or corkscrew) in shape and extending from the gas inlet 148 to the proximal orifice 154.

Referring to FIGS. 1 and 2B, the POD device 10 may be used to deliver a pharmaceutical compound to the olfactory epithelium. A predetermined discharge pressure is set for the pressurized nasal spray. The pneumatic solenoid 30 is activated by a programmable timer to release the pressurized gas from tank 20 for a predetermined amount of time. The pressurized gas released from the tank 20 travels through tubing 22, 32 to the air chamber 42 and through the air chamber 42 into the gas inlet 148 of the housing 142 thereby entering the spin chamber 160 of the nasal delivery device 40. The pressurized gas that enters the spin chamber 160 engages the coiled wire 162 causing the pressurized gas 166 to flow around the exterior surface 158 of the fluid reservoir in a helical or corkscrew shaped path such that the gas acquires a circumferential helical velocity or vortex like velocity having circumferential vector and axial vector components. Circumferential velocity also includes tangential velocity, helical velocity, vortical velocity, and like components.

Referring to FIG. 2B, when the solenoid 30 is activated, the elongate needle 156 disposed within the fluid reservoir 150 retracts from the orifice 154 thereby providing a narrow opening 168 for the fluid within the fluid reservoir 150 to escape. As the pressurized gas 166 leaves the orifice 144 a partial vacuum is generated forcing fluid out of the reservoir 150 through the orifice 154. The fluid is aerosolized by the narrow gap 168. The aerosolized spray 170 is discharged from the nasal spray device 40 as a spray plume having a circumferential velocity and axial velocity as the spray plume enters the nasal cavity. The circumferential velocity of the aerosol spray advantageously penetrates the upper nasal cavity causing direct deposition of aerosolized therapeutic compounds on the olfactory epithelium.

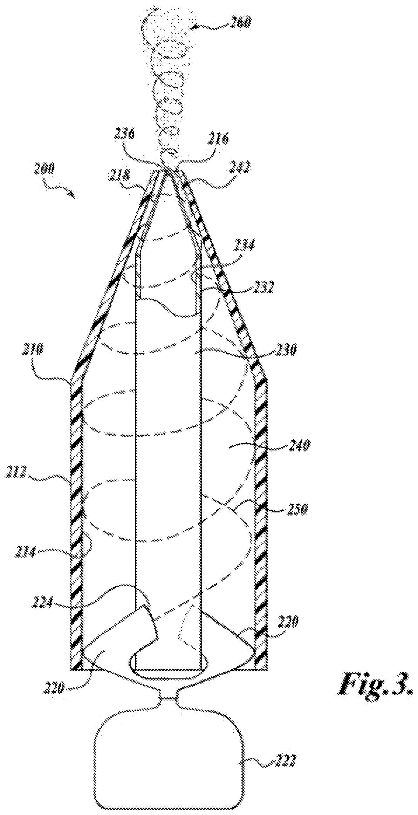

Referring to FIG. 3 a pressurized olfactory delivery device 200 includes a tubular housing 210 having a central longitudinal axis, an exterior surface 212, an interior surface 214, and an orifice 216 at the proximal end 218 thereof. The proximal end 218 of the housing 210 is conically-shaped to facilitate discharge of a pressurized nasal spray into the nasal cavity.

The device 200 may further include a cylindrical fluid reservoir 230 radially-disposed about the longitudinal axis and enclosed by the housing 210. The fluid reservoir 230 has an exterior surface 232 and interior surface 234 as well as an orifice 236 at the proximal end disposed near the orifice 216 of the housing. The orifice 236 having a diameter smaller than the orifice 216 and being generally radially-aligned about the longitudinal axis of the housing 210. The fluid reservoir 230 having a diameter narrower than the diameter of the housing 210. The proximal end of the fluid reservoir 230 being conically-shaped adjacent to and surrounding the orifice 236. The fluid reservoir 230 having a vent (not shown) to prevent a vacuum that would increase the pressure required to remove fluid from the orifice 236.

The distal end of the housing 210 having one or more nozzles 220 in fluid connection/communication to a compressed fluid container 222. The compressed fluid may be compressed air, compressed nitrogen, or a compressed propellant such as CFC or HFA, or any other suitable pharmaceutical propellant. The compressed fluid container may have a metering device (not shown) to deliver a predetermined amount of fluid, gas or propellant upon activation. The compressed fluid container may be a metered dose inhaler (MDI). The proximal end of the nozzles 220 having openings 224 that open into a spin chamber 240 defined by the space between the exterior surface 232 of the fluid reservoir 230 and the interior surface 214 of the housing 210. The nozzles 220 are configured such that the openings 224 discharge the compressed fluid in a circumferential and axial direction thereby establishing a circumferential velocity to the pressurized fluid.

With reference to FIG. 3, the POD device 200 may be used to deliver a pharmaceutical compound to the olfactory epithelium. A user actuates the pressurized gas container 222 to release a predetermined amount of pressurized gas 250 into the spin chamber 240. The pressurized gas acquires a circumferential velocity having axial and circumferential components, and it exits the orifice 216. As the pressurized gas 250 exits the orifice 216, it creates a partial vacuum which forces fluid out of the reservoir 230 through the orifice 236. The fluid is aerosolized by the narrow gap 242 defined by the interior surface 214 of the orifice 216 and exterior surface 232 of the fluid reservoir 230. The aerosolized spray is discharged from the nasal spray device 200 having a circumferential velocity as the aerosol spray 260 enters the nasal cavity.

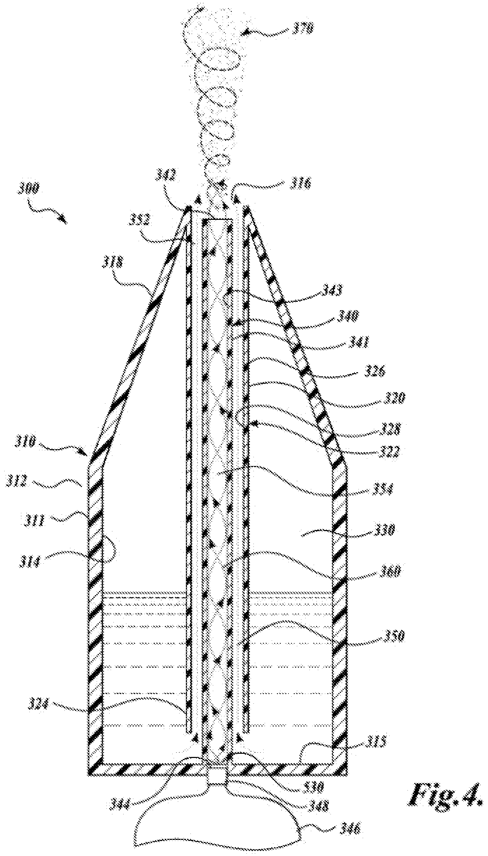

Referring to FIG. 4, the pressurized drug delivery device 300 includes a generally tubular cylindrical housing 310 having a central longitudinal axis, an outer wall 311 having an exterior surface 312, an interior surface 314, and an orifice 316 at the nasal proximal end 318. The proximal end 318 of the housing 310 is conically-shaped to enhance user comfort and facilitate discharge of a nasal spray into the nasal cavity.

The housing 310 also includes an inner wall 320 defining an axially-aligned inner cylinder 322 open at both ends (and connected to the proximal end 318 of the housing 310 at the orifice 316) and having a distal open end 324 disposed near the interior surface 315 of the distal wall of the housing defining a gap sufficient for receiving a fluid between the distal open end 324 and the interior surface 315 of the wall. The inner cylinder 322 has a diameter less than the diameter of the outer wall 311 thereby defining a space between the exterior surface 326 of the cylinder 322 and the interior surface 314 of the outer wall 311 of the housing 310 that serves as a fluid reservoir 330 suitable for storing a liquid pharmaceutical composition.

Referring to FIG. 4, the device 300 further includes an inner cylinder 340 having an exterior surface 341 and interior surface 343, wherein the longitudinal axis of the inner cylinder 340 is axially-aligned with the longitudinal axis of the housing 310. The inner cylinder 340 extending from an orifice 342 disposed at the nasal proximal end of opening 344 at the distal end of the housing 310 that is in fluid connection/communication to a pressurized fluid container 346. The diameter of the inner cylinder 340 is less than the diameter of the inner cylinder 322 thereby defining a tubular channel 350 extending from distal open end 324 of the inner cylinder 322 to orifice 316.

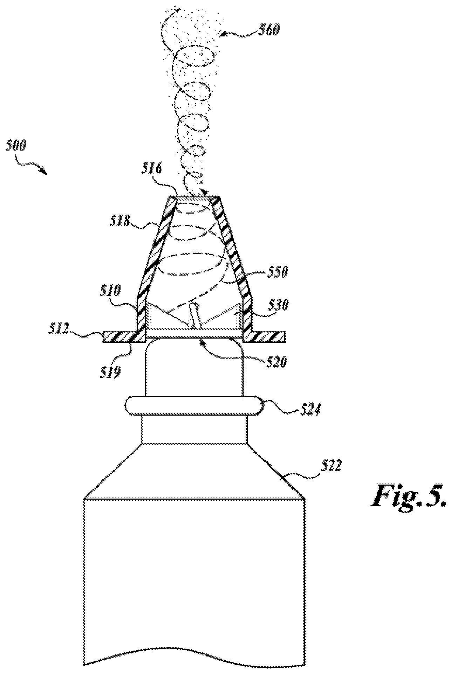

A metering device 348 is in fluid connection/communication to the pressurized fluid container 346 at one end and to opening 344 in the distal end of the second inner cylinder 340 at the other end. The interior surface 343 of the cylinder 340 defines a channel 354 that functions as a spin chamber. The channel 354 being connected at one end to the metering device 348 and at the other end to orifice 342. A plurality of discharge vents 530 are disposed between the metering device 348 and the interior of channel 354. Discharge vents 530 being in fluid connection with the metering device 348. The plurality of discharge vents 530 (which are also shown in FIGS. 5 and 6) are configured to discharge a pressurized nasal spray having a circumferential axial velocity. In an alternative embodiment, the pressurized fluid container 346 can be an MDI.

With continued reference to FIG. 4, POD device 300 may be used to deliver a pharmaceutical compound to the olfactory epithelium. A user (e.g., the patient, or other individual)) actuates the pressurized fluid container 346 and metering device 348 to release a predetermined amount of pressurized fluid 360 into inner cylinder 340. The pressurized fluid 360 is a pressurized gas. The pressurized gas 360 passes through metering device 348 and discharge vents 530 thereby acquiring a circumferential axial velocity, and entering the spin chamber 354 before exiting second orifice 342. As the pressurized fluid 360 leaves the second orifice 342 it creates a partial vacuum which forces fluid up the tubular channel 350. The fluid is aerosolized by the narrow gap 352 defined by the interior surface 328 of inner cylinder 322 and exterior surface 341 of inner cylinder 340. Aerosolized spray is discharged from orifice 316 as a spray plume 370 having a circumferential velocity as aerosol spray 370 enters the nasal cavity. The fluid reservoir 330 has a vent (not shown) to prevent a vacuum that would increase the pressure required to remove fluid from orifice 342.

Referring now to FIG. 5, POD device 500 includes a cylindrical tubular housing 510 having a longitudinal axis and outlet 516 located at proximal end 518 thereof. Proximal end 518 of the housing 510 may be conically-shaped and functioning as a nose cone to enhance user comfort. The housing 510 also includes flanges 512 disposed near the distal end that aid in operation of the device by a user. Distal end 519 of housing 510 is connected to proximal end member 520 of pressurized fluid container 522 having metering device 524. Metering device 524 delivers a predetermined amount of pharmaceutical fluid, gas or propellant upon activation. Alternatively, the pressurized fluid container 522 is an MDI. The metering device may also deliver a predetermined dose of a therapeutic compound (i.e., API) in a pharmaceutical composition containing the pressurized fluid and other inactive pharmaceutical ingredients. The device 500 also includes a plurality of aerosol discharge vents 530 configured to discharge a pressurized nasal spray having a circumferential velocity.

Referring to FIGS. 6A-C, each aerosol discharge vents 530 is elongate rectangular or ovoid in cross section. A slit like or slot like proximal opening (i.e., aperture) 532 is disposed opposite distal opening 534. The vents 530 are configured and oriented generally radially, wherein the angle of the proximal distal axis of each vent 530 is oblique to the longitudinal axis of the device 500 such that an aerosol discharged from the vent has circumferential and axial components. As shown in FIG. 6C, distal opening 534 of each vent is in fluid connection/communication to the proximal end member 520 of the pressurized gas container 522. Each vent 530 may be designed and constructed such that the structure of the vent 530 extends above the surface created by the proximal end 520 of the pressurized gas container 522. Alternatively, the vents 530 may include openings (i.e., apertures) in proximal end 520 of the pressurized gas container 522 or other suitable configurations.

Referring FIGS. 5 and 6A-C, POD device 500 is used to deliver a pharmaceutical compound (i.e., API) to the olfactory epithelium. A user actuates the pressurized gas container 522 to release a predetermined amount of pressurized gas through the metering device 524 into distal opening 534 of each vent 530. The pressurized gas exits the proximal opening 532 of each vent as an aerosol 550 having an axial velocity and a radial velocity (only one discharged aerosol 550 is shown for simplicity).

The discharged aerosols 550 exiting each vent converge into a single pressurized nasal spray pattern 560 having a circumferential velocity that then exits the device through the outlet 516. The proximal end 518 of the housing 510 functions as a nose cone to aid the user (e.g., patient, nurse, doctor or the like) in aligning the device with the nostril to deliver the pressurized nasal spray (having a circumferential velocity) into the nasal cavity.

The housing 510 is not required to produce the circumferential velocity of the spray. The housing 510 illustrated in FIG. is provided for user convenience. As described in Example 3, flow simulation of the spray pattern produced by the plurality of vents 530 (or outlets) similar to the structure shown in FIG. 6 produces a narrow spray plume having circumferential and axial velocity components. A device having a plurality of vents 530 (as illustrated in FIG. 6) does not require a spin chamber or other like chamber at the proximal end of the device for producing a spray plume having circumferential velocity.

The circumferential velocity created by the plurality of vents also advantageously generates a spray plume capable of penetrating the upper regions of the nasal cavity compared to a spray plume produced without circumferential velocity, and be much more narrow than the wide spray plume produced by a device having a single aerosol source with vortical flow, which further facilitates the spray to penetrate the upper nasal cavity and contact the olfactory epithelium.

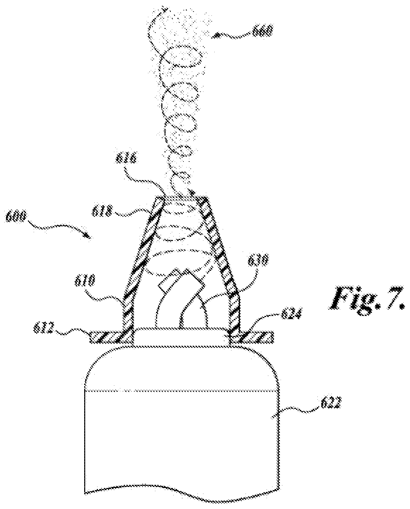

Referring to FIG. 7, pressurized drug delivery device 600 shares many of the features and structures shown in FIG. 5 and includes a housing 610 having an outlet 616 surrounded by a conical proximal end 618 that functions as a nose cone. Instead of a plurality of discharge vents 530, device 600 includes a plurality of discharge nozzles 630 in fluid connection/communication to pressurized fluid container 622 including a metering device 624. Alternatively, pressurized fluid container 622 is an MDI.

Each nozzle 630 is configured to discharge an aerosol spray in an axial and circumferential direction/orientation such that each individual aerosol spray converges into a single pressurized spray pattern 660 having a circumferential velocity. The housing 610 further includes flanges 612 disposed near the distal end to aid user operation of the device. Again, housing 610 is not required to produce the circumferential velocity of the spray.

In the examples described in FIGS. 5-7, the pressurized fluid container contains a mixture of compressed fluid and one or more therapeutic compounds (i.e., APIs). The compressed fluid may be any non-toxic propellant (i.e., pharmaceutical), such as compressed air, or a pressurized propellant (e.g., chlorofluorocarbon (CFC) or hydrofluoroalkane (HFA)).

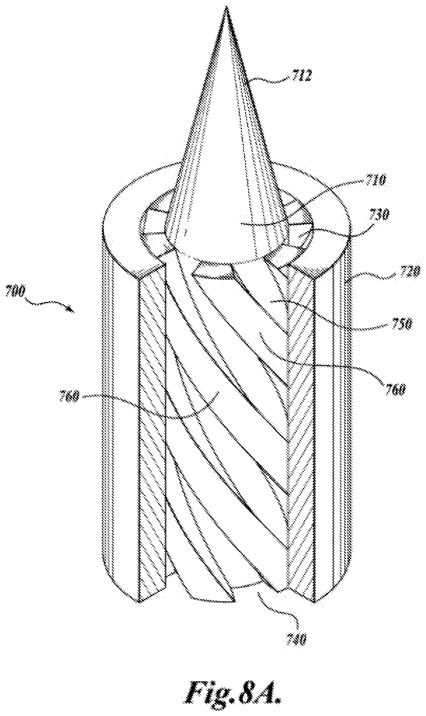

Referring to FIG. 8A-E, delivery device (or nozzle) 700 for use with a pressurized drug delivery device 700 is cylindrically-shaped defining a longitudinal axis. Nozzle 700 also has nasal-proximal and nasal-distal ends, an inner cylinder portion 710, an outer cylinder portion 720, and a plurality of outlet orifices 730. The plurality of outlets 730 are radially-disposed around the longitudinal axis of the nozzle. The outlets 730 may be symmetrically and radially disposed around the longitudinal axis of the nozzle. The plurality of outlets 730 may also be disposed on a surface at the nasal-proximal end of the nozzle. In another example, at least three outlet orifices 730 are provided. The example illustrated in FIGS. 8A-E includes six outlet orifices 730.

Referring to the examples described in FIGS. 8A-E, inner cylinder 710 has a conical extension 712 disposed at the nasal-proximal end to aid the user in directing a pressurized nasal spray into the nasal cavity. Conical extension 712 is optional and not required for the operation of the nozzle. The nasal-proximal end of the nozzle may also be protected by a nose cone (not shown) to enhance the comfort of the user.

With reference to FIG. 8A-E, each outlet orifice 730 is connected to an inlet orifice 740 by an axial channel 750 being corkscrew, helical, or spiral in shape or another suitable shape. Each channel is an enclosed volume or space defined by lateral surfaces 762 of corkscrew shaped axial members 760 that extend along and rotate about the longitudinal axis of the nozzle, the exterior surface 714 of the inner cylinder portion 710, and the interior surface 722 of the outer cylinder portion 720.

The nozzle 700 may be constructed by machining threads or grooves in the exterior surface 714 of the inner cylinder portion 710 to produce the corkscrew-shaped axial members 760 thereof. Alternatively, the nozzle 700 may be constructed by machining threads or grooves in the interior surface 722 of the outer cylinder portion 720 to produce the corkscrew shaped axial members 760 thereof. It is understood that the nozzle is not limited by the method of producing or manufacturing the nozzle. Other suitable method of manufacture know in the art may also be used to make the nozzle 700.

In another example, the cross sectional area of the channel decreases from distal to proximal such that the outlets 730 are smaller than the inlets 740, thereby providing acceleration to a pressurized fluid entering the channel. The channels may be round, square, rectangular, ovoid, or any other suitable shape in cross section. The outlets 730 are configured such that a pressurized fluid discharged from the outlet has an axial velocity and a circumferential velocity. The outlets 730 are further configured to atomize the pressurized fluid into an aerosol spray as the pressurized fluid exits the outlets 730. The outlets 730 may also be configured such that the aerosol spray discharged from the outlet is further directed radially inwardly at an oblique angle toward the longitudinal axis of the nozzle 700.

Referring to FIGS. 8A-E, nozzle 700 is operated to deliver a pharmaceutical compound (i.e., API) to the olfactory epithelium of a human in need of treatment thereof or an animal subject. Nozzle 700 is attached to a pressurized fluid container (not shown) containing a mixture of pressurized fluid and a therapeutic compound (i.e., API) or pharmaceutical composition containing one or more APIs and inactive pharmaceutical ingredients along with the pressurized fluid.

In other examples, the pressurized fluid container includes a metering device that provides a predetermined amount of pressurized fluid containing a predetermined dosage of a therapeutic compound upon activation. The pressurized fluid container may be an MDI. The pressurized fluid may be a compressed gas, such as compressed air, or a suitable propellant known in the art. The pressurized fluid discharges from the pressurized fluid container entering the plurality of inlets 740 and traveling through axial channels 750 and exiting outlets 730.

The pressurized fluid is atomized into an aerosol spray discharging as it exits outlets 730. After exiting the outlets 730, each individual aerosol spray discharge converges into a single spray pattern having circumferential velocity. The nasal-proximal end of the nozzle is partially inserted into the nasal cavity of the human patient or animal subject. The single spray plume maintains circumferential velocity as it exits the device and enters the nasal cavity. Nozzle 700 has the advantage that no spin chamber or other type of chamber is required to induce the circumferential axial velocity of the aerosol spray plume, whereby the circumferential flow is induced by the configuration of axial channels 750 and outlets 730.

The circumferential velocity created by plurality of outlets 730 has the added advantages that the spray plume is capable of penetrating the upper regions of the nasal cavity compared to a spray plume produced without circumferential velocity, and is much narrower than the wide spray plume produced by a device having a single aerosol source with vortical flow. The narrow spray plume, in combination with the circumferential velocity provided by the nozzle 700, allows the aerosolized spray to penetrate the upper nasal cavity and deposit therapeutic compounds on the olfactory epithelium. Representative methods for measuring the diameter of the spray plume are described in Example 1.

An exemplary method of specific delivery to the olfactory epithelium (confirmed with computational fluid dynamic and deposition within a model human nasal cavity) utilizes a spray nozzle that has three or more outlet orifices positioned such that the aerosol patterns from each converge into a single spray pattern having a rotational component while maintaining a narrow spray angle. The resulting spray pattern possesses a substantial rotational component sufficient to displace residual air of the upper nasal cavity resulting in significantly reduced back pressure compared to that experienced with other designs. In addition, the narrow turbulent spray avoids becoming entrained in the natural breath flow towards the lower nasal cavity, trachea and lungs. Increased deposition onto the olfactory epithelium and reduced deposition onto the respiratory epithelium is achieved. Computational fluid dynamics simulations have been incorporated into the POD device to specifically target the olfactory region.

The deposition data demonstrates that the POD device is also capable of depositing drug on the olfactory region in humans. A POD device with a nozzle insert having straight shafts produced enhanced rotational aerosol flow that increased olfactory deposition.

The nasal aerosol device (POD) targets the olfactory region. This device is effective and safe, and it deposits > 40% of dose on the olfactory region in a human nasal cavity model. This device may also be used experimentally to study the impact of olfactory deposition on drug distribution for a wide variety of drugs and molecules.

The device may for example discharge a plurality of particles having an average or mean diameter in the range of about 1 to about 100 micrometers, about 5 to about 50 micrometers, about 5 to about 30 micrometers, about 5 to about 25 micrometers, about 5 to about 20 micrometers, about 5 to about 15 micrometers, or about 10 to about 15 micrometers. For example, at least 70%, at least 80%, at least 90% or at least 95% of the particles produced by the device have a diameter between about 5 and 25 micrometers. The majority of the particles discharged by the device may in the range of about 5 to 20 micrometers. The average particle size, thus, may be in the range of 5-20 micrometers.

The nasal delivery device can be used to deposit numerous types of therapeutic pharmaceutical compounds (i.e., active pharmaceutical ingredients) and compositions on the olfactory epithelium, including neurological, analgesic, anti-viral and cancer treatment compounds. Compounds that can be delivered include, but are not limited to, compounds comprising small molecular weight synthetic organic pharmaceuticals, peptide and protein therapeutic compounds, antibodies and antibody fragments, aptamer compounds, and DNA and RNA compounds. The compounds can be delivered as part of a composition or formulation to aid in stability or penetration of the olfactory epithelium. According to the present invention, the pharmaceutical formulation contains one or more APIs as specified in claim 1 and a pharmaceutical carrier system containing one or more inactive pharmaceutical ingredients.

The inactive pharmaceutical ingredients in the carrier system form an aerosol. They may include stabilizers, preservatives, additives, adjuvants, compressed air or other suitable gases, or other suitable inactive pharmaceutical ingredients formulated with the therapeutic compound (i.e., API). Pharmaceutically suitable carrier systems (e.g., inhalation carrier systems) include the pharmaceutically suitable inactive ingredients known in the art for use in aerosol dosage forms, such as (but not limited to) aerosol propellants (e.g., hydrofluoroalkane propellants), surfactants, additives, suspension agents, solvents, stabilizers and the like.

Various FDA-approved inhalation inactive ingredients are found at the FDA's "The Inactive Ingredients Database" that contains inactive ingredients specifically intended as such by the manufacturer, whereby inactive ingredients can also be considered active ingredients under certain circumstances, according to the definition of an active ingredient given in 21 CFR 210.3(b)(7). Alcohol is a good example of an ingredient that may be considered either active or inactive depending on the product formulation.

According to 21 CFR 210.3(b)(7), an active ingredient (i.e., API) is any component of a drug product intended to furnish pharmacological activity or other direct effect in the diagnosis, cure, mitigation, treatment, or prevention of disease, or to affect the structure or any function of the body of humans or other animals. APIs include those components of the product that may undergo chemical change during the manufacture of the drug product and be present in the drug product in a modified form intended to furnish the specified activity or effect. As used herein, a kit (also referred to as a dosage form) is a packaged collection of related material.

As used herein, an aerosol is a drug product that is packaged under pressure and contains the API and carrier system that are released upon activation of an appropriate valve system intended for topical application to the olfactory epithelium; or, an aerosol spray may be an aerosol product which utilizes a compressed gas as the propellant to provide the force necessary to expel the product as a wet spray, applicable to solutions of medicinal agents in aqueous solvents.

Targeted nanoparticles may be used to increase the binding, penetration, and/or absorption across the olfactory epithelium. Targeted nanoparticles are capable of significantly reducing clearance problems by increasing the residence time on the epithelium and increasing penetration across the epithelium. Various types of nanoparticles may be used to stabilize and improve pharmacokinetic parameters of drugs, such as bioavailability. Nanoparticle formulations and forms include micelles, liposomes, biopolymers, drug polymeric conjugates, carbon nanotubes, biocompatible natural or synthetic derivatives and the like. Ligand or receptor recognition molecules may also be included on nanoparticle surfaces for targeting particular tissue(s) or facilitating crossing of anatomical barriers.

Additional inactive pharmaceutical ingredients include molecules that are useful for intranasal delivery include peptide mimics such as RGD (arginine-glycine-aspartic acid) polymers or cyclic peptides that bind to a matrix protein (such as integrin) and that promote trans epithelial transport. Additional inactive pharmaceutical ingredients include nutrients (such as glucose and amino acid) for their respective transporters, whereby transport-capable recognition molecules are useful in the intranasal carrier system to enhance residence time and penetration of drug deposited by the POD device and to improve delivery of API to brain and cell tissues of the central nervous system. Increasing the residence time alone on the olfactory epithelium significantly improves the fraction of drug transport across the epithelium, thus increasing direct uptake into the brain.

Liposomes were used as a biocompatible and pressure stable nanocarrier. A small validated RGD peptide targeted to integrin was expressed on olfactory epithelial cells to construct targeted nanoparticles. The RGD conjugated to palmitic fatty acyl chain and was used as the peptide in the targeted nanoparticles. RGD is a three amino acid repeating sequence that binds to integrin, which is a stable extracellular matrix protein. As seen in FIG. 13, RGD-liposomes bind significantly more readily to epithelium cells than non-targeted liposomes. Incorporation of nasally delivered API into liposomes increases the residence time on the epithelium, which increases uptake into the brain. Olfactory nasal tissues included those freshly isolated from a healthy primate *m. nemestrina.*

Following is a description of the integrin targeted liposomal formulation: DMPC:DMPG (1 :1 m/m) liposomal formulation, with 1 mole % palmitylated peptide GRGDS (referred to as RGD-liposomes), showed improved binding to epithelial cells compared to non-targeted liposomes. Incorporation of an anticancer drug, CCNU, the RGD-liposomal formulation has improved cytotoxicity due to an increased drug accumulation. This RGD-liposome formulation also shows no sign of disruption when aerosolized using the POD aerosol device producing an average aerodynamic particle diameter of 10.5 $\mu$m.

A RGD-expressed liposomal formulation for aerosolized delivery has been synthesized and characterized. These liposomes are physically intact and retain integrin targeting as well activity of anticancer drug CCNU after aerosol ization.

The invention finds utility in a method for depositing one or more therapeutic compounds (i.e., APIs) as specified in claim 1 on the olfactory epithelium within the nasal cavity of a human patient in need of treatment thereof or animal subject. The method may include administering the therapeutic compound from the pressurized nasal spray device (i.e., POD) into the nasal cavity, wherein the POD device includes the aerosol outlet adapted to discharge a pressurized spray containing the API, wherein the pressurized spray has the circumferential velocity as it exits the outlet and enters the nasal cavity.

The method may also include administering the pressurized fluid containing the one or more therapeutic compounds (APIs) from the POD device into the nasal cavity, wherein the device includes the plurality of outlets adapted to discharge the plurality of pressurized aerosol sprays containing the API, wherein the plurality of pressurized aerosol sprays converging into a single spray plume having a circumferential velocity upon exiting the POD device. Each outlet of the device is located at the nasal-proximal most end of the POD device and discharges the aerosol spray having a circumferential velocity directly into the nasal cavity.

The method may include administering a plurality of particles to the nasal cavity, wherein the plurality of particles have an average or mean diameter in the range of about 1 to about 100 micrometers, about 5 to about 50 micrometers, about 5 to about 30 micrometers, about 5 to about 25 micrometers, about 5 to about 20 micrometers, about 5 to about 15 micrometers, or about 10 to about 15 micrometers. For example, the aerosol spray contains particles having an average or mean diameter in the range of 5 to 25 micrometers compose at least 70%, at least 80%, at least 90% or at least 95% of the aerosol spray; or the majority of the particles administered by the method may be in the range of about 5 to 20 micrometers.

A POD device may be a metered dose inhaler (MDI), which releases a predetermined amount (i.e., weight or volume) of the pressurized fluid containing a predetermined metered dose of the API and carrier system upon activation or actuation of the MDI metering valve. The method delivers at least about 40% of the predetermined amount of the pressurized fluid entering the nasal cavity as an aerosol spray to the olfactory epithelium. The method is capable of delivering higher concentrations of API in the brain as compared to blood.

The API used in the method is provided as a component of the pharmaceutical composition/formulation according to the invention, which may contain various conventional inactive pharmaceutical ingredients such as stabilizers, preservatives, additives or the like. The API in the pharmaceutical composition/formulation may also be formulated as colloids, nanoparticles, liposomes, micelles, or other suspensions.

While not being bound by theory, the POD devices described herein and methods of use thereof are believed to show enhanced penetration of an aerosol spray into the upper nasal cavity by displacing the resident or residual air volume located in the upper naval cavity. As a result, a larger fraction of the API is deposited directly on the olfactory epithelium while also reducing the amount of the API deposited on the respiratory epithelium, esophagus, stomach and/or lungs. Another advantage of the POD devices is a reduction in the back pressure required to deliver drugs to the olfactory epithelium as compared to known nasal drug delivery devices that deliver a narrow spray plume lacking a corresponding centrifugal velocity component.

EXAMPLES

Example 1

Examples using therapeutic compounds other than those of claim 1 are for illustration only.

This example describes various functional parameters of the device illustrated in FIGS. 1 and 2.

The spray rate was tested by varying the driving pressure from 1 to 6 pounds per square inch and the diameter of the orifice 154. The spray rates were reproducible and within the desired range for human application, namely less than 50 microliters per second.

FIG. 9 shows the particle size distribution when water was sprayed from the device into viscous oil at a distance of 2 cm and 4 psi, and the resulting droplet diameters were measured using a microscope with size analysis software.

A total of 199 measurements were made. The distribution shows that the device produces particles having diameters of from 5 to greater than 50 microns, and that the majority of the particle diameters are between 5 and 20 micrometers, with an average diameter of 11.2 microns. The size distribution obtained by this method of atomization is therefore desirable for nasal spray applications.

FIG. 10 shows the penetration of an aerosolized blue dye into the nasal cavity of rats using the device illustrated in FIGS. 1 and 2 compared to the penetration of nose drops. Rats have a maximum naval cavity distance of about 2.5 cm. Increasing the air pressure of the device increases the penetration into the nasal cavity and coverage of the olfactory epithelium. The nasal drops resulted in no deposition on the olfactory epithelium, while the 3 psi spray from the POD device resulted in approximately 15% deposition on the olfactory epithelium, and the 4 psi spray from the POD device resulted in approximately 40% deposition on the olfactory epithelium. The results presented in FIG. 10 indicate that between 3-5 psi, a maximum penetration in nasal cavity is achieved to produce an optimal result. Higher pressures were untested but could lead to even deeper penetration into the nasal cavity.

FIG. 11 shows the spray pattern produced by the device using a blue dye marker sprayed out of the device at various distances from a piece of paper. The left hand side of FIG. 11 illustrates the circumferential flow as the angle of the majority of the dye shifts radially as the distance from the nozzle changes. The right hand side of FIG. 11 illustrates the symmetrical pattern produced by a spray nozzle that does not impart a circumferential velocity to the aerosol spray.

Example 2

Table 1 shows the delivery of the antiviral drug nelfinavir to different brain regions in using rats as a mammal model using nose drops (which approximates nasal distribution with a standard nasal spray) or the POD device illustrated in FIGS. 1 and 2. 30 minutes after delivery, the POD device delivered 42.7% of the drug dose present in the nasal spray to the olfactory epithelium compared to 4.7% of the dose delivered by nose drops. The drug concentrations were higher in various brain regions and lower in the blood when delivered using the POD device.

Table 1: Distribution of nelfinavir in rats 30 minutes after delivery via nose drops or using a pressurized olfactory drug delivery device of the present disclosure (Nelfinavir concentration, nmol/g tissue).

|  | drops | POD Device |
| --- | --- | --- |
| olfactory bulbs | 0.137 ± 0.104 | 0.409 ± 0.057 |
| cortex | 0.011 ± 0.003 | 0.083 ± 0.008 |
| diencephalon | 0.069 ± 0.027 | 0.205 ± 0.02 |
| cerebellum | 0.071 ± 0.008 | 0.302 ± 0.073 |
| brainstem | 0.087 ± 0.026 | 0.117 ± 0.052 |
| blood | 0.0159 ± 0.025 | 0.053 ± 0.010 |
| olfactory delivery | 4.7% | 42.7% |

The results presented in this Example show that the device and methods disclosed in the application are useful for delivering API to the olfactory epithelium and brain regions, and that an unexpectedly superior fractional dose of API was deposited on the olfactory epithelium. The results also show that the POD device delivered a high fraction of drug to the olfactory epithelium, which leads to higher drug concentrations in the brain and lower drug concentrations in the systemic circulation.

Example 3

This example demonstrates the improved penetration of a simulated nose cone using a device comprising a plurality of outlets in comparison to a device having a single outlet with and without circumferential flow.

Flow simulations were carried out using the Star-CCM+ computational fluid dynamics simulation software package, version 3.06.006. In the simulation, a cone was used with similar geometry to a nasal cavity for the sake of simplicity. The cone was designed to be narrow towards the top with the only outlet for residual air located at the bottom of the cone. Thus, the air in the top of the cone was stagnant and had to be displaced in order for the nozzle flow to penetrate the top of the cone, much like the upper nasal cavity of a human. The dimensions of the cone were 7.5 cm from top to bottom, in order to realistically simulate nasal delivery to the olfactory epithelium of a human.

The following nozzle structures were tested: (1) a nozzle without circumferential flow and a single outlet; (2) a nozzle with circumferential flow and a single outlet; and (3) a nozzle with circumferential flow and a plurality of outlets, in accordance with an embodiment of a POD device as illustrated in FIG. 6.

The various nozzle structures were place in the bottom of the cone with the outlets pointed upward towards the top of the cone. The area of flow for each of the nozzles was kept at 3.54 mm$^2$ and the air velocity coming from the outlets was kept constant at 60m/s. The simulation was performed under a steady time condition with k-epsilon turbulence. The simulations were run between 115 to 370 iterations until the momentum residuals remained constant between iterations.

The results of the flow simulations are shown in FIGS. 12A-12D.

FIG. 12A shows the simplex air flow pattern and velocity of the spray from a flow simulation using nozzle structure (1) having an outlet without circumferential velocity. As shown in FIG. 12A, the simplex flow does a poor job of penetrating the cone because it cannot move the air in the narrow top of the cone, so the plume gets pushed off to the sides.

FIG. 12B shows the circumferential flow pattern and velocity of the spray from a flow simulation using nozzle structure (2) having an single outlet with circumferential velocity. As shown in FIG. 12B, the spray flow coming out of the nozzle structure (2) having a single outlet with circumferential velocity does not penetrate into the cone either because a flow with vortical flow coming out of one orifice tends to spread out when exiting the orifice.

FIG. 12C shows the circumferential flow pattern and velocity of the spray from a flow simulation using nozzle structure (3) having a plurality of outlets with circumferential velocity, in accordance with an embodiment of a device of the present disclosure as illustrated in FIG. 6. As shown in FIG. 12C, the spray flow has superior penetration of the cone and penetrates to the top of the cone due to its narrow spray plume having circumferential and axial velocity, which allows for displacement of the air in the upper nasal cavity.

FIG. 12D illustrates the flow streams from the spray pattern shown in FIG. 12C. The flow simulation comparison using the various nozzle structures described in this example demonstrates the advantages of using a nozzle having a plurality of outlets that generates a narrow spray pattern having circumferential velocity to penetrate a narrow area (such as the upper nasal cavity of a human) where the air is displaced to allow for penetration of the spray in order to deposit a large fraction of drug on the olfactory epithelium.

Example 4

The integrin targeted RGD-Liposomes contained equal parts 1,2-dimyristoyl-sn-glycero-3-phosphocholine (DMPC) and 1,2-dimyristoyl-*sn*-3-phosphoglyserol (DMPG) lipids and 1% palmitic acid linked GRGDS peptide. These components were solubilized in a solution of 2:1 chloroform:methanol and then dried down to a thin film. Phosphate buffered saline was added to a suitable concentration and agitated until the lipids went into solution. The solution was sonicated in a bath sonicator for approximately 30 minutes until the liposomes had a measured average diameter of 50 nm. The targeted liposomes were stable with respect to size and encapsulation of aqueous contents and were suitable for use in the POD devices for nose to brain delivery.

Example 5

Various parameters of the POD device were tested. The reproducibility and predictability of the spray rate was tested by varying the pressure and the diameter of the liquid opening. By altering the physical dimensions of the device the spray rates could be increased or decreased.

To test the particle size distribution, water was sprayed from the device into viscous oil at various pressures and the resulting droplet diameters were measured using a microscope with size analysis software. A distribution with a majority of the particle diameters lying between 5 and 20 $\mu$m, with a mean diameter of 11.2 $\mu$m was achieved. This data shows that this method of atomization gives a desirable particle size distribution for nasal spray applications.

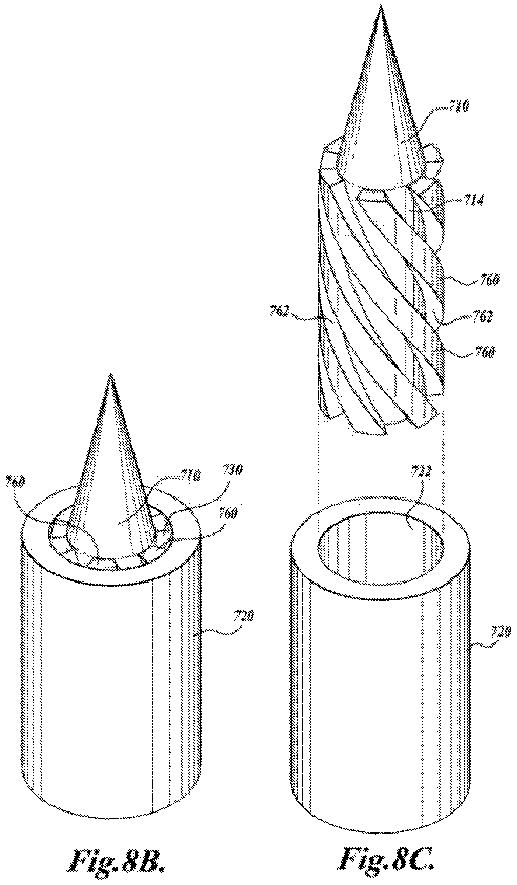

In order to test the vortical flow dispensed from the POD device, a blue dye marker was sprayed out of the device at various distances from a piece of paper. The vortical flow can be seen on the left side of FIG. 8, where the angle where the majority of the dye is located shifts when the distance changes. This can be contrasted with the spray patterns on the right side of FIG. 8 where there is no vortical flow and thus, a very even spray pattern. To test the penetration of the POD device into the nasal cavity compared to standard method of nose drops, rats.

The experimental design was approved by the Animal Care and Use Committee. All the animals were handled according to the guidelines established by the American Physiology Society and the National Institutes of Health.

Sprague Dawley rats average body weights 250 g, were caged in an environment in which the temperature and relative humidity were controlled, with alternating 12 hours light/dark cycles, and were given free access to feed and water during acclimation. Ten animals were tested, five controls and five experimental animals. The animals were surgically implanted with an in-dwelling vascular catheter and was examined for signs of stress and disease prior to any procedure. The animal was weighed prior to procedures and weights were noted on the cage card.

FIG. 9 shows the nasal penetration of a dark blue dye using nose drops and the POD device at various pressures.

FIG. 9 shows that increasing the air pressure of the POD device increases the penetration into the nasal cavity and coverage of the olfactory epithelium. The nasal drops resulted in no deposition on the olfactory epithelium, while the 3 psi spray from the POD device resulted in approximately 15% deposition on the olfactory epithelium, and, the 4 psi spray from the POD device resulted in approximately 40% deposition on the olfactory epithelium. These results are presented in FIG. 9 and indicate that at between 3-4 psi, a maximum penetration in nasal cavity was achieved to produce an optimal result. Higher pressures were untested but could lead to even deeper penetration into the nasal cavity.

The POD device exploits the unique, highly permeable property of olfactory epithelium (nose-to-brain barrier) to deliver neurologically active, analgesic, and cancer drugs to the brain. As discussed herein, the POD device generates a centrifugal rotational pattern of pressurized aerosol that overcomes the natural downward gravitational flow as well as displacement resistance associated with the air volume in nasal cavity to deposit a large fraction of drug at the olfactory epithelium.

Example 6

Free and Liposomal CCNU preparation. 1-(2-chloroethyl)-3-cyclohexyl-1-nitroso-urea (CCNU) was kindly provided by the Drug Synthesis and Chemistry Branch of the Developmental Therapeutics Division of Cancer Treatment from the NCI. The phospholipids, 1,2-dimyristoyl-sn-glycero-3-phosphocholine (DMPC) and 1,2-dimyristoyl-sn-glycero-3-phosphoglycerol (DMPG) were purchased from Sygena, Inc. (Cambridge, MA). Lipid-encapsulated CCNU (drug:lipid molar ratio 1:10) was prepared by first dissolving 20 mg of DMPC and DMPG (1:1, mol/mol) with CCNU in 1 ml of chloroform in a test tube and evaporating off the solvent with a stream of $N_2$ gas to form a dry film. In the targeted liposomes, 0-1% mol/mol PA-GRGDS was added to the organic phase. Where needed, 1% mol/mol of NBD-PE (Avanti Polar Lipids, Alabaster, AL) a fluorescent lipid marker, was added to the organic phase. The dry film was then vacuum desiccated for at least 30 min. To prepare desired lipid concentrations, a 1-ml volume of sterile PBS (pH 7.4), composed of 8 g/liter NaCl, 0.2 g/liter KCl and $KH_2PO_4$, and 0.16 g/liter $Na_2HPO_4$ was then added to create a 20-mg/ml suspension. The mixture was then sonicated at 27°C in a (water) bath type sonicator (Laboratory Supplies, Inc., Hicksville, NY) until a uniform translucent suspension of SUVs was obtained. Under these conditions, it was previously found that greater than 96% of the CCNU in the suspension was lipid associated.

In the case of the drug release example, the PBS added prior to sonication contained 50mM calcein (Sigma, St. Louis, MO). After sonication the free calcein was removed from the solution by dialysis. Free CCNU dosages were prepared just prior to drug administration and consisted of dissolving CCNU in a carrier solution of sterile 0.9% NaCl with 10% ethanol and 2% Tween 80.

The diameters of liposomes were determined by photon correlation spectroscopy (Malvern Zetasizer 5000, Southborough, MA) to be 96 $\pm$ 6 nm in size. Calcein was used as a model soluble compound to determine release of an encapsulated drug. Drug release was determined according to the method of Piperoudi et. al. Briefly, the liposomes were sonicated in a solution of PBS containing 50mM calcein. The liposomes were then dialyzed overnight to remove the non-encapsulated calcein. The fluorescence of the liposome solution was measured on a PerkinElmer 1420 multivariable fluorescence plate reader (PerkinElmer, Waltham, MA). The liposomes and free drug solution were aerosolized by spraying the solution through an airbrush type nozzle (Anest Iwata, Yokohama, Japan) driven by compressed $N_2$. To determine the aerodynamic particle size of the aerosol spray, the aerosol was sprayed into viscous and transparent oil and the diameters of the particles were measured using size analysis software on a Zeiss Fluorescent Microscope (Carl Zeiss Inc., Jena, Germany). The average spray particle was found to be 10.5 $\pm$ 8.8 $\mu$M.

HUVEC, LLC-PK1, and A549 lung cancer cells were purchased from ATCC. LLC-PK1 cells were cultured in DMEM supplemented with 10% FBS and antibiotics (100 U/mL penicillin G and 0.1 mg/mL streptomycin). HUVEC cells were cultured in F12-K media supplemented with 100ug/ml endothelial cell growth supplement (BD Biosciences, Franklin Lakes, NJ), 10% FBS, and antibiotics. A549 human lung cancer cells were cultured in F12-K media with 10% FBS and antibiotics. All cells were grown at 37 °C in a 5% $CO_2$/95% air humidified atmosphere.

The cell lines used for the binding studies were plated into 96-well flat-bottomed cell sterile culture plates (BD Biosciences, Franklin Lakes, NJ) at a density of $1.0 \times 10^5$ cells/well. Once the cells reached confluency, the media was removed and the cells were incubated with 200ul of the various liposome preparations for 30 minutes at 37 °C in a 5% $CO_2$/95% air humidified atmosphere. In the case of the competitive binding experiment the cells were pre-incubated with 25X concentration of soluble cyclo Arg-Gly-Asp-D-Phe-Lys (cRGD) peptide (Peptides International, Louisville, KY). The cells were then gently washed 3 times with PBS, pH 7.4, covered with 200ul of PBS, pH 7.4, and then analyzed with either a fluorescent plate reader or a fluorescent microscope.

Cell viability experiments were performed using the Alamar Blue dye reduction assay (Invitrogen, Carlsbad, CA). The experimental cell lines were plated at $1.0 \times 10^4$ cells/well in 96-well plates in recommended media and allowed to adhere overnight. The next day, cells were incubated with liposomes or free CCNU, with drug concentrations from 2.5 to 100 $\mu$M. Cells were also incubated with empty RGD-expressed and non-targeted liposomes. The control cells were only incubated in the recommended media. All experiments were performed in quadruplicate. Incubations were allowed to carry out for 3 days. After 3 days, Alamar Blue solution was added to the wells in a 1:5 ratio and allowed to incubate for 4 h at 37 °C in 5% $CO_2$-humidified atmosphere. Next, the wells were analyzed with a fluorescence plate reader with absorption and emission of 485/535 nm. All cell viability results are expressed as a percentage of viable cells compared to control cells.

Several in vitro cell binding experiments were conducted to characterize the targeting properties of this liposome formulation. Various concentrations of targeted and untargeted liposomes after incubation with αVβ3 integrin expressing epithelial cells were used. The fluorescence intensity and distribution is much greater with the RGD-expressed liposomes than with the untargeted liposomes. The untargeted liposomes tended to have lower binding at all concentrations, and the binding did not increase visibly with increased dose. The targeted formulation followed a dose dependant increase in binding. While the non-targeted formulation seems to appear in single bright spots, the RGD-expressed liposomes is clearly seen as binding around the cells and appears to be binding to the integrin receptors on the cell surfaces.

To determine that the targeted liposomes were binding to the cells primarily through the RGD targeting peptide, liposome formulations were prepared at various concentrations of PA-GRGDS being from 0.25 to 1.0 percent of the lipid concentration. The various targeted liposome formulations were incubated with human epithelial cells with high expression of αVβ3 integrin. As the relative concentration of PA-GRGDS increased in each formulation, the binding also increased. All of the targeted liposomes again seem to bind most intensely near the cell membranes where the target integrins are expressed.

In addition the liposome formulation with 1% PA-GRGDS clearly shows uptake of the liposomes into the cells, supporting previous findings of RGD mediated cellular uptake. (See Xiong XB et al., Enhanced intracellular uptake of sterically stabilized liposomal Doxorubicin in vitro resulting in improved antitumor activity in vivo. Pharm Res. 2005 Jun;22(6):933-9, Epub 2005 Jun 8; Xiong XB et al., Intracellular delivery of doxorubicin with RGD-modified sterically stabilized liposomes for an improved antitumor efficacy: in vitro and in vivo. J Pharm Sci. 2005 Aug;94(8):1782-93; and, Xiong XB et al., Enhanced intracellular delivery and improved antitumor efficacy of doxorubicin by sterically stabilized liposomes modified with a synthetic RGD mimetic. J Control Release. 2005 Oct 3;107(2):262-75.)

A competitive binding assay was used to confirm that the targeted liposomes displayed increased cellular binding because of the RGD motif of the PA-GRGDS peptide embedded in the liposome. The epithelium cells were pre-incubated with or without 25X the concentration of a cyclic RGD peptide with a high affinity for αVβ3 integrin receptors. The cyclic RGD almost completely inhibited the targeted liposomes from binding, which confirmed that the targeted liposomes were binding due to the RGD motif.

For a targeted liposomal formulation to be effective as an aerosol, the liposome properties are the same (i.e., not materially different) before and after being aerosolized. An artist airbrush type nozzle was used as an aerosol device for its easily adjustable parameters. The mean aerodynamic diameter of the atomized spray was over 100 times the diameter of the average liposome particle. The average spray particle from the airbrush was more than $1 \times 10^6$ times larger by volume than the average liposome particle. This large volume difference minimizes any disruption of the liposomes during aerosolization.

The size distribution of the liposome formulations did not change upon atomization. To test for increased leakiness caused by aerosolizing, the encapsulated liposomes were used with a model drug (calcein) at 50 mM into the liposome formulations. This concentration of calcein is self-quenching and does not emit a fluorescence signal. Any calcein that leaks out of the liposomes becomes diluted and can be measured. After the liposome solution was aerosolized with the airbrush there was only a 2.2±0.05% loss of drug.

The binding experiment shows that aerosolization had no significant effect on the binding properties of either liposome formulation. This experiment was designed similar to the prior experiment except that part of the liposome formulation was aerosolized before incubation with the cells. Aerosolization did not affect the ability PA-GRGDS peptide to improve the binding properties of the targeted liposomes, which implies that the targeting peptide was not damaged or displaced during aerosolization.

To determine if aerosolization of the liposome formulation would alter the effectiveness of a lipid soluble drug, CCNU was incorporated into the targeted and untargeted liposomes and was tested on the A549 human lung cancer cell line. Both liposome formulations improved the efficacy of CCNU, and the targeted therapy was twice as effective at stopping cancer growth compared to the untargeted liposome formulation.

Although RGD peptides have been reported to have anti-angiogenic effects in vitro, the empty liposomes tested had no significant cytotoxic effect. With this targeted formulation, the enhanced cytotoxic effect appeared to come from enhanced binding due to the PA-GRGDS peptide embedded in the liposome surface. The cytotoxicity of the drug formulations was not altered upon aerosolization, which implies that the targeted liposomes remained in tact during the atomization process, and the drug remained incorporated in the liposomes.

The results of this example demonstrate that the integrity of the integrin targeted liposome formulation was not altered by aerosolization, therefore, this formulation is useful as a carrier system for various APIs within or on the surface of the nanoparticle. The formulation is also useful as an aerosol with a hydrophilic marker (calcein) and a hydrophobic drug (CCNU). This formulation is useful with any number of targeting peptides embedded in its outer membrane as the RGD peptide is stable upon aerosolization. The liposomes may also be PEGylated to improve the residence time in circulation after absorption into the blood. The liposomes may also be substituted for another type of nanoparticle constructed with neuclotides, nucleosides, polymer sugars, or dextrans for aerosolized delivery.

Table 2. $IC_{50}$ values of the various liposome formulations and free CCNU, before and after aerosolization, when incubated with A549 human lung cancer cells. NS = no significant difference from control values.

| | $IC_{50}$ values ($\mu$M CCNU) | |
|---|---|---|
| | pre-aerosolization | post-aerosolization |
| Empty non-targeted liposomes | NS | NS |
| Empty RGD-expressed liposomes | NS | NS |
| Free Drug | 68.73 $\pm$ 2.48 | 70.53 $\pm$ 2.97 |
| non-targeted liposomes | 46.44 $\pm$ 1.19 | 43.84 $\pm$ 0.78 |
| targeted liposomes | 24.63 $\pm$ 0.77 | 23.13 $\pm$ 1.13 |

Example 7

Brain Distribution of Mannitol and Nelfinavir after Nasal drug delivery to either the olfactory or the respiratory nasal epithelia in rats. The importance of drug localization within the nasal cavity is demonstrated by the nasal drug delivery methods for delivering drug primarily to either the olfactory or the respiratory epithelium in a consistent manner. These methods are validated by confirming consistent drug localization within the nasal cavity as well by verifying that they did not cause damage to the nasal epithelia or turbinate structures. Delivery of a hydrophilic drug (mannitol) and a hydrophobic drug (nelfinavir) was also demonstrated using these methods, and any material differences in brain distribution after deposition on the olfactory epithelium or the respiratory epithelium were determined.

Three methods of nasal drug delivery were initially tested to establish methods for delivering drug primarily to the respiratory or the olfactory region. A dye solution was administered with either a catheter tube attached to a microsyringe at two different distances (15 and 7 mm) within the rat nasal cavity or administered as nose drops. The nasal cavity was examined to determine both the dye deposition within the nasal cavity as well as the feasibility of the methods. After this initial dye deposition investigation, the POD device was used to deposit drug closer to the cribriform plate region of the olfactory region in a consistent and non-invasive way.

The POD device was characterized to ensure consistency of dose as well as spray pattern characteristics. Several driving POD aerosol pressures (5, 20, 30 psi) were tested within rat nasal cavities with a dye solution to determine localization of the solution. In addition, using these same pressures, a histopathologic analysis of the nasal cavity was conducted to determine any irritation or damage to the nasal cavity from the POD device.

After characterization of the different nasal drug delivery methods, radiolabeled mannitol was administered to either the respiratory region or the olfactory region of the rat nasal cavity or via intravenous (IV) administration, and then brain and blood distributions were determined at 30 and 150 minutes. In addition, a hydrophilic drug, nelfinavir was delivered to either the olfactory or respiratory epithelium of the nasal cavity and brain and blood distribution was determined after 30 minutes.

Coomassie blue (Sigma-Aldrich, St. Louis, MO) was used to determine nasal cavity deposition and aerosol spray patterns. Mannitol was $^{14}$C labeled with a specific activity of 0.1 mCi/ml and purchased from Moravek (Brea, CA). Unlabeled mannitol and nelfinavir was $^{14}$C labeled with a specific activity of 1.0 mCi/ml. Other components included unlabeled nelfinavir, propylene glycol, ethanol, nitrogen gas, 0.9% saline solution, pentobarbital, EDTA, formalin, biosol, and bioscint.

Adult male Sprague-Dawley rats (200-300 g; Harlan, Indianapolis, IN) were housed under a 12 hour light/dark cycle with food and water provided *ad libitum.* Animals were cared for in accordance with institutional guidelines, and all experiments were approved by the University of Washington Animal Care and Use Committee.

Mannitol doses used in the histopathology example consisted of 0.2 mg mannitol dissolved in 0.9% saline solution, pH 7.4. Radiolabeled mannitol dose solutions consisted of 0.2 mg unlabeled mannitol and 2.0 $\mu$Ci $^{14}$C mannitol in a solution of 98% $H_2O$ and 2% EtOH, pH 7.4. The total volume of each nasal mannitol dose was 20 $\mu$l while each IV dose was administered as a volume of 100 $\mu$l. Nelfinavir dose solutions consisted of 0.12 mg unlabeled nelfinavir and 2.0 $\mu$Ci $^{14}$C nelfinavir in a solution of 75% propylene glycol and 25% EtOH. The total volume of each nasal nelfinavir dose was 20 $\mu$l. All drug solutions were mixed on the day of delivery.

The overall construction of the POD nasal aerosol device is shown in FIG. 1. A pressurized nitrogen container was connected to a standard two-valve pressure regulator. Plastic tubing with a 200 psi pressure rating connected the pressure regulator to the inflow connection of a pneumatic solenoid (REF). The solenoid was controlled by a GraLab 555 digital timer (REF), which was foot pedal actuated. On the outflow connection of the solenoid was a 3 ml syringe (REF) with the plunger removed and which had been tapped to fit securely on the threading of the solenoid. On the tip of the syringe was a modified 21 gauge needle (REF). The needle was cut to be 8.0 mm in length and filed to smooth and round the tip. A round cut screen was placed in the base of the needle so that liquid drug could be placed there with a pipette without moving either into the needle or the syringe. The tip had a small length (2.0 mm) of a micro drill bit (REF) placed securely in the tip. This drill bit piece in the needle tip served to mix the nitrogen and liquid drug to create and aerosol output from the device, as well as adding a degree of rotation to the aerosol output to enhance penetration into the nasal cavity towards the cribriform plate area. Finally, a piece of a 21 gauge catheter (REF) was placed over the outside of the needle in order to protect the nasal epithelia from being damaged by the metal of the modified needle.

The basic operation of the POD device was as follows. The needle was removed from the syringe tip and the liquid dose was placed on the screen within the needle. The needle was then securely placed back on the syringe. The needle was carefully placed 8.0 mm into the rat nasal cavity and pointed in the direction of the cribriform plate. Then the foot pedal was pressed which actuated the solenoid for 0.1 seconds to propel the dose to the olfactory region of the nasal cavity.

The aerosol aerodynamic particle size was determined by photon correlation spectroscopy (PCS) (Malvern Zetasizer 5000, Southborough, MA). The solution tested was a 0.9% saline solution. The aerosol droplet size distributions were determined by a Phase Doppler Particle Analyzer (PDPA) (TSI, Shoreview, MN using a 200mW argon laser emitting beams of 488 and 514.5 nm wavelength (Ion Laser Technology, model # 5500A-00). The measurements were taken 2.75 cm from the tip of the nozzle, as this represents the distance from the proximal opening of the nasal vestibule to the center of the respiratory epithelium. Initially, the measurement volume was moved across the aerosol stream to determine the edges of the spray. Then, aerosol sizing measurements were determined at 1 mm intervals across the width of the spray, taking 30,000 measurements at each interval. Sizing data is presented as a volume weighted mean and span, defined as $Span = \dfrac{D_v 90 - D_v 10}{D_v 50}$ where $D_v$ is droplet frequency distribution.

Each time the POD spray device was used, the desired dose was placed onto the wire mesh within the needle body. A fluorescence assay was used to determine if the total desired volume was sprayed out of the device with each actuation. A solution of 50 $\mu$g/ml fluorescein was sprayed from the device into a well in a 24-well plate that was prefilled with 1 ml ddH$_2$O. After the spray, the solution was mixed by pipetted up and down several times. Three volumes which could be used for rat nasal delivery, (5, 10, 25 $\mu$l), were tested at two different pressures, 20 and 30 psi. The fluorescence of the liposome solution was measured on a PerkinElmer 1420 multivariable fluorescence plate reader (PerkinElmer, Waltham, MA).

In order to test rotational pattern of the POD aerosol spray, the POD device was loaded with coomassie blue dye and sprayed onto an absorbent paper. The POD body was mounted above the piece of paper to minimize any effects of gravity on the plume geometry. Two level tools were attached to the POD spray device, parallel and perpendicular to the ground, in order to ensure that the aerosol plume was directed directly perpendicular to the ground for each aerosol plume tested. The aerosol plume was sprayed at distances of 1.0, 2.0, and 3.0 cm from the absorbent paper with driving pressures of 20 and 30 psi. The diameter and geometric dye profile were observed at each distance and for each driving pressure.

Rats (N=6) were anesthetized with 50 mg/kg sodium pentobarbital delivered via intraperitoneal injection (IP). Once under anesthesia the rats receiving dye solution via a catheter were placed on their back, a microsyringe was attached to a catheter, which was either 15 or 7 mm long, was inserted into each nasal cavity and 20 $\mu$l of dye solution was administered. Animals receiving dye solution via POD spray were anesthetized The animals receiving dye solution via nose drops were placed on their back and a 5 $\mu$l drop of dye solution was placed near the right naris with a pipette and allowed to be snorted into the nasal cavity. After 2 minutes the process was repeated in the opposite naris. Shortly after administration (< 5 minutes), pentobarbital was administered the nasal cavity was then dissected and the tissues were closely examined for dye placement.

Rats that received POD spray for histopathologic analysis were anesthetized with 50 mg/kg sodium pentobarbital delivered via IP injection. Once anesthetized the animals were placed on their backs and given a dose of POD spray according to the method described above. Rats (N=6) received a single 10 $\mu$l POD spray of 0.1 mg mannitol dissolved in 0.9% NaCl. Nasal tissues from two untreated control rat were processed in the same manner as the treated tissues.

Three driving pressures (10, 20, and 30 psi) of the POD spray were tested. Histopathologic analysis of the POD nasal spray device was conducted according to the method disclosed in Young JT. Histopathologic examination of the rat nasal cavity, Fundam Appl Toxicol, 1981 Jul-Aug;1(4):309-12. The nasal cavity was initially fixed in a solution of 10% formalin and then decalcified in a solution of 10% EDTA. The tissue was then placed in 70% EtOH before being embedded in paraffin, sectioned, and stained with hematoxylin and eosin stain.

Animals used in the radiolabeled mannitol and radiolabeled nelfinavir distribution experiments were anesthetized at the beginning of the experiment with 50 mg/kg sodium pentobarbital delivered via IP injection. The anesthetized animals were placed on their backs on a rodent heat pad (REF) to maintain body heat. Mannitol doses given as nose drops were given as a 5 $\mu$l drop every minute in alternating naris for a total volume of 20 $\mu$l. Mannitol doses administered with the POD spray device were delivered first as a 10 $\mu$l spray into the left naris, with a second dose administered into the right naris 2 minutes after the first nasal spray for a total volume of 20 $\mu$l. The IV mannitol doses were administered with a tail vein injection.

Nelfinavir nose drop and POD spray doses were administered according to the same method and volumes as the mannitol dosing. At either 30 or 150 minutes after radiolabeled drug dosing an IP injection of 250 mg/kg sodium pentobarbital was administered. The brain was dissected into the olfactory bulbs, cortex, diencephalon, brainstem, and cerebellum. The olfactory bulbs were the last tissues to be removed from the skull. Cervical spinal cord from C1 to C5 were removed from the body as well. The nasal cavity was also carefully opened and the olfactory and respiratory epithelia were removed.

Tissue samples were weighed and placed into 4 ml polypropylene scintillation vials with 400 $\mu$l of Biosol. Each blood sample was placed into 400 $\mu$l of Biosol immediately after collection. All samples were placed in a water bath at 55°C overnight to dissolve the tissues. The tissues were allowed to cool to room temperature and the vials were filled with Bioscint scintillation fluid. A volume of 40 $\mu$l of 30% hydrogen peroxide was added into each of the blood sample scintillation vials.

Radioactivity in each sample was analyzed with a radiocounter.

Brain and blood concentrations were determined with standard curves made with blank tissue or blood that was spiked with radiolabeled drug and processed according to the methods for the radiolabeled samples. A one-way ANOVA with a Tukey post test was used to compare drug distribution in the brain after nose drops, POD spray, or IV delivery with mannitol, while an unpaired t-test was used to compare drug distribution in the brain after nose drops or POD spray of nelfinavir.

Example 8

Pharmacokinetic and Pharmacodynamic Analysis of Opioid Analgesic drugs after Nasal, Olfactory or Systemic Administration.

In this example, the pharmacokinetic and pharmacodynamic effects of morphine and fentanyl were determined after administration to either the respiratory region or the olfactory region of the nasal cavity. Morphine and fentanyl have logP values of 0.8 and 3.9 respectively permitting extrapolation of these results to a variety of small molecule drugs, which also yields insight into the usefulness and limitations of nose-to-brain delivery.

The percentage of direct nose-to-brain uptake of morphine and fentanyl (after delivering drug primarily to the cribriform plate region) was determined.

A method for delivering drug to the cribriform plate region was used. The POD device was used to quickly deliver a majority of drug to the cribriform plate region. The POD device and low volume nose drops were compared for delivering API either primarily on the olfactory epithelium or respiratory epithelium of the nasal cavity. 7-15 $\mu$l of drug solution was used for nasal drug delivery, which is representative of the dose volumes used in human nasal drug delivery.

Analgesic inducing effects of morphine or fentanyl were analyzed upon nasal delivery primarily to the olfactory epithelium or respiratory epithelium. Systemic delivery using the tail-flick test in rats was also determined. Each animal (N=9) received a single dose strength (2.5, 5.0, 10.0 mg/kg morphine and 7.5, 15, and 25 $\mu$g/kg fentanyl) of opioid analgesic drug by the three different delivery routes. This was a randomized cross over study with two days between each experiment. The animals were each briefly anesthetized and given a single drug administration. They were then allowed to recover and the tail flick test was performed multiple times during the 2 hours after treatment.

A pharmacokinetic (PK) analysis of each drug was carried out after a single treatment of the opioid drugs. The PK analysis mirrored the tail flick study in order to compare the analgesic effect of the opioid drugs with the blood concentration to determine any apparent direct nose-to-brain delivery. The single treatment of opioid analgesic was administered via POD nasal spray, nose drops, or IP injection.

Animals were dosed with 2.5, 5.0, 10.0 mg/kg morphine and 7.5, 15, and 25 $\mu$g/kg fentanyl. The animals were anesthetized throughout the duration of these experiments. The animals (N=3) were anesthetized and given a single drug treatment and blood was drawn from a femoral catheterization over the course of the experiment. Blood was drawn over the course of 2 hours at the same time points as the tail flick test after drug administration.

A tissue distribution study was performed. The purpose was to confirm the tail flick study by determining the tissue concentrations of drug in the CNS. Comparing the tissue concentrations to the tail flick test allows for a better understanding of direct nose-to-brain distribution and how it affects the action of the opioid drugs. The animals (N=3) were briefly anesthetized and given a dose of either morphine or fentanyl by nasal spray, nose drops, or IP injection. Only a single dose of each drug was tested in this part of the example (5.0 mg/kg morphine or 50 $\mu$g/kg fentanyl).

Morphine sulfate, fentanyl citrate, and DAMGO were purchased from Sigma-Aldrich (St. Louis, MO). Beuthanasia-D (Shering Plough Animal Health Corp, North Chicago, IL) was used for euthanasia at the end of the distribution studies. The drug doses were dissolved in 0.9% saline solution (Hospira Inc, Lake Forest, IL). Morphine and fentanyl LC/MS standards were purchased from Cerilliant (Round Rock, TX).

Adult male Sprague-Dawley rats (200-300 g; Harlan, Indianapolis, IN) were housed under a 12 hour light/dark cycle with food and water provided *ad libitum.* Animals were cared for in accordance with institutional guidelines, and all experiments were approved by the University of Washington Animal Care and Use Committee.

Animals were anesthetized with 2% isoflurane (Novaplus, Lake Forest, IL). Body temperature was maintained at 37°C by a heating pad (Fine Science Tools, Inc., Foster City, CA). For PK experiments, the femoral vein was cannulated for blood draw with PE10 polyethylene tubing (Becton Dickinson, Franklin Lakes, NJ) connected to blunt tipped 23 gauge needle (Becton Dickinson, Franklin Lakes, NJ). The PE-10 tubing was inserted 2 cm into the femoral vein to ensure blood sampling was from the vena cava.

Animals were maintained on anesthesia for 30 minutes prior to drug administration. Following the final blood draw, the catheter was removed from the femoral vein, the proximal end of the femoral vein was tied off with suture string to assure hemostasis, and the incision stitched with suture string (Harvard Apparatus, Holliston, MA). The animal was allowed to recover from anesthesia in a padded container and a subcutaneous dose of 0.025 mg/kg IP injected.

Morphine and fentanyl were stored at -20°C as a lyophilized powder. On the day of each experiment, the necessary doses of morphine and fentanyl were solubilized in 0.9% saline solution (Sigma-Aldrich, St. Louis, MO). Each formulation had a pH of 7.0 for each of the doses tested.

For each experiment, the animals were dosed morphine or fentanyl while under isoflurane anesthesia. For the tail-flick and distribution study, each animal was briefly anesthetized with 5% isoflurane in an induction chamber, the animal was removed from the induction chamber and the drug was administered. The animal was turned on to its side and then the animal was allowed to recover from the anesthesia.

Each animal remained anesthetized with 2% isoflurane throughout the pharmacokinetic example. First the femoral vein was cannulated in order to draw blood during the experiment. After surgery the animal was allowed to remain under anesthesia for 20 minutes. Then, for the olfactory and respiratory nasal drug delivery, the anesthesia nose cone was removed, the dose was given quickly (< 45 seconds) and the nose cone was replaced. For IP delivery the dose was simply injected with a 23 gauge syringe into the peritoneal cavity. After drug administration each animal was placed on its side on the heat pad.

In the CNS distribution experiment, animals were initially anesthetized with 5% isoflurane. Once they were unconscious, they were quickly removed from the induction box and dosed as described for the tail flick experiment with either nose drops, POD spray, or IP injection. They were allowed to naturally recover from the anesthesia.

Each group of animals for a dose strength of morphine or fentanyl (N=9) first went through three days of placebo testing to get a base line reading for the tail flick test as well as acclimating the animals to handling. Each animal was exposed to 5% isoflurane in an induction box. Once anesthetized the animal was removed and given a 10$\mu$l dose of 0.9% saline solution, pH 7.4 via nose drop, POD nasal spray, or IP injection. Once the placebo dose was administered, the animals were allowed to fully wake from the isoflurane in a padded tray. Then at 5, 10, 30, 45, 60, 90, and 120 minutes each animal was wrapped gently in a towel, had their tails placed in room temperature water (18°$\pm$0.5°C) for 5 seconds, the tail was quickly dried, and then the distal 3 cm of the tail was placed in 55°$\pm$0.5°C water. The time until tail removal was measured with a digital stopwatch.

After the initial placebo trials, the same procedure was repeated three times, every other day over 5 days, with each rat receiving a single dose of either morphine or fentanyl by nasal spray, nose drops, and IP injection in a randomly chosen order. The cutoff time, at which the tail would be removed from the water to prevent tissue damage, was set at 10 seconds for all tail flick trials.

In the PK experiments, 300 $\mu$l of blood was drawn from the femoral vein catheter at 5, 10, 30, 45, 60, 90, and 120 minutes after drug administration. The blood was collected in a 1ml syringe (Becton Dickinson, Franklin Lakes, NJ) and transferred to a microcentrifuge tube for blood/plasma separation. The tubes were immediately centrifuged at 8,000 g for 5 minutes. Then the plasma was removed and frozen on dry ice. At the end of the experiment 2.0 mls of sterile 0.9% saline solution was injected via the femoral vein catheter to replace the removed blood volume.

A fixed volume (20 $\mu$l) of morphine d-6 (Cerilliant, Palo Alto, CA) was added into each tissue and plasma sample to act as an internal standard. Morphine tissue samples were homogenized in 5-10 times volume of 0.1M borate buffer, pH 8.9 and centrifuged for 10 minutes at 1000g. The morphine tissue supernatant and plasma samples were passed over Certify solid phase extraction cartridges (Varian, Palo Alto, CA) and eluted with methylene chloride: isopropanol: ammonium hydroxide (80:20:2). After elution the samples were evaporated under $N_2$ gas until dry. The samples were resuspended in 75 $\mu$l of mobile phase which consisted of 92% of 0.05% acetic acid and 8% acetonitrile mobile phase. An Agilent HPLC/MS series 1100 series B with autosampler (Agilent, Santa Clara, CA) was used for quantification. The injection volume was 5 $\mu$l. The morphine samples were passed over a Zorbax SB-C8 column (Agilent, Santa Clara, CA) with a flow rate of 0.25 ml/min. The ionization setting was API-ES in positive mode with a capillary voltage of 1400V.

The fentanyl samples were quantified in a similar process. A fixed volume (20 $\mu$l) of fentanyl d-6 (Cerilliant, Palo Alto, CA) was added into each tissue and plasma sample to act as an internal standard. Fentanyl tissue samples were homogenized in 5-10 times volume of 0.1M potassium phosphate buffer, pH 6.0 and centrifuged for 10 minutes at 1000g. The fentanyl tissue supernatant and plasma samples were passed over Certify solid phase extraction cartridges (Varian, Palo Alto, CA) and eluted with methylene chloride: isopropanol: ammonium hydroxide (80:20:2). After elution the samples were evaporated under $N_2$ gas until dry.

The samples were resuspended in 75 $\mu$l of mobile phase which consisted of 40% 10 mM ammonium acetate and 60% acetonitrile. An Agilent HPLC/MS series 1100 series B with autosampler (Agilent, Santa Clara, CA) was used for quantification. The injection volume was 5 $\mu$l. The fentanyl samples were passed over a Zorbax SB-C8 column (Agilent, Santa Clara, CA) with a flow rate of 0.25 ml/min. The ionization setting was API-ES in positive mode with a capillary voltage of 1400V.

For both morphine and fentanyl, a standard curve was created on the day of analysis according to the same process described for the samples. Each standard curve was linear with a coefficient of linear regression $R^2 > 0.99$. In addition, two quality control samples with a known amount of drug were processed on the day of analysis in order to ensure day-to-day consistency of the analytical assay.

All tail flick test values are presented as a percentage of maximal possible effect which is defined as:

$$\frac{(Post\ drug\ latency - baseline\ latency)}{(cutoff\ time - baseline\ latency)} \times 100$$

.

AUC values from all experiments were calculated using the trapezoidal rule without extrapolation to infinity. Tail flick data was compared using repeated measures ANOVA. Plasma and tissue concentrations were compared using a one-way ANOVA with a Tukey post-test. All statistical analyses were performed using Sigma Plot software version 11.0 (Systat Software Inc, San Jose, CA).

Example 9

Summary of select intranasal drug studies demonstrating direct distribution to the CNS is shown in Table 3. Multiple studies in rodents, primates, and humans have shown that a wide variety of intranasally administered small molecule and macromolecule drugs exhibit rapid delivery to the brain and CSF and result in larger brain/blood ratios than can be achieved by systemic administration.

Table 3

| Drug | mw (da) | Dose | Specie | CNS Are Targeted | Results (reference) |
|---|---|---|---|---|---|
| Morphine | 285.3 | 1 mg/kg | Rat | Cortex | Brain/Plasma AUC ratio 3.0 IN; 0.1 IV (Westin et al., 2006) |
| TS-002 | 420.99 | 0.4 mg | Primate | Basal forebrain | 4 fold increase in AUCeffect / AUCplasma with intranasal over intravenous (Yamada et al., 2007) |
| IGF-1 | 7649 | 143 $\mu$g/kg | Rat | Olf. Bulb, Forebrain, Brainstem, Spine | No change in plasma AUC; 100X brain concentrations with intranasal v. intravenous (Thorne et al., 2004) |
| Orexin-A | 3562 | 1.0 $\mu$g/kg | Primate | Hypothalamus | Intranasal dose was 10X lower and produced greater effect in the hypothalamus (Deadwyler et al., 2007) |
| Interferon $\beta$ | 22,500 | 4 mg | Primate | Olf. Bulb, Dura, Cortex, Basal Ganglia | Clinically effective levels of protein in most brain regions with low blood levels (Thorne et al., 2008) |
| MSH | 962.1 | 10 mg | Human | CSF | No significant change in plasma AUC; 69 X increase in CSF AUC (Born et al., 2002) |

Example 10

Table 4. Olfactory deposition of POD device, as percentage of dose, in a human nasal cavity model. Olfactory deposition within a silicon human nasal cavity model was tested using various horizontal and vertical angles of administration and two pressures. The deposition changed significantly when the orientation changes, with a 30-40° vertical angle producing the greatest olfactory deposition.

Table 4

| Vertical Angle (°) | Driving Pressure of 30 psi | | | Driving Pressure of 45 psi | | |
|---|---|---|---|---|---|---|
| | Horizontal Angle (°) | | | Horizontal Angle (°) | | |
| | 0° | 5° | 10° | 0° | 5° | 10° |
| 60 | 3.3 ± 1.8 | 7.8 ± 9.3 | 22.6 ± 4.5 | 8.8 ± 3.6 | 41.7 ± 13.6 | 13.4 ± 6.5 |
| 50 | 11.0 ± 10.2 | 34.2 ± 7.0 | 57.0 ± 7.2 | 20.3 ± 11.5 | 48.1 ± 10.4 | 33.5 ± 11.0 |
| 40 | 36.8 ± 2.4 | 37.8 ± 5.4 | 51.4 ± 8.5 | 40.5 ± 6.3 | 43.6 ± 4.2 | 48.2 ± 5.0 |
| 30 | 48.2 ± 3.5 | 46.5 ± 4.7 | 42.6 ± 6.2 | 36.0 ± 6.7 | 42.5 ± 5.1 | 47.9 ± 3.4 |

Example 11

Table 5. Effect and plasma AUC ratios after morphine delivery. POD administered morphine resulted in significantly greater $AUC_{effect}$ for 1.0 mg/kg and 5.0 mg/kg doses, while resulting in significantly lower $AUC_{plasma}$ at the 5.0 mg/kg dose. POD administration also resulted in a significantly greater $AUC_{effect}$ / $AUC_{plasma}$ ratio at all concentrations compared to systemic administration indicating a significant portion of the dose was directly distributed to the CNS from the nasal cavity. (* = $P < 0.05$ compared to nose drops; † = $P < 0.05$ compared to IP; ‡ = $P < 0.05$ compared to POD).

Table 5

| Dose (mg/kg) | Device | AUCeffect (%MPE*min) | AUCplasma (ng*min/ml) | $\dfrac{AUCeffect}{AUCplasma}$ | DTP% |
|---|---|---|---|---|---|
| 1.0 | drop | 731 | 6298 | 0.097 | -25.2 |
| 1.0 | POD | 1220*† | 7168 | 0.161*† | 38.5 |
| 1.0 | IP | 687 | 6263 | 0.098 | |
| 2.5 | drop | 1466 | 15470 | 0.108 | 10.9 |
| 2.5 | POD | 1899 | 13617 | 0.143† | 38.1 |
| 2.5 | IP | 1287 | 19470 | 0.087 | |
| 5.0 | drop | 1516† | 28710 | 0.069 | 14.8 |
| 5.0 | POD | 3565* | 28508† | 0.132*† | 55.0 |
| 5.0 | IP | 2789* | 41904 | 0.059 | |

Example 12

Table 6: Concentration and effect data after morphine delivery in rats.

| Dose (mg/kg) | Device | $C_{max\ (ng/ml)}$ | $T_{max}$ (min) Cone. | $E_{max\ (\%MPE)}$ | $T_{max}$ (min) effect |
|---|---|---|---|---|---|
| 1.0 | drop | 112 | 10 | 10.9 | 10 |
| 1.0 | POD | 104 | 10 | 17.1 | 10 |
| 1.0 | IP | 97 | 10 | 9.8 | 30 |

(continued)

| Dose (mg/kg) | Device | $C_{max\ (ng/ml)}$ | $T_{max}$ (min) Cone. | $E_{max\ (\%MPE)}$ | $T_{max}$ (min) effect |
|---|---|---|---|---|---|
| 2.5 | drop | 189 | 30 | 18.6 | 30 |
| 2.5 | POD | 195 | 10 | 30.6 | 10 |
| 2.5 | IP | 273 | 10 | 23.3 | 10 |
| 5.0 | drop | 296 | 30 | 24.1 | 10 |
| 5.0 | POD | 322 | 30 | 41.8 | 10 |
| 5.0 | IP | 579 | 10 | 42.8 | 45 |

Example 13

Table 7. Effect and plasma AUC ratios after fentanyl delivery. POD administration resulted in significantly lower AUCeffect compared to IP and nose drop administration at various doses. IN addition the AUCplasma was significantly higher than IP administration at 7.5 and 15 $\mu$g/kg doses. There were no significant differences in AUCeffect/AUCplasma ratios between any of the routes at any of the doses administered. (* = $P < 0.05$ compared to nose drops; † = $P < 0.05$ compared to IP; ‡ = $P < 0.05$ compared to POD).

Table 7

| Dose ($\mu$g/kg) | Device | AUCeffect (%MPE*min) | AUCplasma (ng*min/ml) | $\dfrac{\textbf{AUCeffect}}{\textbf{AUCplasma}}$ |
|---|---|---|---|---|
| 7.5 | drop | 691.2 | 135 | 4.90 |
| 7.5 | POD | 258.9* | 202† | 1.56 |
| 7.5 | IP | 689.8 | 50 | 11.38 |
| 15.0 | drop | 800.5 | 293† | 1.83 |
| 15.0 | POD | 221.5 *† | 285† | 1.22 |
| 15.0 | IP | 657.7 | 46* | 5.33 |
| 25.0 | drop | 2080.2† | 508 | 4.76 |
| 25.0 | POD | 2392.3† | 367 | 6.80 |
| 25.0 | IP | 868.6* | 148 | 10.41 |

Example 14

Table 8: Concentration and effect data after fentanyl delivery in rats.

Table 8

| Dose (mg/kg) | Device | $C_{max\ (ng/ml)}$ | $T_{max}$ (min) Conc. | $E_{max\ (\%MPE)}$ | $T_{max}$ (min) effect |
|---|---|---|---|---|---|
| 7.5 | drop | 3.0 | 5 | 26.4 | 10 |
| 7.5 | POD | 5.9 | 5 | 25.7 | 5 |
| 7.5 | IP | 1.1 | 5 | 25.8 | 10 |
| 15.0 | drop | 5.8 | 10 | 38.2 | 5 |
| 15.0 | POD | 7.8 | 5 | 73.8 | 5 |
| 15.0 | IP | 1.1 | 5 | 24.7 | 10 |
| 25.0 | drop | 6.3 | 10 | 59.2 | 10 |
| 25.0 | POD | 10.0 | 5 | 93.0 | 5 |

(continued)

| Dose (mg/kg) | Device | $C_{max\ (ng/ml)}$ | $T_{max}$ (min) Conc. | $E_{max\ (\%MPE)}$ | $T_{max}$ (min) effect |
|---|---|---|---|---|---|
| 25.0 | IP | 3.3 | 5 | 26.7 | 10 |

Example 15

Coomassie blue (Sigma-Aldrich, St. Louis, MO) was used to determine nasal cavity deposition and aerosol spray patterns. Medical grade nitrogen gas was purchased from Airgas Nor Pac (Vancouver, WA). The 0.9% saline solution was purchased from Hospira Inc (Lake Forest, IL). All other materials were reagent grade. The silicon human nasal cavity model was purchased from Koken Inc. (Tokyo, Japan).

The initial testing for the POD device was done *in silico* using Star-CCM+ fluid dynamics software (CD-Adapco, Detroit, MI). Software ws used to develop and optimize the basic design for the POD nozzle. The POD device was modified for liquid single dose use. The basic aerosol properties of the device were tested as well as the rotational properties of the aerosol spray exiting the device. After this, the ability of the device to specifically target the olfactory region of the nasal cavity was tested.

Three methods of nasal drug delivery were initially tested to establish methods for delivering drug primarily to the respiratory or the olfactory region in rats. A dye solution was administered with either a catheter tube attached to a microsyringe at two different distances (15 and 7 mm) within the rat nasal cavity or administered as nose drops. The nasal cavity was examined to determine both the dye deposition within the nasal cavity as well as the feasibility of the methods. After this initial dye deposition investigation, a pressurized olfactory delivery (POD) device was developed to deposit drug closer to the cribriform plate region of the olfactory region in a consistent and non-invasive way. The device was characterized to ensure consistency of dose as well as spray pattern characteristics. Several input POD aerosol pressures (5, 20, 30 psi) were tested with a dye solution in rats to determine localization of the dye within the nasal cavities. In addition, a histopathologic analysis of the nasal cavity was conducted in rats to determine any irritation or damage to the nasal cavity from the POD device. These rats were treated with aerosol generated by POD using the same settings.

To test the device for possible human use, a human nasal cavity model was tested for the percent olfactory deposition. Several vertical angles (40, 50, 60, and 70°) and horizontal angles were tested in order to understand the influence of POD device orientation on olfactory deposition.

Adult male Sprague-Dawley rats (200-300 g; Harlan, Indianapolis, IN) were housed under a 12 hour light/dark cycle with food and water provided *ad libitum.* Animals were cared for in accordance with institutional guidelines, and all experiments were approved by the University of Washington Animal Care and Use Committee.

Flow simulations were carried out using the Star-CCM+ computational fluid dynamics simulation software package, version 3.06.006. In the simulation, a cone was used with similar geometry to a nasal cavity for the sake of simplicity. The cone was designed to be narrow towards the top with the only outlet for residual air located at the bottom of the cone. Thus, the air in the top of the cone was stagnant and had to be displaced in order for the nozzle flow to penetrate the top of the cone, much like the upper nasal cavity of a human. The dimensions of the cone were 7.5 cm from top to bottom in order to realistically simulate nasal delivery to the olfactory epithelium of a human.

The following nozzle structures were tested: (1) a nozzle without circumferential flow and a single outlet; (2) a nozzle with circumferential flow and a single outlet; and (3) several nozzles with circumferential flow and a plurality of outlets. The various nozzle structures were placed in the bottom of the cone with the outlets pointed upward towards the top of the cone. The area of flow for each of the nozzles was kept at 3.54 mm$^2$ and the air velocity coming from the outlets was kept constant at 60m/s. The simulation was performed under a steady time condition with k-epsilon turbulence. The simulations were run between 115 to 370 iterations until the momentum residuals remained constant between iterations.

The overall construction of the POD nasal aerosol device is shown in FIG. 28. A pressurized nitrogen gas supply was connected to a standard two-valve pressure regulator. Plastic tubing with a 200 psi pressure rating connected the pressure regulator to the inflow connection of a pneumatic solenoid (Cramer Decker Industries, Santa Ana, CA). The solenoid was regulated with a foot pedal actuated GraLab 555 digital timer (Gralab Corporation, Centerville, OH). On the outflow connection of the solenoid was a 3 ml cylinder, which made up the device body and had been tapped to fit securely on the threading of the solenoid. On the end of the device body was a custom fit aerosol nozzle with a 0.8 mm outside diameter. The nozzle was fitted with a spin insert composed of a small length (2.0 mm) of metal cylinder, which had two spiral grooves in which the fluid/gas mixture traveled. This served to mix the nitrogen and liquid drug to create an aerosol output from the device as well as adding rotation to the aerosol output to enhance penetration into the nasal cavity towards the cribriform plate area. A piece of 21 gauge catheter (BD, Franklin Lakes, NJ) was placed over the outside of the nozzle in order to protect the nasal epithelia from being damaged by the nozzle during use.

The basic operation of the POD device in rats was as follows: the dose was loaded into the device and the needle was carefully placed 8.0 mm into the rat nasal cavity and pointed in the direction of the cribriform plate. Then the foot pedal was pressed to actuate the solenoid for 0.1 seconds to propel the dose to the olfactory region of the nasal cavity.

The POD generated aerosol droplet size distributions were determined by a Phase Doppler Particle Analyzer (PDPA) (TSI, Shoreview, MN) using a 200mW argon laser emitting beams of 488 and 514.5 nm wavelength (Ion Laser Technology, model # 5500A-00). The measurements were taken 2.75 cm from the tip of the nozzle, as this represents the distance from the proximal opening of the nasal vestibule to the center of the respiratory epithelium. Initially, the measurement volume was moved across the aerosol stream to determine the edges of the aerosol. Then, aerosol sizing measurements were determined at 1mm intervals across the width of the spray, taking 30,000 measurements at each interval. Sizing data is presented as a volume weighted mean and span, defined as $Span = \dfrac{D_v 90 - D_v 10}{D_v 50}$ where $D_v$ is droplet frequency distribution.

Each time the POD spray device was used, the desired dose was placed onto the wire mesh within the needle body. A fluorescence assay was used to determine if the total desired volume was dispensed from the device with each actuation. A solution of 50 $\mu$g/ml fluorescein was dispensed from the device into a well in a 24-well plate that was prefilled with 1 ml deionized $H_2O$. After collection of the aerosol, the solution was mixed by pipetting up and down several times. Three volumes 5 $\mu$l, 10 $\mu$l, and 25 $\mu$l which could be used for nasal delivery were tested at two different pressures, 20 and 30 psi. The fluorescence of the liposome solution was measured on a PerkinElmer 1420 multivariable fluorescence plate reader (PerkinElmer, Waltham, MA).

In order to test rotational pattern of the aerosol dispensed from the POD device, coomassie blue dye was dispensed from the device onto an absorbent paper in order to measure the aerosol pattern at various distances from the nozzle tip. The POD body was mounted above the piece of paper to minimize any effects of gravity on the plume geometry. Two level tools were attached to the POD device, parallel and perpendicular to the ground, in order to ensure that the aerosol plume was directed perpendicular to the ground for each aerosol plume tested. The aerosol plume was dispensed at distances of 1.0, 2.0, and 3.0 cm from the absorbent paper with driving pressures of 20 and 30 psi. The diameter and geometric dye profile were observed at each distance and for each driving pressure.

Several methods of nasal administration were tested for deposition within the nasal cavity. Under anesthesia, the rats (N = 6) received dye solution as a nasal instillation of a dye solution via a catheter, which was either 15 or 7 mm long, while in the supine position. The test animals receiving dye solution via the POD device were treated as described above as the standard use of the POD device. The test animals receiving dye solution via nose drops were placed on their back and a 5 $\mu$l drop of dye solution was placed near the right naris with a pipette and allowed to be snorted into the nasal cavity. After 2 minutes, the process was repeated in the opposite naris. Shortly after administration (< 5 minutes), the animals were overdosed with 250 mg/kg pentobarbital. The nasal cavity was then bisected at the septum, the septum was removed, and the tissues were examined for dye localization.

For histopathologic analysis, rats that received POD spray at the highest and repeated doses were anesthetized with 50 mg/kg sodium pentobarbital delivered via IP injection. These animals (N = 6) were placed in the supine position and they received a single 10 $\mu$l dose of 0.1 mg mannitol dissolved in 0.9% NaCl with the POD device according to the method described above. Nasal tissues from two untreated control rats were processed in the same manner as the treated tissues. Three driving pressures (10, 20, and 30 psi) of the POD device were tested. Within 5 minutes of dosing the animal was euthanized with an IP injection of 250 mg/kg sodium pentobarbital. Histopathologic analysis of the POD nasal device was conducted according to the method of Young (Young, 1981). In brief, the head was removed and the brain and jaw were removed from the head along with any other listed tissues. The nasal cavity was initially fixed in a solution of 10% formalin and then decalcified in a solution of 10% EDTA. The tissue was then placed in 70% ethanol before being embedded in paraffin, sectioned, and stained with hematoxylin and eosin stain.

The silicon nasal model was held in position with a clamp. Two angle measures were attached on the side and the top of the nasal cavity model to measure the horizontal and vertical angle with respect to the model. The POD device was positioned with another clamp at a set angle with the nozzle of the device placed 1 cm into the naris and along the bottom of the naris. Olfactory deposition was tested with the same POD device setup using a nozzle without a rotational component to the aerosol and a standard nasal spray pump (Pfeiffer, Radolfzell, Germany). A dose of 50 $\mu$l deionized water was sprayed from the device with each actuation. The olfactory region of the model was demarcated on both the septum and turbinates of the model (Leopold et al., 2000). Immediately after spraying, the model was disassembled and a pre-weighed paper (Kimwipe, Kiberly-Clark, Roswell, GA) was used to absorb all of the liquid in the demarcated olfactory area. The Kimwipe was then weighed to determine the amount of the dose deposited on the olfactory region. In one experimental set, the full dose was collected in order to determine the weight loss due to evaporation.

These simulations were set up to determine the ability of different nozzles to displace dead air in the cone and deposit aerosol on the upper part of the cone as a rough simulation of deposition on the olfactory region (FIG. 27). The simplex air flow pattern and velocity of the spray from a flow simulation using nozzle structure having an outlet without circumferential velocity did a poor job of penetrating to the top of the cone because it could not move the air in the narrow part of the cone. Therefore, the aerosol plume deposited mostly on the sides of the nasal cavity. The circumferential flow pattern and velocity of the aerosol plume using a single outlet nozzle structure also resulted in poor deposition on the olfactory region. The spray flow coming out of the nozzle structure having a single outlet with circumferential velocity does not penetrate into the cone either because a flow with vortical flow coming out of one orifice tends to spread out when exiting the orifice.

The nozzles with a plurality of outlets resulted in the desired narrow, circumferential flow pattern. The narrow circumferential spray flow improved penetration of the cone and lead to penetration of a majority of the aerosol to the top of the cone due to displacement of the air in the upper nasal cavity. The flow simulation comparison using the various nozzle structures described in this example demonstrates the advantages of using a nozzle having a plurality of outlets which generate a narrow spray pattern having circumferential velocity to penetrate a narrow area such as the upper nasal cavity of a human, where the air must be displaced to allow for penetration of the spray in order to deposit a large fraction of drug on the olfactory epithelium.

A prototype was used to evaluate this optimized *in silico* model. As schematically presented in FIG. 28, the aerosol dispensing intranasal device which is designed to reliably dispense the target drug solution in a fixed dose for the nasal cavity is intended for olfactory deposition. The voltage timer used was employed as a simple design to program and reliably actuated the pneumatic solenoid. The foot pedal switch actuation of the timer then allowed the user to properly place the POD nozzle within the nasal cavity before and during dose release or administration to rats. The solenoid was placed between the pressurized air supply and the device body to control the duration of the valve opening on a consistent basis. Using a large capacity nitrogen tank as a pressure source, no discernable pressure drop is expected for during the duration when the valve was open. The screen within the nozzle body provided homogeneity of the aerosol released from this device. During testing of the dose volume output, for dispensing a dose of 10 $\mu$l, the device performed at the set volume with 99.3 $\pm$ 2.6% accuracy. At 50 $\mu$l dose, the dose accuracy was 98.4 $\pm$ 3.1%.

To evaluate vortical flow pattern of aerosol dispensed or discharged from the POD prototype device, a color dye in POD was dispensed with a fixed perpendicular orientation, onto a semi-absorbent surface at increasing distance. The effect of the spin insert within the nozzle resulted in a rotational component to the aerosol released from the device while maintaining a narrow aerosol plume (FIG. 29). The panels on the left display the resulting aerosol pattern created by the POD device with the spin insert placed in the nozzle, while the panels on the right display the aerosol pattern from the same device using the same device and settings, except that an insert that had straight channels with no rotation about the cylindrical axis was used. The dye pattern from the POD with the spin insert resulted in a narrow spray pattern with the bulk of the dye in a different location relative to the device outlet as the distance from the target increases. This indicates a rotating spray plume. When the bulk of the dye location was measured as a function of distance, the rotation of the aerosol plume was calculated to be 22.5 °/cm. In addition, the aerosol plume created with the spin insert within the nozzle appeared to have a smaller diameter at the distances tested, although due to the spread of the dye upon impaction with the surface, the exact diameters could not be measured.

Dye deposition within the nasal cavity was determined after delivery with either the POD device or nose drops. All of the panels in FIG. 30 show a sagittal view of the nasal cavity. The left panels of FIG. 30 show the deposition after delivery with the POD device with either 10 or 30 $\mu$l, while the panels on the right show the deposition in the nasal cavity after delivery with nose drops. The blue circle indicates the olfactory epithelium within the nasal cavity, while the green circle outlines the respiratory epithelium. The white line indicates the cribriform plate, which is the interface between the nasal cavity and the olfactory bulb area of the brain. When using 10 $\mu$l of dye, the POD spray resulted in deposition primarily on the olfactory epithelium area of the nasal cavity. The dye was found primarily on the posterior two-thirds of the olfactory turbinates and within the folds of the turbinates as well as deeper within the nasal cavity along the cribriform plate region of the nasal cavity. In addition, the dye could be visualized on the cribriform plate from both the nasal cavity and brain cavity. When using 30 $\mu$l of dye was administered with the POD device, the localization within the nasal cavity was similar to that after a 10 $\mu$l POD spray except that the dye localized more broadly on the olfactory turbinate structures including the front third and there was minor deposition on the respiratory epithelium near the center of the nasal cavity.

Administering the dye by nose drops (FIG. 30, right panels) resulted in deposition primarily on the respiratory epithelium. Administering 10 $\mu$l of dye as nose drops resulted in the dye being localized completely to the respiratory epithelium with no noticeable dye staining in the olfactory region, or in the trachea or esophagus. The nose drop administration of 30 $\mu$l of dye resulted in saturation of the entire nasal cavity and thus, deposition throughout the respiratory epithelium and possibly partially on the olfactory epithelium. In contrast to the deposition of dye after the POD delivery, the 30 $\mu$l nose drops only led to minor if any deposition on the olfactory epithelium and that was limited to the very anterior portion of the epithelium.

To determine whether the impact of aerosol delivered by POD device on nasal tissue, histopathological analysis was performed on the nasal tissues after exposing the with aerosol generated by POD device. Histopathology analysis of the tissues collected from rats exposed to aerosol generated by various pressure did not appeared to be different from the control rats (FIG. 31). The septum area of the nasal cavity was closely examined because the method of administration of the POD device included placing the nozzle of the spray device approximately 1 cm into the naris primarily traveling along the septum. The histopathology sections show the same location of the septum which would have been in contact with the POD aerosol, in which there is no discernable damage in the POD administered animals compared to untreated control animals. There was also no detectable difference in the mucosa after administration of POD with increasing pressures tested.

It was determined that the olfactory deposition of the POD device in a human nasal cavity model in order to confirm the results of the computational fluid dynamics simulations and to determine the suitability of the POD device for use in humans. To do so, olfactory not only at the natural device angle of 30-35 degree in relation to the vertical plane was evaluated, 30-60 degree was tested to simulate conditions where such a device will be used in practice. When comparing the POD device having the rotational component in the aerosol output with a POD setup with no rotational component, the POD device with a rotational component in the aerosol consistently deposited a higher fraction of dose on the olfactory region, regardless of the vertical angle. These values are significant different from those achieved with POD device dispensing aerosol without any rotational flow at many of high vertical angles (FIG. 32). The olfactory deposition of a standard nasal pump was also determined for comparison. The standard nasal pump consistently deposited a low percentage of the dose on the olfactory region with most angles of administration resulting in less than 5% of dose depositing on the olfactory region.

By testing various pressures and angles of administration in the human nasal cavity model, the impact of device orientation on olfactory deposition was determined. In general, the greatest olfactory deposition was attained when the vertical angle was between 30° and 40°. These vertical angles of administration also produced the lowest levels of variability when the horizontal angle was changed. Using a vertical administration angle of 60° or higher resulted in significantly decreased olfactory region deposition. There was no significant difference when the driving pressure was increased from 30 psi to 45 psi. The output pressure was approximately 5-10 psi.

Example 16

Mannitol was $^{14}$C labeled with a specific activity of 0.1 mCi/ml and purchased from Moravek (Brea, CA). Unlabeled mannitol was purchased from Sigma-Aldrich (St. Louis, MO). Nelfinavir was $^{14}$C labeled with a specific activity of 1.0 mCi/ml and purchased from Amersham Biosciences (Piscataway, NJ). Unlabeled nelfinavir was gratefully donated by the NIH AIDS Research and Reference Reagent Program (Germantown, MD). Propylene glycol was purchased from Sigma (St. Louis, MO). EtOH was purchased from Fisher Scientific (Fair Lawn, NJ). Medical grade nitrogen gas was purchased from Airgas Nor Pac (Vancouver, WA). The 0.9% saline solution was purchased from Hospira Inc (Lake Forest, IL). Nembutal was purchased from Abbot Laboratories (North Chicago, IL). Biosol and Bioscint were purchased from National Diagnostics (Atlanta, GA). All other materials were reagent grade.

After characterization of the different nasal drug delivery methods mentioned in the previous chapter, $^{14}$C-labeled mannitol was administered to either the respiratory region of the nose with nose drops or to the olfactory region of the nasal cavity with the POD device or via intravenous (IV) administration, and then brain and blood distributions were determined at 30 and 150 minutes. In addition, a hydrophilic drug, nelfinavir was delivered to either the respiratory region of the nose with nose drops or to the olfactory region of the nasal cavity with the POD device and brain and blood distribution was determined after 30 minutes.

Adult male Sprague-Dawley rats (200-300g,) were purchased from Harlan (Indianapolis, IN), and were housed under a 12 hour light/dark cycle with food and water provided *ad libitum.* Animals were cared for in accordance with institutional guidelines, and all experiments were approved by the University of Washington Animal Care and Use Committee.

Mannitol doses used in the histopathology example consisted of 0.2 mg mannitol dissolved in 0.9% saline solution, pH 7.4. Radiolabeled mannitol dose solutions consisted of 0.2 mg unlabeled mannitol and 2.0 $\mu$Ci $^{14}$C mannitol in a solution of 98% $H_2O$ and 2% EtOH, pH 7.4. The total volume of each nasal mannitol dose was 20 $\mu$l while each IV dose was administered as a volume of 100 $\mu$l. Nelfinavir dose solutions consisted of 0.12 mg unlabeled nelfinavir and 2.0 $\mu$Ci $^{14}$C nelfinavir in a solution of 75% propylene glycol and 25% EtOH. The total volume of each nasal nelfinavir dose was 20 $\mu$l. All drug solutions were mixed on the day of delivery.

The POD device was constructed as described previously. Basic operation of the POD device was as follows. The dose was loaded into the POD device and the nozzle was carefully placed 8.0 mm into the nasal cavity and pointed in the direction of the cribriform plate. Then the foot pedal was pressed to actuate the solenoid for 0.1 seconds to propel the dose to the olfactory region of the nasal cavity.

Animals w under anesthesia were placed on their backs on a rodent heat pad (Harvard Apparatus, Holliston, Massachusetts) to maintain body heat. A group of animals were given 5 $\mu$l of mannitol as nose drops every minute in alternating naris for a total of 20 $\mu$l volume. In another group, mannitol was given using the POD aerosol device. These rats were first given a spray in the left naris; followed after 2 minutes with a second 10 $\mu$l dose in the right naris for a total volume of 20 μl. The intravenous (IV) mannitol doses were administered with a tail vein injection. Nelfinavir nose drop and POD spray doses were administered with identical method and volumes as described for the mannitol. At either 30 or 150 minutes after radiolabeled drug dosing the animals were euthanized. Blood was collected and the brain was removed. The brain was dissected into the olfactory bulbs, cortex, diencephalon, brainstem, and cerebellum. The olfactory bulbs were the last tissues to be removed from the skull. Cervical spinal cord from C1 to C5 was removed from the body as well. The nasal cavity was also carefully opened and the olfactory and respiratory epithelia were removed.

Tissue samples were weighed and placed in 4 ml polypropylene scintillation vials with 400 μl of Biosol. Each blood sample was placed into 400 μl of Biosol immediately after collection. All samples were placed in a water bath at 55°C overnight to digest the tissues. The tissues were allowed to cool to room temperature and the vials were filled with Bioscint scintillation fluid. A volume of 40 μl of 30% hydrogen peroxide was added to each of the samples before scintillation counting to determine radio activity of the drugs.

Radioactivity in each sample was analyzed with a Packard Tricarb 1600 TR liquid scintillation counter (Packard Instruments, Meriden, CT). With each sample set analysis, standard curve and control samples were run with the unknown samples.

Brain and blood concentrations were determined with standard curves constructed with blank tissue or blood that was spiked with radiolabeled drug and processed according to the methods for the radiolabeled samples. A one-way ANOVA with a Tukey post test was used to compare drug distribution in the brain after nose drops, POD spray, or IV delivery with mannitol, while an unpaired t-test was used to compare drug distribution in the brain after nose drops or POD spray of nelfinavir.

To evaluate the effects of POD device that provide preferential deposition of aerosolized compound to olfactory regions can enhance drug exposure in the brain and also the role of hydrophobicity of the compound in POD mediated drug delivery to the brain, a hydrophilic compound mannitol (log P = -3.9) and a hydrophobic anti-HIV drug nelfinavir (log P = 6.0) were employed. Rat administered with these two compounds in the POD device or nose drop method were evaluated at 30 and 150 minutes to determine the differential effects in the drug concentrations in the brain. Mannitol and nelfinavir were chosen based on each drug having very poor penetration into the brain, due to limited membrane crossing and efflux transport respectively. In addition, using these two drugs allows for a better understanding of the impact of hydrophobicity on transport from the nasal cavity to the brain.

As shown in FIG. 33, compared to animals treated with either nose drops (which deposit mannitol primarily on the respiratory region of the nasal cavity) or IV route, those received mannitol in POD device by nasal route exhibit significantly different brain distribution profile. The animals dosed with mannitol via the POD device exhibited about 2 nmol/g at olfactory bulbs and 0.2 - 0.4 nmol/g in the brain's cortex, brain stem and spine by 30 min. In comparison, animals receiving mannitol by IV or by nose drops exhibit less than 0.1 mmol/g at the olfactory bulbs, and less than 0.02 nmol/g in the brain's cortex, brain stem and spine at identical time point. These data were analyzed further by normalization with mannitol concentrations in blood and presented in FIG. 34. Again, the largest difference observed was in the olfactory bulbs. While there was no significant difference in the concentrations between nose drop and IV administration, administration of mannitol with the POD device resulted in a 25.6-fold increased concentration compared to nose drops and an 53.1-fold increase in concentration compared to IV. The cortex also had a large concentration difference with POD administration of mannitol resulting in 11.6 and 8.69-fold increases in concentration compared to nose drop and IV administration respectively. The diencephalon and cerebellum were also significantly different when dosed via POD nasal spray compared to nose drops and IV administration. The brainstem and spinal cord had the highest degree of variability and therefore, there was no significant difference between the routes of administration. There was no significant difference in blood concentration between the three routes of administration.

Mannitol concentrations within the brain were also determined at 150 minutes in order to better understand the distribution and clearance of this drug after distribution to the olfactory region. After mannitol administration, most of the brain regions had decreased concentration (FIG. 35). After nose drop or POD mannitol administration, the olfactory bulbs had the highest concentration in comparison to the rest of the brain regions. The olfactory bulbs after POD device administration of mannitol to the olfactory region of the nasal cavity resulted in a 22.1-fold increased concentration (P < 0.05) compared to those with nose drops, and a 40.0-fold increase (P < 0.05) compared to IV administration. There was no significant difference between any of the other brain regions or the blood concentrations.

Lipophilic (log P = 6) anti-HIV drug nelfinavir. Despite the lipophilicity of nelfinavir, a lower fraction of drug accumulated between the olfactory and the brain than that observed for mannitol. Nevertheless, compared to animals treated with nelfinavir in nose drops, those receiving nelfinavir in POD device exhibit higher drug concentrations in olfactory epithelium of the nasal cavity and significantly higher brain concentrations (P < 0.05) (FIG. 36) At 30 min, the olfactory bulb and the cortex nelfinavir concentrations in animals treated with POD device were 1.91 and 4.05 fold higher, respectively, than those treated with nose drop. Interestingly, the blood concentration of nelfinavir was 3.2 times higher (P < 0.05) after nose drop administration than after POD administration.

POD administration of both mannitol and nelfinavir to the olfactory region resulted in significantly higher (P < 0.05) olfactory bulb and brain concentrations at 30 minutes compared with nose drop delivery to the respiratory epithelium (FIG. 37). The olfactory bulb concentration after POD administration of mannitol had the highest blood normalized concentration of any tissue measured and was 18.6 times greater than the blood normalized olfactory bulb concentration after nose drop delivery. The nelfinavir POD led to a 3.6 fold increase in blood normalized olfactory bulb concentration compared to nose drops. An average of all brain tissues displayed that POD deposited mannitol resulted in a 3.4 fold increase compared to nose drops and a 2.1 fold increase with nelfinavir.

Taking advantage of the ability of the POD device to preferentially deposit drugs to the olfactory region, the impact of enhanced drug accumulation in olfactory bulb in increasing the drug concentration in the brain and spinal cord were evaluated. The results of this example show that POD drug delivery specifically to the posterior olfactory region of the nasal cavity resulted in preferential brain distribution compared with drug delivery to the anterior respiratory region in rats by typical nasal drop administration. Also the POD device mediated brain penetration was observed for both a hydrophilic and hydrophobic small molecule drug, *albeit* to different degrees. In the case of mannitol, olfactory administration also increased brain levels compared to systemic, IV, administration. Administering the drug to the olfactory region led to dramatically increased concentrations in olfactory bulbs, with a 25.6-fold increased concentration compared to nose drops and a 53.1-fold increase concentration compared to IV injection. The cortex, diencephalon, and cerebellum within the brain also exhibited significantly increased concentrations after delivery to the olfactory region. In addition, delivery of the lipophilic drug nelfinavir to the olfactory region of the nasal cavity resulted in higher concentrations in the brain, with 1.4-fold higher concentrations in the olfactory bulbs and 4.0-fold higher concentrations in the cortex, and lower concentrations in the blood stream compared to drug delivery to the respiratory region of the nasal cavity.

Mannitol is a hydrophilic small molecule drug (log P = -3.4) with properties that make it an ideal substrate to investigate direct transport from the nasal cavity to the brain. It is metabolically inert and exclusively eliminated by the kidneys, with a half life of 100 minutes (Cloyd et al., 1986). Mannitol also does not react with any known receptors and has an extremely low permeability across membranes (Miki et al., 1996). Mannitol has long been used as a diuretic and as a marker to test renal function (Williams et al., 1955), and also used as a non-absorbable marker for intestinal drug absorption studies. There were significant differences in brain exposure at 30 minutes after mannitol delivery with the two nasal delivery methods as well as IV administration. After IV administration the brain exposure is very low, which is expected. Once mannitol is in the blood stream it would have to cross the BBB by non-saturable passive diffusion in order to penetrate the brain parenchyma. An example investigating mannitol distribution into the CNS spaces found that after a 10 minute IV infusion, brain concentrations quickly reached 1.2% of blood concentration and then slowly increased (Sisson and Oldendorf, 1971).

Similarly low brain levels after IV administration were observed (FIG. 33). After nose drop delivery to the respiratory epithelium, a significant reduction in blood levels with little to no significant difference in brain concentrations was observed. Since the respiratory epithelium is bound by tight junctions (Young, 1981) very little mannitol would be expected to penetrate to the lamina propria where it could be taken up into the blood stream or directly distributed to the brain. If the mannitol that penetrated the nasal respiratory epithelium was only taken up into the blood stream and then distributed to the brain, the blood normalized brain concentration would be the same after nose drop or IV delivery. As seen in FIG. 34, the blood normalized brain concentrations after nose drop administration were significantly greater than those after IV administration. The greatest differences were observed in the olfactory bulb region.

These differences between the blood normalized mannitol concentrations after nose drops compared to IV administration imply that after nose drop administration to the respiratory epithelium, there is some degree of direct transport to the brain with the greatest amount of direct transport to the olfactory bulbs. One explanation for this data is that a portion of the nose drop dose naturally migrated to the olfactory epithelium region of the nasal cavity and was directly transported to the brain along the olfactory nervous connections. Although the dye deposition staining from the previous chapter displayed no dye staining on the olfactory epithelium after nose drops, some of the drug may have moved within the nasal cavity to the olfactory epithelium. Although the volume of mannitol solution administered (20 μl) was low and the animal head position was maintained throughout the experiment to minimize movement within the nasal cavity, the natural mucocilliary clearance and breathing of the animal could have caused some of the drug to be displaced within the nasal cavity. Another possibility is that the drug is directly traveling from the nasal cavity to the brain along the trigeminal nerve pathway, as indicated in several drug delivery studies (Thorne et al., 2004, Ross et al., 2004). The trigeminal nerves innervate the respiratory region of the nasal cavity and connect with several brain regions including the olfactory bulbs and the brainstem (Anton and Peppel, 1991). Interestingly, the increased blood normalized brain concentrations seen after nose drops to the respiratory epithelium come about primarily from the decreased blood levels compared to IV administration.

In contrast to nose drop delivery of mannitol, POD delivery of mannitol to the cribriform plate region led to increased concentrations at 30 minutes compared to IV administration. The olfactory bulbs had the highest concentrations of any brain tissues after any route of administration. All of the rostral regions of the brain, including the olfactory bulbs, cortex, and diencephalon had significantly higher brain concentrations after POD administration (FIG. 33). A similar pattern of drug distribution brain has been reported with other nasal drug delivery studies that reported evidence of direct transport from the nasal cavity to the brain (Graff et al., 2005, Westin et al., 2006), in which drugs were visually detected, by fluorescence or autoradiography respectively, crossing the cribriform plate from the nasal cavity into the subarachnoid space surrounding the olfactory bulbs and cortex.

POD delivery to the olfactory region of the nasal cavity also resulted in higher blood concentrations compared to nose drop delivery. This is most likely due to the mannitol being able to more easily penetrate the olfactory epithelia and gain access to the lamina propria. Jansson et al. showed that hydrophilic dextran could not penetrate the tight junctions of the respiratory epithelium but could penetrate beyond the olfactory epithelium to the lamina propria within 5 minutes of administration (Jansson and Bjork, 2002). Similarly, mannitol appears to have more easily penetrated the olfactory epithelium and gained greater access to the lamina propria and the blood.

Despite the higher blood concentrations of mannitol, the blood normalized mannitol concentrations after POD administration were significantly higher than nose drops in the olfactory bulbs and the cortex, and supports the conclusion that POD delivery to the olfactory region resulted in a greater fraction of drug directly delivered to the brain. This data suggests that the POD spray enabled a greater fraction of the mannitol to travel directly from the nasal cavity to the brain. Our results are consistent with the hypothesis that the olfactory nerves in the nasal cavity create fluid filled pathways in which molecules can undergo a rapid non-receptor mediated transport to the olfactory bulbs and other brain regions.

POD delivery of the hydrophobic anti-HIV drug nelfinavir also resulted in higher concentrations in brain compared to nose drop delivery (FIGS. 36 and 37). Nelfinavir was chosen as a model lipophilic drug for this example because of it exhibits low brain penetration despite it hydrophobicity with log P value of 6.0. It has been shown by many investigators, including us that nelfinavir is a substrate of multidrug drug resistant transporter MDR1 or P-glycoprotein that expressed at the blood brain barrier and remove nelfinavir from the brain. As a result, like other oral anti-HIV drugs it does not reach the brain in effective concentrations after reaching therapeutic drug levels in the blood which can lead to neurological complications in HIV patients (Minagar et al., 2008).

After POD distribution to the olfactory region, nelfinavir concentrations in the olfactory bulbs and cortex were significantly increased compared to nose drop delivery to the respiratory region. In addition, the blood concentrations were significantly lower than after nose drops administration. The primary reason that systemically administered nelfinavir does not readily appear in the brain is due to the efflux pump p-glycoprotein which is highly expressed at the BBB (Jarvis and Faulds, 1998). This protein is also highly expressed within the olfactory epithelium of the nasal cavity and much less so at the respiratory region (Kandimalla and Donovan, 2005). Due to its lipophilicity (log P = 6.0), it is likely that nelfinavir rapidly penetrates the respiratory epithelium after nose drop delivery and quickly enter the capillary system in the lamina propria. This would result in the higher blood concentrations and lower brain concentrations observed after nose drop delivery to the respiratory epithelium.

After POD delivery to the olfactory region, nelfinavir would not readily cross the olfactory epithelium due to p-glycoprotein. This would limit its uptake into the blood from lamina propria near the cribriform plate. The drug may still be directly transported into the CNS region surrounding the olfactory bulb by utilizing the olfactory nerve connections, as visually observed with morphine (Westin et al., 2005). Several other studies have shown that intranasally delivered p-glycoprotein substrates can still result in improved brain concentrations (Graff and Pollack, 2005, Padowski and Pollack, 2010). It seems from this example that most of the enhanced brain distribution of nelfinavir, and possibly other lipophilic compounds that are P-gp substrates, results from this direct nose-to-brain transport from the olfactory region of the nasal cavity.

The POD device preferentially deposits drugs at the olfactory region occupying upper third of nasal cavity for both a hydrophilic and hydrophobic compound. Depositing a majority of drug on the olfactory region in the nasal cavity results in enhanced brain-to-blood ratios. This enhanced drug delivery to the brain is likely due to the drug molecules utilizing anatomical connections in the olfactory region of the nasal cavity to bypass the blood stream and distribute directly from the nasal cavity to the brain. Localizing the drug to the respiratory epithelium could be desirable when trying to avoid distribution in the brain. Depositing a majority of drug on the olfactory region could lead to enhancing effective brain concentrations for many drugs with limited ability to penetrate or cross the BBB.

Example 17

Morphine sulfate and fentanyl citrate were purchased from Sigma-Aldrich (St. Louis, MO). Beuthanasia-D (Shering Plough Animal Health Corp, North Chicago, IL) was used for euthanasia at the end of the distribution studies. The drug doses were dissolved in 0.9% saline solution (Hospira Inc, Lake Forest, IL). Morphine and fentanyl LC/MS standards were purchased from Cerilliant (Round Rock, TX). All other materials used were reagent grade.

This example consisted of three parts. In the first part of the example, analgesic effect using the well established tail-flick latency test in rats was evaluated. This was a randomized crossover study with two days between each experiment. The two day rest time is sufficient to allow both morphine and fentanyl levels to return to baseline and ensure that no opioid tolerance effects would develop. The animals were each briefly anesthetized and given a single drug administration. Each animal (N = 9) received a single dose strength (1.0, 2.5, 5.0 mg/kg morphine and 7.5, 15, and 25 $\mu$g/kg fentanyl) of opioid analgesic drug by the three different delivery routes. They were then allowed to recover and the tail-flick test was performed multiple times during the 2 hours after treatment.

In the second phase, plasma drug concentrations were determined after a single treatment of the opioid drugs. The plasma draws mirrored the tail-flick study in order to compare the analgesic effect of the opioid drugs with the blood concentration to determine any apparent direct nose-to-brain delivery. Again, the single treatment of opioid analgesic was administered via POD nasal spray, nose drops, or IP injection. The same three doses of drug were used in this part of the example (2.5, 5.0, 10.0mg/kg morphine and 7.5, 15, and 25 μg/kg fentanyl). The animals (N=3) were anesthetized and given a single drug treatment. Blood was drawn from a femoral catheterization over the course of the experiment. Blood was drawn over the course of 2 hours at the same time points as the tail-flick test after drug administration.

The third part of the example included a tissue distribution study. The goal of this part of the study was to confirm the tail-flick study by determining the tissue concentrations of drug in the CNS 5 minutes after administration. The animals (N=6) were briefly anesthetized and given a dose of either morphine or fentanyl by nasal spray, nose drops, or IP injection. Only a single dose of each drug was tested in this part of the example (2.5 mg/kg morphine or 15 μg/kg fentanyl).

Adult male Sprague-Dawley rats (200-300 g; Harlan, Indianapolis, IN) were housed under a 12 hour light/dark cycle with food and water provided *ad libitum.* Animals were cared for in accordance with institutional guidelines, and all experiments were approved by the University of Washington Animal Care and Use Committee.

Animals were anesthetized with 2% isoflurane (Novaplus, Lake Forest, IL). Body temperature was maintained at 37°C by a heating pad (Fine Science Tools, Inc., Foster City, CA). For PK experiments, the femoral vein was cannulated for blood draw with PE10 polyethylene tubing (Becton Dickinson, Franklin Lakes, NJ) connected to blunt tipped 23 gauge needle (Becton Dickinson, Franklin Lakes, NJ). The PE-10 tubing was inserted 2 cm into the femoral vein to ensure blood sampling was from the vena cava. Animals were maintained on anesthesia for 30 minutes prior to drug administration. Following the final blood draw, the catheter was removed from the femoral vein, the proximal end of the femoral vein was tied off with suture string to assure hemostasis, and the incision stitched with suture string (Harvard Apparatus, Holliston, MA).

Morphine and fentanyl were stored at -20°C as a lyophilized powder. On the day of each experiment, the necessary doses of morphine and fentanyl were solubilized in 0.9% saline solution (Sigma-Aldrich, St. Louis, MO). Each formulation had a pH of 7.0 for each of the doses tested.

For each experiment, the animals were dosed morphine or fentanyl while under isoflurane anesthesia. For the tail-flick and distribution study, each animal was briefly anesthetized with 5% isoflurane in an induction chamber, the animal was removed from the induction chamber and the drug was administered, the animal was turned on to its side to prevent drug drainage and then the animal was allowed to recover from the anesthesia.

Each animal remained anesthetized with 2% isoflurane throughout the plasma draw study. First the femoral vein was cannulated in order to draw blood during the experiment. After surgery the animal was allowed to remain under anesthesia for 20 minutes before drug administration. Then, for the olfactory and respiratory nasal drug delivery, the anesthesia nose cone was removed, the dose was given quickly (< 45 seconds) and the nose cone was replaced. For IP delivery the dose was simply injected with a 23 gauge syringe into the peritoneal cavity. After drug administration each animal was placed on its side on the heat pad.

In the CNS distribution experiment, animals were initially anesthetized with 5% isoflurane. Once unconscious, they were quickly removed from the induction box and dosed as described for the tail-flick experiment with nose drops, POD spray, or IP injection. They were allowed to naturally recover from the anesthesia.

Each group of animals for a dose strength of morphine or fentanyl (N=9) first went through three days of placebo testing to get a baseline reading for the tail-flick test as well as acclimating the animals to handling. Each animal was exposed to 5% isoflurane in an induction box. Once anesthetized the animal was removed and given a 10μl dose of 0.9% saline solution, pH 7.4 via nose drop, POD nasal spray, or IP injection. Once the placebo dose was administered, the animals were allowed to fully wake from the isoflurane in a padded tray. Then at 5, 10, 30, 45, 60, 90, and 120 minutes each animal was wrapped gently in a towel, had their tails placed in room temperature water (18°±0.5°C) for 5 seconds, the tail was quickly dried, and then the distal 3 cm of the tail was placed in 55°±0.5°C water. The time until tail removal was measured with a digital stopwatch.

After the initial placebo trials, the same procedure was repeated three times, every other day over 5 days, with each rat receiving a single dose of either morphine or fentanyl by nasal spray, nose drops, and IP injection in a randomly chosen order. The cutoff time, at which the tail would be removed from the water to prevent tissue damage, was set at 10 seconds for all tail flick trials.

In the PK experiments, 300μl of blood was drawn from the femoral vein catheter at 5, 10, 30, 45, 60, 90, and 120 minutes after drug administration. The blood was collected in a 1ml syringe (Becton Dickinson, Franklin Lakes, NJ) and transferred to a microcentrifuge tube for blood/plasma separation. The tubes were immediately centrifuged at 8,000 g for 5 minutes. Then the plasma was removed and frozen on dry ice. At the end of the experiment 2.0 mls of sterile 0.9% saline solution was injected via the femoral vein catheter to replace the removed blood volume.

A fixed volume (20 μl) of morphine d-6 (Cerilliant, Palo Alto, CA) was added into each tissue and plasma sample to act as an internal standard. Morphine tissue samples were homogenized in 5-10 times volume of 0.1M borate buffer, pH 8.9 and centrifuged for 10 minutes at 1000g. The morphine tissue supernatant and plasma samples were passed over Certify solid phase extraction cartridges (Varian, Palo Alto, CA) and eluted with methylene chloride: isopropanol: ammonium hydroxide (80:20:2). After elution the samples were evaporated under $N_2$ gas until dry. The samples were resuspended in 75 $\mu$l of mobile phase which consisted of 92% of 0.05% acetic acid and 8% acetonitrile mobile phase. An Agilent HPLC/MS series 1100 series B with autosampler (Agilent, Santa Clara, CA) was used for quantification. The injection volume was 5 $\mu$l. The morphine samples were passed over a Zorbax SB-C8 column (Agilent, Santa Clara, CA) with a flow rate of 0.25 ml/min. The ionization setting was API-ES in positive mode with a capillary voltage of 1400V.

The fentanyl samples were quantified in a similar process. A fixed volume (20 $\mu$l) of fentanyl d-6 (Cerilliant, Palo Alto, CA) was added into each tissue and plasma sample to act as an internal standard. Fentanyl tissue samples were homogenized in 5-10 times volume of 0.1M potassium phosphate buffer, pH 6.0 and centrifuged for 10 minutes at 1000g. The fentanyl tissue supernatant and plasma samples were passed over Certify solid phase extraction cartridges (Varian, Palo Alto, CA) and eluted with methylene chloride: isopropanol: ammonium hydroxide (80:20:2). After elution the samples were evaporated under $N_2$ gas until dry. The samples were resuspended in 75 $\mu$l of mobile phase which consisted of 40% 10mM ammonium acetate and 60% acetonitrile. An Agilent HPLC/MS series 1100 series B with autosampler (Agilent, Santa Clara, CA) was used for quantification. The injection volume was 5 $\mu$l. The fentanyl samples were passed over a Zorbax SB-C8 column (Agilent, Santa Clara, CA) with a flow rate of 0.25 ml/min. The ionization setting was API-ES in positive mode with a capillary voltage of 1400V.

For both morphine and fentanyl, a standard curve was created on the day of analysis according to the same process described for the samples. Each standard curve was linear with a coefficient of linear regression $R^2 > 0.99$. In addition, two quality control samples with a known amount of drug were processed on the day of analysis in order to ensure day-to-day consistency of the analytical assay.

All tail-flick test values are presented as a percentage of maximal possible effect, which is defined as:

$$\%MPE = \frac{(Post\ drug\ latency - baseline\ latency)}{(cutoff\ time - baseline\ latency)} \times 100\%$$

AUC values from all experiments were calculated using the trapezoidal rule without extrapolation to infinity. Tail-flick data was compared using repeated measures ANOVA. Plasma and tissue concentrations were compared using a one-way ANOVA with a Tukey post-test. $AUC_{effect}/AUC_{plasma}$ ratios were calculated from individual animals so they could be statistically compared with a one-way ANOVA. All statistical analyses were performed using Sigma Plot software version 11.0 (Systat Software Inc, San Jose, CA).

A direct transport percentage (DTP%) was calculated in order to determine the amount of drug in the brain that was distributed directly from the nasal cavity to the CNS. Analgesic effect instead of brain concentrations was used. This can be done as tail-flick analgesic effect has been shown to correlate well with morphine concentrations in the extracellular brain fluid (Letrent et al., 1999a). The DTP% is used to estimate the amount of drug in the brain that cannot be accounted for by systemic distribution. The DTP is defined was calculated as follows (Zhang et al., 2004):

$$\frac{AUC_{effect(IP)}}{AUC_{plasma(IP)}} = \frac{B_X}{AUC_{plasma(nasal)}} ;$$

$$DTP\% = \frac{AUC_{effect(nasal)} - \bar{B_X}}{AUC_{effect(nasal)}} \times 100\% .$$

Plasma concentrations were taken over the course of 120 minutes after drug administration at the same time points as the tail-flick test. The tail-flick test was done to determine the effect and act as a surrogate for morphine concentrations in the brain (Letrent et al., 1999a). By analyzing analgesic effect along with plasma concentrations, it can be determined whether there is any difference in direct distribution from the nasal cavity to the brain between the routes of administration.

Dosing with 5.0 mg/kg morphine resulted in significantly higher plasma concentrations after IP administration compared to nose drop or POD spray administration (FIG. 38 Panel A). The plasma concentrations after IP administration were significantly higher ($p < 0.05$) over the first 30 minutes with a $T_{max}$ at 10 minutes with a $C_{max}$ of 579.0 ng/ml. The IP plasma values at 10 minutes were roughly 2.6-fold higher than the plasma concentrations 10 minutes after either

nasal administration. The $AUC_{0-120}$ after IP administration of 5.0 mg/kg morphine was significantly higher than after either type of nasal administration. The only point where there was a significant difference between the POD spray and nose drops was at the 5 minute time point (p < 0.05), when the plasma concentration after POD spray was nearly 2-fold higher than after nose drops. Both the POD spray and the nose drops resulted in a $T_{max}$ of 30 minutes and $C_{max}$ values of 322.0 and 296.2 ng/ml respectively.

The plasma concentrations of 15 μg/kg fentanyl were significantly higher (p < 0.05) after both POD spray and nose drop administration than after IP administration over the course of 120 minutes (FIG. 38 Panel B). In the case of POD spray and IP administration, the $T_{max}$ was at the earliest recorded time point of 5 minutes with the plasma concentration decreasing after that. The plasma concentrations after nose drop administered fentanyl resulted in a $T_{max}$ at 10 minutes and although there was high variation in the first two time points with nose drops, all animals had the same $T_{max}$ and overall plasma curve shape. At the 5 minute time point the nose drop and POD spray plasma concentrations were 5 and 6.7 times higher than the IP plasma levels respectively.

The analgesic effect, as measured by the tail-flick test, resulting from 5.0 mg/kg morphine was significantly higher (p < 0.05) over the first 60 minutes after POD spray as compared with nose drops. In addition, POD spray resulted in a significantly higher analgesic effect (p < 0.05) than IP administration at the initial 5 minute time point. The $T_{max}$ after POD spray was at 10 minutes and remained at approximately the same level over the following 50 minutes. The $AUC_{0-120}$ after both POD spray and IP were significantly greater (p < 0.05) than the $AUC_{0-120}$ after nose drop administration. There was no significant difference in analgesic AUC between POD spray and IP administrations after 5.0 mg/kg morphine.

POD administration to the olfactory region of the nasal cavity of 15 μg/kg fentanyl resulted in a much greater analgesic effect (FIG. 39 Panel D) at 5 minutes compared to nose drops or IP administration (p < 0.05 vs nose drops and p < 0.01 vs IP). It should be noted that at the 5 minute time point over half of the POD treated rats did not pull their tails from the water bath before the ten second cutoff time. Therefore, the analgesic effect from the POD spray at 5 minutes is underestimated. After the 5 minute point however, the analgesic effect after POD spray dropped quickly. After this initial time point there was no significant difference between the analgesic effects from three routes of administration. The total $AUC_{0-120}$ after POD administration was significantly less (p < 0.05) than after either nose drop or IP administration. At the 5 and 10 minute time points the nose drops also resulted in a significantly higher analgesic effect (4 < 0.05) than IP delivery.

POD administration of morphine resulted in a significantly lower plasma AUC after a 5.0 mg/kg dose and a non-significantly lower plasma AUC at 2.5 mg/kg (FIG. 39). POD administration resulted in non-significantly higher plasma AUC at 1.0 mg/kg dose. In addition, POD administration resulted in a significantly higher effect AUC compared to nose drops at 1.0 and 5.0 mg/kg doses and a significantly higher effect AUC compared to IP administration after a 1.0 mg/kg dose. POD administration also resulted in 1.64, 1.61, and 2.24 fold increases in $AUC_{effect}/AUC_{plasma}$ ratio compared to IP after 1.0, 2.5, and 5.0 mg/kg doses administration at each dose tested. There was no significant difference in this AUC ratio between IP administration and nose drop administration. The DTP% after POD administration was estimated to be 38.5%, 38.1%, and 55.0% after 1.0, 2.5, and 5.0 mg/kg doses respectively. The DTP% after nose drop administration was estimated to be 0%, 10.9%, and 14.8% after 1.0, 2.5, and 5.0 mg/kg doses respectively.

When plotted as effect vs plasma concentration, several differences between nose drop, POD spray, and IP administration become apparent. The point on each graph at (0,0) represents the baseline tail-flick test before dosing. It is semi-artificial and was primarily added for easier visualization of the data sequence. After a 2.5 mg/kg morphine dose (FIG. 39 Panels A-C) both nose drop and IP administration resulted in a counterclockwise hysteresis, whereas the POD administration resulted in a clockwise hysteresis. After 15 μg/kg fentanyl nose drops led to no discernable hysteresis (ignoring the baseline point), POD led to a clockwise hysteresis, and IP administration led to a counterclockwise hysteresis (FIG. 39 Panels D-F). All three fentanyl effect-concentration curves were plotted on the same axis scale so the plot for IP administration appears much smaller due to the limited increase in plasma concentration and analgesic effect from this route of administration with fentanyl.

The brain concentrations of morphine at 5 minutes after POD administration of 2.5 mg/kg to the olfactory region of the nasal cavity were higher than either nose drop administration to the respiratory region of the nasal cavity or IP injection (FIG. 40). Although the olfactory bulb morphine concentration was much higher after POD administration, 7.60 times the concentration after nose drop administration and 39.58 times the concentration after IP administration, it was not significantly higher due to the high amount of variation after the POD spray and the low sample number (N=3). However the rest of the brain (including the upper cervical spinal cord) did have a significantly higher (p < 0.05) morphine concentration at 5 minutes after POD spray. Administration with POD spray resulted in 1.68 times the brain concentration after nose drops and 3.38 times the brain concentration after IP administration. There was no significant difference between brain concentrations at 5 minutes after nose drop or IP administration.

Similar differences were observed in the brain tissue morphine concentration when normalized by plasma morphine concentration (FIG. 41). Again, POD spray resulted in non-significantly greater blood normalized morphine concentrations in the olfactory bulbs. In the rest of the brain, POD spray to the olfactory region of the nasal cavity resulted in significantly higher (p < 0.05 vs nose drops and p < 0.01 vs IP) blood normalized brain concentrations. The blood normalized brain concentrations at 5 minutes after POD spray were observed to be 2.27 and 6.48 times the concentrations after nose drop and IP administration.

Nasal delivery to the olfactory region 5 minutes after a dose of 15 $\mu$g/kg fentanyl with the POD spray resulted in significantly higher (p < 0.05) concentrations in the brain than after either nose drop administration to the respiratory region or IP injection (FIG. 39 Panel B). In this example the fore brain included the olfactory bulbs. At 5 minutes, the POD spray of fentanyl resulted in 1.63 increased concentration compared to nose drop application and a 5.61-fold increase compared to IP administration. The results in the midbrain, cerebellum and brain stem (MCS) were similar. The POD spray application resulted in 1.56 and 5.32 times the fentanyl concentration when compared with nose drops administration or IP injection respectively. In addition, the POD spray resulted in significantly greater (p < 0.05) plasma concentrations at 5 minutes after 15 $\mu$g/kg fentanyl administration. The POD spray resulted 1.28 times higher plasma fentanyl concentrations compared with nose drops and a 4.5 times higher concentration than IP administration.

Neither POD administration nor nose drop administration led to statistically significant higher $AUC_{effect}/AUC_{plasma}$ ratios compared to IP administration. Therefore, the DTP% were not calculated for fentanyl. In addition, there were no significant differences between the plasma normalized brain concentrations after nose drops, POD spray, or IP administration (FIG. 43). In both the forebrain and MCS the POD spray resulted in a non significant trend of higher blood to brain ratios at 5 minutes after administration of 15 $\mu$g/kg fentanyl.

The results of this example show that there are significant differences in the analgesic effect and plasma concentration-time profile of both fentanyl and morphine when delivered to the olfactory region of the nasal cavity compared to the respiratory region. Delivering morphine to the olfactory region with the POD device resulted in direct transport from the nasal cavity to the CNS, increasing the analgesic effect while maintaining low plasma values. Delivering fentanyl to the olfactory region resulted in a very strong and rapid analgesic effect, which appears to be primarily due to uptake into the blood stream followed by distribution to the CNS.

Morphine administration to the olfactory region with the POD device of the nasal cavity resulted in statistically significant differences in both plasma concentration and analgesic effect. After a 5.0 mg/kg dose of morphine, IP administration resulted in significantly higher plasma concentrations, while POD and nose drop delivery to the nasal cavity both resulted in much lower plasma concentration with approximately 30% lower AUC values. The plasma concentrations after nose drops and POD administration were nearly identical which means that there was a similar uptake of morphine in the blood from both the respiratory and olfactory epithelium. In contrast, the POD device lead to significantly higher analgesic effect compared to nose drops and had similar analgesic effect compared to IP administration. The $AUC_{effect}/AUC_{plasma}$ ratio was higher after POD deposition compared to nose drops or IP administration. When taken together this data shows that in addition to morphine uptake into the blood, which was similar to uptake at the respiratory region, there was further uptake into the CNS after deposition at the olfactory region of the nasal cavity.

This additional uptake into the CNS observed after olfactory delivery was rapid in nature. At the initial time point measured, POD administration did not result in higher plasma concentration, but did result in significantly higher analgesic effect compared to the other two routes of administration. This rapid nature of distribution into the CNS is seen in the plasma concentration vs analgesic effect curves, where POD administration of morphine resulted in a clockwise hysteresis curve compared to the other two routes which displayed a clockwise hysteresis. This clockwise hysteresis could be explained by either acute tolerance to the morphine or the pain test, or it could be due to the analgesic effect taking place before the rise of the plasma levels (Perez-Urizar et al., 2000).

Shang et al. (Shang et al., 2006) reported a clockwise hysteresis in CSF concentration vs. effect which they attributed to a sensitization to the tail-flick test. However, sensitization to the tail-flick test is not a likely explanation for our observed results, as no loss of response to the pain test when doing placebo testing was observed (data not shown). The clockwise hysteresis observed after POD administration of morphine is primarily due to a rapid rise in effect before the rise in plasma concentration, in contrast to Shang et al., where it was primarily due to a decreased effect at the later time points. In addition, the nose drop administration to the respiratory epithelium and the systemic IP administration in our example resulted in a counter clockwise hysteresis, which is typical of systemically administered morphine due to difficulties crossing the BBB (Letrent et al., 1999b). Instead, this clockwise hysteresis after administration with the POD device is more likely due to direct transport from the nasal cavity to the CNS such as that observed by Westin et al. (Westin et al., 2005, Westin et al., 2006).

This conclusion is supported by the brain/plasma ratios observed 5 minutes after administration with the POD device, which were significantly higher than after nose drop or IP administration. This increase in the brain/plasma ratios, especially at the earliest time points was also observed by Westin et al. (Westin et al., 2006) where brain-to-plasma AUC ratios of 0.5 were observed in the first 10 minutes after nasal administration. In this example the morphine was visually observed to distribute from the nasal cavity to the lamina propria surrounding the olfactory bulb. In our example, a large difference was also observed in the olfactory bulb morphine concentrations; however, these differences were not significant, most likely due to the method of collection of the olfactory bulb. The olfactory bulbs were scraped out of the cranial cavity and the subarachnoid space surrounding the olfactory bulbs, which may have contained a high concentration of morphine, was most likely also collected in an inconsistent manner. The variability of this collection method along with the small sample size (N=3) most likely caused the lack of significance.

Westin et al. calculated the percentage of drug delivered directly to the brain, or DTP%, over the course of 240 minutes. They reported that from 0-60 minutes and 0-240 minutes the DTP% morphine were 48% and 10% respectively. In this example, it was found that the DTP% after nose drops was between 0-10% depending on the dose, while after POD administration the DTP% was between 38 and 55%. It is difficult to directly compare our DTP% values to Westin et al. because they used brain concentrations while analgesic effect was used. They also did not calculate a 0-120 min DTP%. However, it is clear from our example that POD delivery to the olfactory region resulted in a higher DTP% compared to nose drop administration to the respiratory epithelium.

In contrast to the enhanced CNS distribution observed after POD administration to the olfactory region, nose drop distribution to the respiratory region displayed little to no evidence of direct transport form the nasal cavity to the brain. Although the nose drop distribution resulted in lower plasma concentrations compared to IP administration, it also resulted in significantly lower analgesic effect. The $AUC_{effect}$ / $AUC_{plasma}$ after respiratory epithelium and IP administration were not significantly different across the doses tested and resulted in a counter clockwise hysteresis typical of systemic administration. IP administration was chosen as a systemic method of administration because the plasma time course profile of morphine is similar after nasal administration and IP administration (Letrent et al., 1999a), thus the AUCs can be directly compared. These data indicate that morphine that was applied to the respiratory epithelium distributed primarily into the capillaries of the nasal cavity where it was distributed to the CNS across the BBB.

Lipophilic fentanyl (log P = 3.9) administration to the olfactory region resulted in a different pattern of distribution compared with hydrophilic morphine (log P = 0.8). Immediately after POD administration of fentanyl the plasma and analgesic levels were very high and then both dropped dramatically. In fact, POD administered doses of 7.5 and 15.0 μg/kg led to significantly lower $AUC_{effect}$ compared with nose drop administration. These results are what would be expected from a systemic administration of fentanyl (Yassen et al., 2006, Yassen et al., 2008). The hysteresis curve after POD administration of a 15.0 μg/kg indicated slight hysteresis but only at the initial 5 minute point, and this seems to be due to the dramatic decrease in analgesic effect with the slight drop in plasma effect. Although this could indicate some direct transport of fentanyl from the olfactory region to the CNS, this conclusion is not supported by the measured brain tissue concentrations. The forebrain and the midbrain, cerebellum, and brainstem/spinal cord (MCS) contained higher fentanyl concentrations 5 minutes after POD administration to the olfactory region. However, when normalized by plasma, there was no statistical difference between any of the routes of administration. In addition, both forms of nasal administration resulted in lower $AUC_{effect}$/$AUC_{plasma}$ ratios compared with IP delivery, indicating that all three routes of administration resulted in CNS distribution from the blood stream.

This example suggests that POD administration of fentanyl to the olfactory region of the nasal cavity primarily leads to uptake into the capillaries of the olfactory epithelium followed by distribution to the CNS across the BBB. Fentanyl is a very lipophilic compound, which readily penetrates biological membranes including the BBB (Bernards, 1994). In fact, the bioavailability of nasally administered fentanyl is 71% (Dale et al., 2002). Thus, fentanyl applied to either the respiratory or olfactory epithelium would likely be rapidly absorbed into the blood stream. This would leave little drug to distribute directly to the CNS. In addition, because fentanyl in the blood stream quickly crosses the BBB to cause an analgesic effect, any direct nose-to-brain delivery would likely be difficult to detect. Several other studies of nasally administered lipophilic drugs such as estradiol (van den Berg et al., 2004) and lidocaine (Bagger and Bechgaard, 2004) have also been unable to observe any direct distribution from the nasal cavity to the CNS. Interestingly, an increased level of analgesia and brain concentrations 5 minutes after POD administration to the olfactory epithelium compared with nose drop delivery to the respiratory region of the nasal cavity was observed. One explanation for this is that in rats, the density of blood vessels in the lamina propria of the olfactory region is more than double that in the lamina propria of the respiratory epithelium (Yuasa, 1991). This implies that fentanyl applied to the olfactory epithelium could be absorbed into the blood stream at a greater rate than when applied to the respiratory epithelium.

These results with both morphine and fentanyl could have significant significance for clinical application of nasally applied opioid drugs. There were significant differences after deposition on the olfactory or respiratory epithelium with both drugs. Interestingly, the plasma and effect profiles of both fentanyl and morphine after nose drop administration to the respiratory epithelium were the most similar to the values obtained from human nasal administration of these opioid drugs (Illum et al., 2002, Kress et al., 2009) with morphine having $T_{max}$ plasma values between 10 and 30 minutes, and fentanyl having $T_{max}$ values between 5 and 10 minutes. This is not surprising considering that most of these trials use standard nasal pumps, which primarily deposit drug on the respiratory epithelium (Newman et al., 2004). Thus, low volume (<15 μl) nose drop administration in rats may be an appropriate model for nasal administration in humans with a standard nasal pump.

In contrast, POD distribution to the olfactory region could be representative of deeper localized olfactory delivery within the nasal cavity for humans. POD administration of morphine resulted in an increased effect compared to respiratory epithelial deposition and lower plasma values compared systemic administration. In addition, POD administration resulted in a measurable analgesic effect at 5 minutes. Thus, administering morphine to the olfactory region of the nasal cavity could be an effective, non-invasive method of opioid administration, which could lower systemic side effects such as opioid induced constipation. Olfactory delivery of fentanyl resulted in a very high immediate analgesic effect resulting from rapid distribution into the blood stream. This could be of clinical value to reduce the onset time and dose required for outpatient fentanyl use.

This example shows that there are clinically important distributional differences of morphine and fentanyl based on the localization of deposition within the nasal cavity. Delivering morphine to the olfactory region compared to the respiratory region of the nasal cavity resulted in a portion of the drug directly distributing from the nasal cavity to the CNS and greater analgesic effect. In contrast, delivering fentanyl to the olfactory region resulted in an increased rate of drug uptake into the blood stream.

Example 18

1-(2-chloroethyl)-3-cyclohexyl-1-nitroso-urea (CCNU) was kindly provided by the Drug Synthesis and Chemistry Branch of the Developmental Therapeutics Division of Cancer Treatment of the NCI (> 99.8% purity). The phospholipids, 1,2-dimyristoyl-sn-glycero-3-phosphocholine (DMPC) and 1,2-dimyristoyl-sn-glycero-3-phosphoglycerol (DMPG) (both > 99% purity) were purchased from Sygena, Inc. (Cambridge, MA). Palmitylated peptide Gly-Arg-Gly-Asp-Ser (RGD) (> 99.9% purity) was custom synthesized by United Biochemical Research, Inc. 1,2-dipalmitoyl-sn-glycero-3-phosphoethanolamine-N-(7-nitro-2-1,3-benzoxadiazol-4-yl) (> 99% purity) from Avanti Polar Lipids (Alabaster, AL). Fentanyl citrate purchased from Sigma-Aldrich (St. Louis, MO). Beuthanasia-D (Shering Plough Animal Health Corp, North Chicago, IL) was used for euthanasia at the end of the distribution studies. Fentanyl was dissolved in 0.9% saline solution (Hospira Inc, Lake Forest, IL). All other chemicals used were of analytical grade.

Routinely, lipid-associated fentanyl (drug:lipid molar ratio 1:10) was prepared by first dissolving 20 mg of DMPC and DMPG (1:1, mol/mol) with CCNU in 1 ml of chloroform in a test tube and evaporating off the solvent with a stream of $N_2$ gas to form a dry film, followed by vacuum desiccation overnight to remove residual solvent. In the targeted liposomes, 0-1% mol/mol RGD was added to the organic phase. Where needed, 1% mol/mol of NBD-PE, (Avanti Polar Lipids, Alabaster, AL) a fluorescent lipid marker, was added to the organic phase. The dry film was then vacuum desiccated for at least 30 min. To prepare desired lipid concentrations, a 1-ml volume of 0.9% saline solution with 0.375 mg/ml fentanyl was then added to create a 20-mg/ml suspension. The mixture was then sonicated at 27°C in a (water) bath type sonicator (Laboratory Supplies, Inc., Hicksville, NY) until a uniform translucent suspension of small unilamellar vesicles (SUVs) was obtained. Under these conditions, it was found that roughly 80% of the fentanyl in the suspension was lipid associated. In the case of the drug release example, the PBS added prior to sonication contained 50mM calcein (Sigma, St. Louis, MO). After sonication the free calcein was removed from the solution by dialysis. Free CCNU dosages were prepared just prior to drug administration and consisted of dissolved CCNU in a carrier solution of sterile 0.9% NaCl with 10% ethanol and 2% Tween 80.

The size of liposomes were determined by photon correlation spectroscopy (PCS) (Malvern Zetasizer 5000, Southborough, MA). The liposomes and free drug solution were aerosolized by spraying the solution through a pressure driven aerosol nozzle. The liposome containing aerosol droplet size distributions were determined by a Phase Doppler Particle Analyzer (PDPA) (TSI, Shoreview, MN using a 200mW argon laser emitting beams of 488 and 514.5 nm wavelength (Ion Laser Technology, model # 5500A-00). The measurements were taken 2.75 cm from the tip of the nozzle, as this represents the distance from the proximal opening of the nasal vestibule to the center of the respiratory epithelium. Initially, the measurement volume was moved across the aerosol stream to determine the edges of the spray. Then, sizing measurements were determined at 1mm intervals across the width of the spray, taking 30,000 measurements at each interval. Sizing data is presented as a volume weighted mean and span, defined as $Span = \dfrac{D_v 90 - D_v 10}{D_v 50}$

where $D_v$ is droplet frequency distribution. For determination of a change in liposome particle size due to aerosolization, liposome size was determined by PCS before and after the solution was collected in a 25 ml conical tube at a 5° angle to the container.

The amount of liposome leakage due to aerosolization was determined according to the method of Piperoudi et. al. (Piperoudi et al., 2006) . The liposomes were sonicated in a solution of PBS containing 50mM calcein. The liposomes were then dialyzed overnight to remove the non-encapsulated calcein. The fluorescence signal from the encapsulated calcein at 50mM is quenched and has no fluorescence signal, but as the liposomes leak calcein into the surrounding buffer the fluorescence signal increases. A fraction of the liposomes were aerosolized and collected in a 5 ml conical tube and measured for fluorescence signal (cite calcein paper here). The percent leakage was calculated from the difference in fluorescence signal before and after aerosolization compared to the total encapsulated calcein (determined by adding 1% Tween 20 to liposomes to release all encapsulated calcein and measuring fluorescence signal). The fluorescence of the liposome solution was measured on a PerkinElmer 1420 multivariable fluorescence plate reader (PerkinElmer, Waltham, MA).

HUVEC, LLC-PK1, and A549 cell lines are well validated cell lines to study RGD-integrin interactions and were purchased from ATCC (Manassas, VA). LLC-PK1 cells were cultured in DMEM supplemented with 10% FBS and antibiotics (100 U/mL penicillin G and 0.1 mg/mL streptomycin). HUVEC cells were cultured in F12-K media supplemented with 100ug/ml endothelial cell growth supplement (BD Biosciences, Franklin Lakes, NJ), 10% FBS, and antibiotics. A549 human lung cancer cells were cultured in F12-K media with 10% FBS and antibiotics. All cells were grown at 37 °C in a 5% $CO_2$ and 95% air humidified atmosphere.

The cell lines used for the binding studies were plated into 96-well flat-bottomed cell sterile culture plates (BD Biosciences, Franklin Lakes, NJ) at a density of $1.0 \times 10^5$ cells/well. Once the cells reached confluency, the media was removed and the cells were incubated with 200µl of the various liposome preparations for 30 minutes at 37 °C in a 5% $CO_2$ and 95% air humidified atmosphere. In the case of the competitive binding experiment the cells were pre-incubated for 20 minutes with 25X concentration of soluble cyclo Arg-Gly-Asp-D-Phe-Lys (cRGD) peptide (Peptides International, Louisville, KY). The cells were then gently washed 3 times with PBS, pH 7.4, covered with 200ul of PBS, pH 7.4, and then analyzed with either a fluorescent plate reader or a Zeiss Axiovert 200 fluorescent microscope (Carl Zeiss Inc., Jena, Germany).

For each experiment, the animals were dosed free drug or liposome encapsulated fentanyl while under isoflurane anesthesia. For the tail-flick and distribution study, each animal was briefly anesthetized with 5% isoflurane in an induction chamber, the animal was removed from the induction chamber and the drug was administered, the animal was turned on to its side and then the animal was allowed to recover from the anesthesia.

Each animal remained anesthetized with 2% isoflurane throughout the pharmacokinetic example. First the femoral vein was cannulated in order to draw blood during the experiment. After surgery, the animal was allowed to remain under anesthesia for 20 minutes. Then, for the olfactory and respiratory nasal drug delivery, the anesthesia nose cone was removed, the dose was given quickly (< 45 seconds) and the nose cone was replaced. After drug administration, each animal was placed on its side on a heat pad.

In the CNS distribution experiment, animals were initially anesthetized with 5% isoflurane. Once they were unconscious, they were quickly removed from the induction box and dosed as described for the tail-flick experiment. They were allowed to naturally recover from the anesthesia.

Each group of animals first went through three days of placebo testing to get a baseline reading for the tail flick test as well as acclimating the animals to handling. Each animal was exposed to 5% isoflurane in an induction box. Once anesthetized the animal was removed and given a 10µl dose of 0.9% saline solution, pH 7.4 via POD device. Once the placebo dose was administered, the animals were allowed to fully wake from the isoflurane in a padded tray. Then at 5, 10, 30, 45, 60, 90, and 120 minutes each animal was wrapped gently in a towel, had their tails placed in room temperature water ($18° \pm 0.5°C$) for 5 seconds, the tail was quickly dried, and then the distal 3 cm of the tail was placed in $55° \pm 0.5°C$ water. The time until tail removal was measured with a digital stopwatch.

After the initial placebo trials, the same procedure was repeated three times, every other day over 5 days, with each rat receiving a single dose of fentanyl by POD spray with either free drug fentanyl or fentanyl incorporated into RGD-liposomes. The cutoff time, at which the tail would be removed from the water to prevent tissue damage, was set at 10 seconds for all tail-flick trials.

In the PK experiments, 300µl of blood was drawn from the femoral vein catheter at 5, 10, 30, 45, 60, 90, and 120 minutes after drug administration. The blood was collected in a 1ml syringe (Becton Dickinson, Franklin Lakes, NJ) and transferred to a microcentrifuge tube for blood/plasma separation. The tubes were immediately centrifuged at 8,000g for 5 minutes. Then the plasma was removed and frozen on dry ice. At the end of the experiment 2.0 mls of sterile 0.9% saline solution was injected via the femoral vein catheter to replace the removed blood volume.

A fixed volume (20 µl) of fentanyl d-6 (Cerilliant, Palo Alto, CA) was added into each tissue and plasma sample to act as an internal standard. Fentanyl tissue samples were homogenized in 5-10 times volume of 0.1M potassium phosphate buffer, pH 6.0 and centrifuged for 10 minutes at 1000g. The fentanyl tissue supernatant and plasma samples were passed over Certify solid phase extraction cartridges (Varian, Palo Alto, CA) and eluted with methylene chloride: isopropanol: ammonium hydroxide (80:20:2). After elution the samples were evaporated under $N_2$ gas until dry. The samples were resuspended in 75 µl of mobile phase which consisted of 40% 10mM ammonium acetate and 60% acetonitrile. An Agilent HPLC/MS series 1100 series B with autosampler (Agilent, Santa Clara, CA) was used for quantification. The injection volume was 5 µl. The fentanyl samples were passed over a Zorbax SB-C8 column (Agilent, Santa Clara, CA) with a flow rate of 0.25 ml/min. The ionization setting was API-ES in positive mode with a capillary voltage of 1400V.

A standard curve was created on the day of analysis according to the same process described for the samples. Each standard curve was linear with a coefficient of linear regression $R^2 > 0.99$. In addition, two quality control samples with a known amount of drug were processed on the day of analysis in order to ensure day-to-day consistency of the analytical assay.

All tail flick test values are presented as a percentage of maximal possible effect which is defined as:

$$\frac{(Post\ drug\ latency - baseline\ latency)}{(cutoff\ time - baseline\ latency)} \times 100 \quad .$$

AUC values from all experiments were calculated using the trapezoidal rule without extrapolation to infinity. Data are presented as the mean $\pm$ SD. Statistical significance was evaluated either by unpaired Student's t-tests (two-sided) or one way ANOVA (either paired or unpaired) using SigmaPlot software (Systat, San Jose, CA).

First, the binding selectivity of RGD liposome to cells expressing integrin was determined. RGD-liposomes containing NBD-PE fluorescent marker and LLCPK epithelial cells that express integrin to characterize and visualize the targeting and binding properties were used. FIG. 44 shows increasing concentrations of both targeted and non-targeted liposomes incubated with $\alpha V \beta 3$ integrin expressing LLC-PK1 epithelial cells. The fluorescence intensity and distribution are observed to be greater with the RGD-expressed liposomes than with the control liposomes. The targeted formulation follows a dose dependent increase in binding along with a higher affinity for the cells implying RGD mediated binding. The control liposomes exhibited lower binding at all concentrations and the binding did not increase with increasing liposome concentrations.

To further determine the contribution of RGD to the targeted liposome binding to Integrin expressing cells, liposome formulations with various ratios of RGD: lipid, from 0.25% to 1.0% of the lipid concentration, were prepared for cell binding experiments. The targeted liposome formulations with varying ratios of RGD on their surface (under a fixed and identical lipid concentration) were incubated with HUVEC epithelial cells. As the relative density of RGD on the surface of the liposome was increased from 0.25% to 1.0%, the level of cell binding increased (FIG. 45). These data suggests that the binding is dependent on RGD density on lipid membrane of the liposomes.

To confirm that the integrin targeted liposomes displayed increased cellular binding due to the RGD motif of the RGD peptide embedded in the liposome, a competitive binding assay experiment was performed. When the cells were pre-incubated with 25X molar excess of a cyclic RGD peptide (which exhibits much higher affinity for $\alpha V \beta 3$ integrin receptors), targeted binding of the targeted liposomes was completely inhibited (FIG. 46).

For a targeted liposomal formulation to be effective for aerosol delivery, the liposome properties must be physically stable during aerosolization. The performance of aerosol particle size distributions of the aerosolized targeted liposomes was determined at 0.1 mM lipid concentration using an air assist aerosol device. The driving pressures of 2.0 psi produced a spray pressure similar to that produced by a simple nasal spray pump. The volumetric mean aerosol diameter of the liposome solution using the aerosol device was 29.18 $\mu$m with a span of 0.95 $\mu$m with a driving pressure of 2.0 psi. The first test of the targeted liposome stability was to determine any changes in liposome particle size after aerosolization. The RGD-liposome size distribution under these conditions was not significantly different before (mean of 96.5 $\pm$ 6.1 nm) or after (mean of 104.1 $\pm$ 4.9 nm) (P>0.05) being aerosolized, as seen in FIG. 47.

To evaluate the stability of the liposome membrane and whether these liposomes could retain the hydrophilic drugs encapsulated within aqueous compartment, water soluble calcein was used as a water soluble fluorescence marker (Hu et al., 1986). As a self-quenching dye, a high (50 mM) concentration of calcein encapsulated in the liposomes is quenched. If the calcein leaks out of the liposomes due to membrane instability during aerosolization, the calcein released into the surrounding buffer medium is greatly diluted and as a result, calcein fluorescence is unquenched or increased. The gain in calcein fluorescence provides a measure of calcein leakage from liposomes (ref Ho & Huang). Release of calcein due to aerosolization was minimal and recorded at between 2.0% and 2.2% of total encapsulated calcein in the liposomes.

To determine the stability of the RGD binding peptide due to aerosolization, binding affinity of the integrin targeted RGD liposomes before and after aerosolization to detect changes that could arise from the shearing forces involved in exiting the aerosol nozzle was determined. The binding experiments using LLCPK cells, which over-express integrin (FIG. 48) show no significant effect of RGD-liposome formulation after aerosolization. The integrin targeted liposomes did have significantly higher cell binding at all concentrations, implying that the integrin targeting peptide was not damaged or displaced during aerosolization. The control liposomes also exhibit similar stability further verifying the stability of the liposome composition during aerosolization.

In order to determine the effect of the RGD liposomes on nasal epithelial binding *in vivo*, fentanyl was incorporated into the liposomes and the analgesic and plasma concentration time profiles were determined (FIG. 49). The plasma profile after POD delivery of RGD-liposome incorporated fentanyl was similar to that of free drug. At 5 minutes after administration, the RGD-liposome formulation resulted in a significantly lower plasma concentration than free drug. Other than that the plasma profiles of free drug and RGD-liposome formulations were very similar. The RGD- liposomes resulted in a non-significantly lower AUC.

The tail-flick test was used to determine the analgesic affect after fentanyl dosing. There was a noticeable difference in the analgesic effect between the free drug formulation and the RGD-liposome formulation (FIG. 50). The free drug fentanyl formulation resulted in a powerful analgesic effect at the first recorded time point of 5 minutes and then rapidly decreased until there was no measurable analgesic effect at 30 minutes after delivery. In contrast, the fentanyl in the RGD-liposome formulation resulted in an analgesic effect with an effect$_{max}$ of 10 minutes. While the RGD-liposomes resulted in a lower analgesic effect at the initial 5 minute time point, this formulation resulted in a higher analgesic effect at every other time point measured. The RGD-liposomes also resulted in a significantly higher AUC$_{effect}$ than the free drug formulation.

To determine if there was any direct transport of fentanyl from the nasal cavity to the brain, fentanyl concentrations of brain and plasma 5 minutes after administration were determined. No difference in the blood normalized brain levels in either the forebrain or the MCS (midbrain, cerebellum, and brainstem were observed. Unlike the administration of free drug with the POD device, administration of the fentanyl containing RGD-liposomes resulted in very even distribution between the rostral and caudal brain regions. Neither formulation showed any statistical difference in blood normalized brain levels after delivery to the olfactory region compared to systemic intraperitoneal (IP) administration.

Taking advantage of liposomes containing DMPC and DMPG that are in gel-phase under room temperature to retain membrane integrity and retain incorporated as well as encapsulated materials under aerosolization, an integrin targeted liposome aerosol intended for nasal mucosa was made. FIG. 44 shows that the RGD peptide mediated a concentration dependant binding to the epithelial cells. The control liposomes tend to have lower binding at all concentrations and the binding doesn't increase visibly with increased dose. This is to be expected as non-targeted, uncharged liposomes tend to have very low levels of cell binding within the first 30 minutes (Lee et al., 1993). While the non-targeted formulation seems to appear in bright spots due to liposome aggregation, the RGD-expressed liposomes can clearly be seen binding around the cells on the cell surface where the integrin proteins are expressed.

The increased binding with increased levels of RGD (FIG. 45) shows that the cell binding is RGD mediated. As in FIG. 44, the targeted liposomes bind most intensely near the cell membranes where the target integrin proteins are expressed. In addition, the liposome formulation with 1% RGD clearly shows uptake of the liposomes into the cells, supporting previous reports of integrin mediated cellular uptake (Xiong et al., 2005, Xiong et al., 2008). The blocking of binding by incubation with free cRGD confirmed that the RGD was incorporated into the liposome membrane and exposed to the cells providing site-specific binding. The level of targeted control liposome binding with the blocking agent (cRGD) was comparable to the binding of the untargeted liposomes, confirming that the targeted liposomes are binding due to the RGD motif.

A pressure driven aerosol (POD) device was used to generate the aerodynamic particle size being in the $\mu$m range. With an aerodynamic particle size of 29.18 $\mu$m, this means that the average aerosol particle is more than $2.9 \times 10^6$ times larger by volume than the average liposome particle (d = 97 - 104 $\mu$m) in this formulation. This large volume difference could minimize any disruption of the liposomes when they are being aerosolized. The stable size distribution indicates minimal disruption of the liposomes due to the shear force of the aerosolization. The calcein release data indicate that there was minimal disruption of the liposome membrane during the aerosolization process, confirming the physical stability of the liposome membrane during aerosolization.

The stability studies confirm that both the liposomal membrane and the integrin targeting peptide remain stable during the aerosolization process. The stability of the liposome membrane is most likely due to the choice of lipids, which form gel-phase liposome membranes at room temperature during aerosolization. In addition, the short RGD peptide was chosen to ensure minimal damage due to shear force. This is relevant since liposomal formulations, and especially targeted liposomal formulations, could potentially improve drug absorption across the nasal and upper respiratory epithelia.

A 15 $\mu$g/kg dose of fentanyl was delivered with the POD device to the olfactory region resulting in a very high analgesic effect at 5 minutes which rapidly decreased thereafter. In this example, the effect of incorporating the fentanyl in RGD-liposomes (which bind to the olfactory epithelial cells) was determined. There were significant differences in both the on plasma levels and analgesic effect after incorporating the fentanyl into the RGD-liposomes.

The only statistically significant difference in the plasma time profiles of the free drug fentanyl and the RGD-liposome fentanyl was at 5 minutes, when the RGD-liposomes lead to a lower plasma value (FIG. 49). In contrast, the analgesic effect time profile was very different between the two formulations (FIG. 50). Neither formulation seemed to provide any evidence of direct distribution from the nasal cavity to the brain (FIG. 51), so the differences in analgesic effect must have come from differences in uptake into the brain.

**Claims**

1. A pharmaceutical aerosol suspension for use in a method of delivering a therapeutic compound to an olfactory epithelium in a nasal cavity of a mammal, the method comprising:

   providing a pharmaceutical aerosol suspension comprising an aerosol and the therapeutic compound, wherein the therapeutic compound is an opioid analgesic, mannitol, TS-002, IGF-1, orexin-A, interferon-$\beta$, MSH or an RGD peptide;

aerosolizing the suspension to generate a stream of droplets; and,
administering the stream of droplets having a rotational component having a circumferential velocity after entering the nasal cavity such that at least 15% of the stream of droplets are delivered directly to the olfactory epithelium.

2. The pharmaceutical aerosol suspension for use according to claim 1, wherein the opioid analgesic is morphine or fentanyl.

3. The pharmaceutical aerosol suspension for use according to claim 2, wherein the mammal is a human.

4. The pharmaceutical aerosol suspension for use according to claim 1, wherein the therapeutic compound is encapsulated within a liposome nanoparticle.

5. The pharmaceutical aerosol suspension for use according to claim 1, wherein greater than 40% of the stream of droplets is delivered to the olfactory epithelium.

6. The pharmaceutical aerosol suspension for use according to claim 1, wherein the suspension is the aerosol and orexin-A.

7. The pharmaceutical aerosol suspension for use according to claim 1, wherein the suspension is the aerosol and RGD peptide.

8. The pharmaceutical aerosol suspension for use according to claim 4, wherein the liposome nanoparticle comprises RGD peptide conjugated to palmitic fatty acyl chain.

9. The pharmaceutical aerosol suspension for use according to claim 8, wherein the liposome nanoparticle comprises the phospholipids 1,2-dimyristoyl-sn-glycero-3-phosphocholine (DMPC) and 1,2-dimyristoyl-sn-glycero-3-phosphoglycerol (DMPG) in a 1:1 ratio (mol/mol) and palmitic acid linked GRGDS peptide at 0.25 to 1.0 percent of the lipid concentration.

10. The pharmaceutical aerosol suspension for use according to claim 9, wherein the liposome nanoparticle further comprises 1-(2-chloroethyl)-3-cyclohexyl-1-nitroso-urea (CCNU).

11. The pharmaceutical aerosol suspension for use according to any preceding claim, wherein the method is for use in the treatment or prevention of a disease, condition or disorder.

12. The pharmaceutical aerosol suspension for use according to claim 11, wherein the disease, condition or disorder is selected from neurodegenerative diseases and hyperproliferative disorders, immune disorders, neurological disease or disorders for example epilepsy and seizure disorders, cardiovascular disease, metabolic disease, infectious disease, lung disease, genetic disease, autoimmune disease, and immune-related disease.

**Patentansprüche**

1. Pharmazeutische Aerosolzusammensetzung zur Verwendung in einem Verfahren zur Abgabe einer therapeutischen Verbindung an ein olfaktorisches Epithel im Nasenraum eines Säugers, wobei das Verfahren umfasst:

   Bereitstellen einer pharmazeutischen Aerosolsuspension, umfassend ein Aerosol und die therapeutische Verbindung, wobei die therapeutische Verbindung ein Opioidanalgetikum, Mannitol, TS-002, IGF-1, Orexin-A, Interferon-$\beta$, MSH oder ein RGD-Peptid ist;
   Aerosolisieren der Suspension, um einen Tröpfchenstrom zu erzeugen, und
   Verabreichen des Tröpfchenstroms, der eine Rotationskomponente mit einer derartigen Umfangsgeschwindigkeit nach Eintritt in den Nasenraum aufweist, dass mindestens 15% des Tröpfchenstroms direkt an das olfaktorische Epithel abgegeben werden.

2. Pharmazeutische Aerosolsuspension zur Verwendung nach Anspruch 1, wobei das Opioidanalgetikum Morphin oder Fentanyl ist.

3. Pharmazeutische Aerosolsuspension zur Verwendung nach Anspruch 2, wobei der Säuger ein Mensch ist.

**4.** Pharmazeutische Aerosolsuspension zur Verwendung nach Anspruch 1, wobei die therapeutische Verbindung in einem Liposomennanopartikel verkapselt ist.

**5.** Pharmazeutische Aerosolsuspension zur Verwendung nach Anspruch 1, wobei mehr als 40% des Tröpfchenstroms an das olfaktorische Epithel abgegeben wird.

**6.** Pharmazeutische Aerosolsuspension zur Verwendung nach Anspruch 1, wobei die Suspension das Aerosol und Orexin-A ist.

**7.** Pharmazeutische Aerosolsuspension zur Verwendung nach Anspruch 1, wobei die Suspension das Aerosol und RGD Peptid ist.

**8.** Pharmazeutische Aerosolsuspension zur Verwendung nach Anspruch 4, wobei das Liposomennanopartikel RGD Peptid, konjugiert an eine Palmitinfettsäureacylkette, umfasst.

**9.** Pharmazeutische Aerosolsuspension zur Verwendung nach Anspruch 8, wobei das Liposomennanopartikel die Phospholipide 1,2-Dimyristoyl-sn-glycero-3-phosphocholin (DMPC) und 1,2-Dimyristoyl-sn-glycero-3-phosphoglycerol (DMPG) im Verhältnis 1: 1 (Mol/Mol) und Palmitinsäure verknüpftes GRGDS-Peptid bei 0,25 bis 1,0 Prozent der Lipidkonzentration umfasst.

**10.** Pharmazeutische Aerosolsuspension zur Verwendung nach Anspruch 9, wobei das Liposomennanopartikel weiter 1-(2-Chloro-ethyl)-3-cyclohexyl-1-nitrosoharnstoff (CCNU) umfasst.

**11.** Pharmazeutische Aerosolsuspension zur Verwendung nach einem der vorstehenden Ansprüche, wobei das Verfahren zur Verwendung bei der Behandlung oder Vorbeugung einer Krankheit, eines Zustands oder einer Störung vorgesehen ist.

**12.** Pharmazeutische Aerosolsuspension zur Verwendung nach Anspruch 11, wobei die Krankheit, der Zustand oder die Störung ausgewählt ist aus neurodegenerativen Erkrankungen und hyperproliferativen Störungen, Immunstörungen, neurologischen Erkrankungen oder Störungen, beispielsweise Epilepsie und Anfallserkrankungen, kardiovaskuläre Erkrankungen, metabolische Erkrankungen, Infektionskrankheiten, Lungenerkrankungen, genetische Erkrankungen, Autoimmunkrankheiten und immunbedingte Erkrankungen.

## Revendications

**1.** Suspension aérosol pharmaceutique pour une utilisation dans une méthode d'administration d'un composé thérapeutique au niveau de l'épithélium olfactif dans une cavité nasale d'un mammifère, la méthode comprenant :

l'utilisation d'une suspension aérosol pharmaceutique comprenant un aérosol et un composé thérapeutique, dans laquelle le composé thérapeutique est un analgésique opioïde, le mannitol, le TS-002, l'IGF-1, l'orexine-A, l'interféron-β, le MSH ou le peptide RGD ;
l'aérosolisation de la suspension pour générer un flux de gouttelettes ; et
l'administration du flux de gouttelettes ayant un composant rotatif ayant une vitesse circonférentielle après son entrée dans la cavité nasale de telle sorte qu'au moins 15 % du flux de gouttelettes est administré directement au niveau de l'épithélium olfactif.

**2.** Suspension aérosol pharmaceutique pour une utilisation selon la revendication 1, dans laquelle l'analgésique opioïde est la morphine ou le fentanyl.

**3.** Suspension aérosol pharmaceutique pour une utilisation selon la revendication 2, dans laquelle le mammifère est un être humain.

**4.** Suspension aérosol pharmaceutique pour une utilisation selon la revendication 1, dans laquelle le composé thérapeutique est encapsulé dans une nanoparticule de liposome.

**5.** Suspension aérosol pharmaceutique pour une utilisation selon la revendication 1, dans laquelle plus de 40 % du flux de gouttelettes est administré au niveau de l'épithélium olfactif.

EP 2 605 816 B1

**6.** Suspension aérosol pharmaceutique pour une utilisation selon la revendication 1, dans laquelle la suspension est l'aérosol et l'orexine-A.

**7.** Suspension aérosol pharmaceutique pour une utilisation selon la revendication 1, dans laquelle la suspension est l'aérosol et le peptide RGD.

**8.** Suspension aérosol pharmaceutique pour une utilisation selon la revendication 4, dans laquelle la nanoparticule de liposome comprend le peptide RGD conjugué à une chaîne acyle grasse palmitique.

**9.** Suspension aérosol pharmaceutique pour une utilisation selon la revendication 8, dans laquelle la nanoparticule de liposome comprend les phospholipides 1,2-dimyristoyl-sn-glycéro-3-phosphocholine (DMPC) et 1,2-dimyristoyl-sn-glycéro-3-phosphoglycérol (DMPG) selon un rapport 1:1 (mol/mol) et le peptide GRGDS lié à de l'acide palmitique à 0,25 à 1,0 pour cent de la concentration en lipide.

**10.** Suspension aérosol pharmaceutique pour une utilisation selon la revendication 9, dans laquelle la nanoparticule de liposome comprend en outre la 1-(2-chloroéthyl)-3-cyclohexyl-1-nitroso-urée (CCNU).

**11.** Suspension aérosol pharmaceutique pour une utilisation selon l'une quelconque des revendications précédentes, dans laquelle la méthode est destinée à une utilisation dans le traitement ou la prévention d'une maladie, d'une pathologie ou d'un trouble.

**12.** Suspension aérosol pharmaceutique pour une utilisation selon la revendication 11, dans laquelle la maladie, la pathologie ou le trouble est choisi(e) parmi les maladies neurodégénératives et les troubles hyperprolifératifs, les troubles immunitaires, les maladies ou troubles neurologiques, par exemple l'épilepsie et les convulsions, les maladies cardiovasculaires, les maladies métaboliques, les maladies infectieuses, les maladies pulmonaires, les maladies génétiques, les maladies auto-immunes et les maladies du système immunitaire.

Fig.1.

**Fig.2A.**

**Fig.2B.**

*Fig.3.*

*Fig.4.*

Fig.5.

*Fig.6A.*

*Fig.6B.*

*Fig.6C.*

*Fig. 7.*

*Fig.8A.*

**Fig.8B.**

**Fig.8C.**

*Fig.8D.*

*Fig.8E.*

**Fig.9.**

**Fig.10.**

EP 2 605 816 B1

Fig.11.

FIG. 12

FIG. 13

FIG. 14

INDD spray particle distribution from 199 measurements
90 deg, 2 cm, 4 psi, water

FIG. 15

FIG. 16

FIG. 17

FIG. 18

FIG. 19

0% RGD

0.25% RGD

0.5% RGD

1% RGD

FIG. 20

with 25x cRGD

without 25x cRGD

FIG. 21

FIG. 22

FIG. 23

FIG. 24

FIG. 25

FIG. 26

FIG. 27

FIG. 28

Spray distance (cm)

1.0

2.0

3.0

FIG. 29

FIG. 30

FIG. 31

FIG. 32

FIG. 33

FIG. 34

FIG. 35

FIG. 36

FIG. 37

FIG. 38

FIG. 39

FIG. 40

FIG. 41

FIG. 42

FIG. 43

FIG. 44

FIG. 45

FIG. 46

FIG. 47

FIG. 48

FIG. 49

FIG. 50

DMPG  DMPC          GRGDS

A          B

PA-GRGDS

CNT

RGD
liposome          Integrin

Integrin expressing cell

C

FIG. 51

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 200209707 A

**Non-patent literature cited in the description**

• Remington's Pharmaceutical Sciences. Lippincott, Williams and Wilkins, 2005
• **XIONG XB et al.** Enhanced intracellular uptake of sterically stabilized liposomal Doxorubicin in vitro resulting in improved antitumor activity in vivo. *Pharm Res. 2005 Jun,* 08 June 2005, vol. 22 (6), 933-9
• **XIONG XB et al.** Intracellular delivery of doxorubicin with RGD-modified sterically stabilized liposomes for an improved antitumor efficacy: in vitro and in vivo. *J Pharm Sci.,* August 2005, vol. 94 (8), 1782-93
• **XIONG XB et al.** Enhanced intracellular delivery and improved antitumor efficacy of doxorubicin by sterically stabilized liposomes modified with a synthetic RGD mimetic. *J Control Release,* 03 October 2005, vol. 107 (2), 262-75
• **YOUNG JT.** Histopathologic examination of the rat nasal cavity. *Fundam Appl Toxicol,* July 1981, vol. 1 (4), 309-12